(19) Europäisches Patentamt
European Patent Office
Office européen des brevets

(11) Publication number: **0 347 987 A2**

## (12) EUROPEAN PATENT APPLICATION

(21) Application number: 89201563.7

(51) Int. Cl.⁴: C07K 5/02 , A61K 37/64

(22) Date of filing: 15.06.89

(30) Priority: 20.06.88 US 209749

(43) Date of publication of application: 27.12.89 Bulletin 89/52

(84) Designated Contracting States: CH DE FR GB IT LI NL

(71) Applicant: MERCK & CO. INC.
126, East Lincoln Avenue P.O. Box 2000
Rahway New Jersey 07065-0900(US)

(72) Inventor: Schoen, William R.
196 White Birch Road
Edison New Jersey 08837(US)

(74) Representative: Hesketh, Alan, Dr. et al
European Patent Department Merck & Co., Inc. Terlings Park Eastwick Road
Harlow Essex, CM20 2QR(GB)

## (54) Tripeptide renin inhibitors with N-terminal carbamoyl or acyl groups.

(57) Tripeptide enzyme inhibitors of the formula:

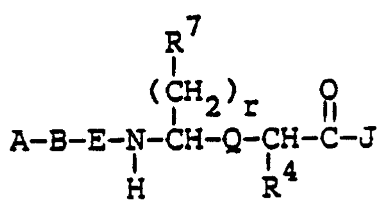

which have N-terminal carbamoyl or acyl functional groups and analogs thereof, which inhibit renin and are useful for treating various forms of renin-associated hypertension and congestive heart failure; compositions containing these renin-inhibitory peptides, optionally with other antihypertensive agents; and methods of treating hypertension or congestive heart failure or of establishing renin as a causative factor in these problems which employ the novel tripeptides.

EP 0 347 987 A2

# TRIPEPTIDE RENIN INHIBITORS WITH N-TERMINAL CARBAMOYL OR ACYL GROUPS

The present invention is concerned with novel peptides having N-terminal carbamoyl or acyl functional groups, which inhibit the angiotensinogen-cleaving action of the proteolytic enzyme, renin, with pharmaceutical compositions containing the novel peptides of the present invention as active ingredients, with methods of treating renin-associated hypertension, hyperaldosteronism and congestive heart failure, with diagnostic methods which utilize the novel peptides of the present invention, and with methods of preparing the novel peptides of the present invention.

## BACKGROUND OF THE INVENTION

Renin is an aspartic proteinase (molecular weight about 40,000) produced and secreted by the juxtaglomerular cells of the kidney. Renin has a high specificity for and cleaves the naturally-occurring plasma glycoprotein, angiotensinogen, at only the 10, 11 peptide bond, i.e., between the 10th (Leu) and 11th (Leu) amino acid residues in the equine substrate, as described by Skeggs et al, J. Exper. Med. 1957, 106, 439, or between Leu 10 and Val 11 in the human renin substrate, as elucidated by Tewksbury et al., Circulation 59, 60, Supp. II: 132, Oct. 1979.

This cleavage of its protein substrate, angiotensinogen, by the endopeptidase, renin, splits off the decapeptide, angiotensin I, which itself is thought to be hemodynamically-inactive, but which is converted in the lungs, kidneys or other tissue by the peptidase, angiotensin-converting enzyme (ACE), to the potent vasopressor octapeptide, angiotensin II. Thus released, the hormone, angiotensin II, then causes constriction of the arterioles and is also believed to stimulate release of the sodium-retaining hormone, aldosterone, from the adrenal cortex, thereby causing a rise in extracellular fluid volume. Accordingly, the renin-angiotensin system plays an important role in normal cardiovascular homeostasis and in some forms of elevated blood pressure (hypertension).

Inhibitors of angiotensin I converting enzyme have proven useful in the modulation of the renin-angiotensin system. Consequently, specific inhibitors of the catalytic and rate-limiting enzymatic step that ultimately regulates angiotensin II production, the action of renin on its substrate, have also been sought as effective investigative tools, as well as therapeutic agents in the treatment of hypertension and congestive heart failure.

Renin antibody, pepstatin, phospholipids, and substrate analogs, including tetrapeptides and octa- to tridecapeptides, with inhibition constants ($K_i$) in the $10^{-3}$ to $10^{-6}$M region, have been studied.

Umezawa et al., in J. Antibiot. (Tokyo) 23: 259-262, 1970, reported the isolation of a peptide, pepstatin, from actinomyces that was an inhibitor of aspartyl proteases such as pepsin, cathepsin D, and renin. Gross et al., Science 175:656, 1972, reported that pepstatin reduces blood pressure in vivo after the injection of hog renin into nephrectomized rats. However, pepstatin has not found very wide application as an experimental agent because of its limited solubility and its inhibition of a variety of other acid proteases in addition to renin.

Many efforts have been made to prepare a specific renin inhibitor based on pig renin substrate analogy, since such analogy has been shown to correlate well with and predict human renin inhibitor activity. The octapeptide amino acid sequence extending from histidine-6 through tyrosine-13

**6 7 8 9 10 11 12 13**
**(-His-Pro-Phe-His-Leu-Leu-Val-Tyr-)**

has been shown to have kinetic parameters essentially the same as those of the full tetradecapeptide renin substrate.

Kokubu et al., Biochem. Pharmacol., 22, 3217-3223, 1973, synthesized a number of analogs of the tetrapeptide found between residues 10 to 13, but while inhibition could be shown, inhibitory constants were only of the order of $10^{-3}$M. Analogs of a larger segment of renin substrate have been also synthesized, e.g., Burton et al., Biochemistry 14: 3892-3898, 1975, and Poulsen et al., Biochemistry 12: 3877-3882, 1973, but a lack of solubility and weak binding (large inhibitory constant) generally resulted.

Modifications to increase solubility soon established that the inhibitory properties of the peptides are markedly dependent on the hydrophobicity of various amino acid residues. These modifications also established that increasing solubility by replacing lipophilic amino acids with hydrophilic isosteric residues

can become counter-productive. Other approaches to increasing solubility have also had limited success.

Modifications designed to increase binding to renin have also been made, but here too, with mixed results.

A series of inhibitors of renin have been disclosed which contain the unnatural amino acid, statine or its analogs: see, e.g., Veber et al, U.S. Patents 4,384,994 and 4,478,826; Evans et al, U.S. Patent 4,397,786; Boger et al, Nature, 1983, 303, 81-84 and U.S. Patents 4,470,971; 4,485,099; 4,663,310 and 4,668,770; Matsueda et al, EP-A 128 762, 152 255; Morisawa et al., EP-A 186 977; Riniker et al, EP-A 111 266; Bindra et al, EP-A 155 809; Stein et al, Fed. Proc. 1986, 45, 869; and Hölzemann et al, German Offenlegungsschrift DE 3438545. Attempting to explain the effect of statine, Powers et al., in Acid Proteases, Structure, Function and Biology, Plenum Press, 1977, 141-157, observed that in pepstatin, statine occupies the space of the two amino acids on either side of the cleavage site of a pepsin substrate and Tang et al., in Trends in Biochem Sci., 1:205-208 (1976) and J. Biol. Chem., 251:7088-94, 1976, suggested that the statine residue of pepstatin resembles the transition state for pepsin hydrolysis of peptide bonds.

Renin inhibitors containing other peptide bond isosteres, including a reduced carbonyl isostere, have been disclosed by M. Szelke et al, in work described in published European Patent Applications 45 665 and 104 041; in U.S. Patent 4,424,207, and in PCT Int. APPl. WO 84/03044; in Nature, 299, 555 (1982); Hypertension, 4, Supp. 2, 59, 1981; and British Patent 1,587,809. In Peptides, Structure and Function: Proceedings of the Eighth American Peptide Symposium, ed. V. J. Hruby and D. H. Rich, p. 579, Pierce Chemical Co., Rockford, IL., 1983, Szelke et al also showed isosteric substitutions at the Leu-Leu site of cleavage, resulting in compounds with excellent potency. These and other peptide bond isosteres have also been disclosed in Buhlmayer et al in EP-A 144 290 and 184 550; Hester et al, EP-A 173 481; Raddatz, EP-A 161 588; Dann et al, Biochem. Biophys. Res. Commun. 1986, 134, 71-77; Fuhrer et al, EP-A 143 746; Kamijo et al, EP-A 181 110; Thaisrivongs et al, J. Med. Chem., 1985, 28, 1553-1555; Ryono et al., EP-A 181 071; and Evans et al, U.S. Patent 4,609,641.

Other modifications which have been tried include preparing renin inhibitors with non-peptide C-termini, such as disclosed in European Published Applications 172 346 and 172 347; Evans et al, J. Med. Chem., 1985, 28, 1755-1756; and Bock et al, Peptides, Structure and Function: Proceedings of the Ninth American Peptide Symposium, ed. C. M. Deber et al, pp.751-754, Pierce Chemical Co., Rockford, IL, 1985. Kokubu et al, in Hypertension, 1985, 7, Suppl. I, p. 8-10 and Matsueda et al, in Chemistry Letters, 1985, 1041-1044 and in European Published Applications 128 762 and 152 255 disclosed peptide aldehyde renin inhibitors, and Hanson et al in Biochem. Biophys. Res. Commun. 1985, 132, 155-161, reported peptide glycol inhibitors.

There have been very general descriptions of N-terminal modifications which have included within their broad scope some of the carbamoyl and acyl functions of the present invention. However, these descriptions have all failed to single out said functions of the present invention from among the extensive general recitals in which they are found; and these descriptions have all failed as well to appreciate the surprising improvement in preperties which can be achieved with the renin-inhibitory compounds of the present invention. Typical of such descriptions are those found in DE 3601248 A1, EP-A 0 239 874, and EP-A 0 255 082, all assigned to Hoechst AG.

These various renin inhibitors all generally comprise peptide-based inhibitors in which a sequence of the type: ...A-B-D-E-F-G-J-K-L... , where G is a peptide bond mimic and A,B,D,E,F,J,K, and L may individually be absent or may represent naturally-occuring or modified amino acids. Typical sequences of this type include:

**7 8 9 10 11 12**
**...BOC-Pro-Phe-His-Sta-Leu-Phe... ,**

or

**8 9 10 11**
**...BOC-Phe-His-Sta-Leu... ,**

where the N-terminus typically comprises an amino acid protecting group such as BOC or CBZ, and the N-terminal amino acids are Pro-Phe-His or Phe-His.

It was an object of this invention to prepare potent, lower molecular weight renin-inhibitory peptides which have improved water solubility and oral bioavailability. It was a further object of this invention to prepare peptides comprising modifications at the C- and N-terminii of a shortened peptide. It was still a

further object of this invention to prepare novel peptides which more effectively inhibit the activity of renin and thereby are more useful antihypertensive agents, and are also compounds useful in treating congestive heart failure.

## DETAILED DESCRIPTION OF THE INVENTION

The present invention discloses renin-inhibitory tripeptides having carbamoyl and acyl functions at the N-termini, which have the formula:

A-B-E-G-J,

wherein:

A  is X′-(alkyl)- and bound to the "B" moiety by a linkage comprising carbonyl:

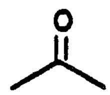

or oxycarbonyl:

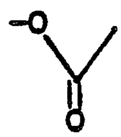

wherein:

(alkyl) is a 2 to 4 carbon chain in which 1 or 2 of the carbon atoms can each be mono- or disubstituted by $C_1$-$C_2$ alkyl;

X′ is independently selected from

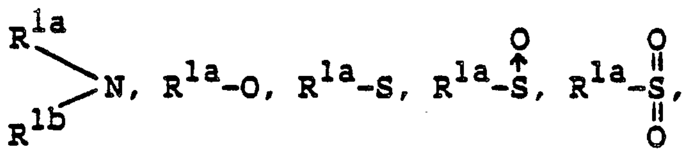

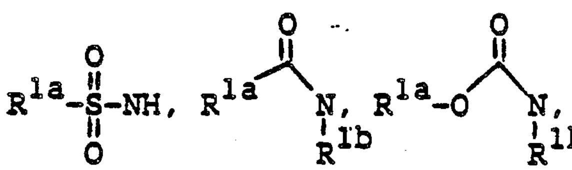

and

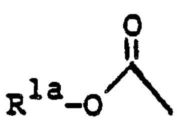

,

where $R^{1a}$ is hydrogen; $C_1$-$C_6$ alkyl, optionally substituted by amino, mono- or di-$C_1$-$C_4$-alkylamino; $C_3$-$C_6$ cycloalkyl; $C_3$-$C_6$ cycloalkyl $C_1$-$C_4$ alkyl; or heterocyclyl or heterocyclyl-$C_1$-$C_4$ alkyl where the heterocyclyl group is selected from pyrrolidine, pyrazoli dine, piperidine, and piperazine, and the heterocyclyl group is optionally monosubstituted by $C_1$-$C_4$ alkyl; and

$R^{1b}$ is hydrogen or $C_1$-$C_4$ alkyl;

B  is

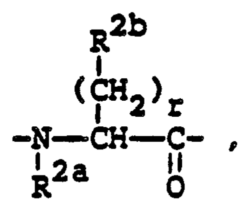

where

r is 1-to-4;

$R^{2a}$ is hydrogen or $C_1$-$C_4$ alkyl; and

$R^{2b}$ is hydrogen; indolyl; or aryl, wherein aryl is unsubstituted or mono- or disubstituted phenyl or naphthyl, where the substituent(s) is/are independently selected from the group consisting of $C_1$-$C_4$-alkyl, amino, mono- or di-$C_1$-$C_4$-alkylamino, amino-$C_1$-$C_4$-alkyl, hydroxy-$C_1$-$C_4$-alkylphenyl-$C_1$-$C_4$-alkyl, mono- or di-$C_1$-$C_4$-alkylamino-$C_1$-$C_4$-alkyl, guanidyl, guanidyl-$C_1$-$C_4$-alkyl, hydroxyl, $C_1$-$C_4$-alkoxy, $CF_3$, halo, CHO, $CO_2H$, $CO_2$-$C_1$-$C_4$-alkyl, and $CO_2$-$C_1$-$C_4$-alkoxy;

or

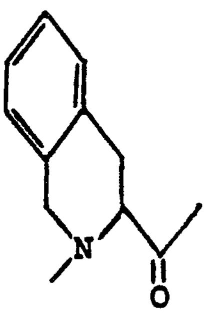

;

Preferably, r is 1 or 2; and $R^3$ is aryl substituted by $C_1$-$C_4$ alkyl, $C_1$-$C_4$ alkoxy, hydroxyl, halo, or $CF_3$;

E is

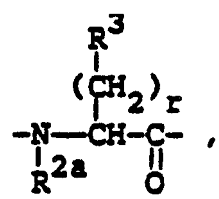

where

r is as defined above; and

$R^3$ is hydrogen; aryl, as defined above; Het, wherein Het is an unsubstituted or mono- or disubstituted 5- to 7-membered mono- or 7- to 10-membered bicyclic heterocyclic ring, where there are one or two heteroatoms which are independently selected from the group consisting of N, O, S, NO, SO, $SO_2$ and quaternized. N, and the substituent(s) is/are independently selected from the group consisting of hydroxyl, thiol, $C_1$-$C_6$-alkyl, $CF_3$, $C_1$-$C_4$-alkoxy, halo, aryl, as defined above, aryl-$C_1$-$C_4$-alkyl, amino, mono- or di-$C_1$-$C_4$-alkylamino, amino-$C_1$-$C_4$-alkylamino, amino-$C_1$-$C_4$-alkyl, hydroxyl-$C_1$-$C_4$-alkyl, di-$C_1$-$C_4$-alkylamino-$C_1$-$C_4$-alkyl, guanidyl, guanidyl-$C_1$-$C_4$-alkyl, CHO, $CO_2H$, and $CO_2$-$C_1$-$C_4$-alkyl; or unsubstituted or mono- or disubstituted $C_1$-$C_4$-alkyl, wherein the substituent(s) is/are on the terminal carbon atom(s) and is/are chosen from the group consisting of $-CO_2R^{2a}$ and $-CONR^{2a}R^{2b}$, where $R^{2a}$ is as defined above and $R^{2b}$ has the same definition as $R^{2a}$ but is independently selected therefrom; and when r is 2, 3 or 4, alternatively $R^3$ is $C_1$-$C_4$-alkoxy, mono- or di-$C_1$-$C_4$-alkyl, $NH_2$, or OH;

G is

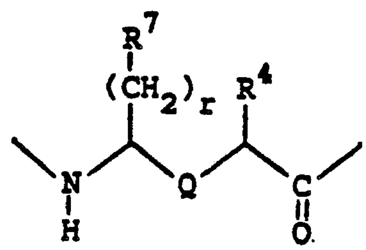

where

$R^4$ is hydrogen; $C_1$-$C_6$-alkyl; $C_2$-$C_6$-alkenyl; mono- or disubstituted $C_2$-$C_6$-alkyl, wherein the substituent(s) is/are on the final 1 or/and 2 carbcn atoms of the alkyl chain and is/are independently selected from the group consisting of hydroxyl carboxy, $CO_2$-$C_1$-$C_4$-alkyl, amino, mono- or di-$C_1$-$C_4$-alkylamino, aryl, as defined above, and unsubstituted or mono- or disubstituted $C_3$-$C_7$-cycloalkyl, wherein the substituent(s) is/are independently selected from the group consisting of $C_1$-$C_4$-alkyl, trifluoromethyl, hydroxy, $C_1$-$C_4$-alkoxy, and halo; $R^7$ is hydrogen; $C_3$-$C_6$-alkyl; aryl, as defined above; or unsubstituted or mono- or disubstituted $C_3$-$C_7$-cycloalkyl, wherein the substituent(s) is/are independently selected from the group consisting of $C_1$-$C_4$-alkyl, trifluoromethyl, hydroxy, $C_1$-$C_4$-alkoxy, and halo;

Q is

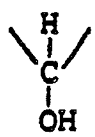 or 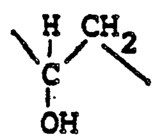

and

r is as defined above.

Preferably, r is 1; and $R^4$ is $C_1$-$C_4$ alkyl, $C_2$-$C_4$ alkenyl, or $C_2$-$C_4$ alkyl monosubstituted by hydroxyl, amino, or mono-or di-$C_1$-$C_4$ alkylamino; and

J is $-Y-(CH_2)_x-[CH(R^5)]_y-(CH_2)_z-R^{10}$

Y is O, NH, or N-$C_1$-$C_4$ alkyl;

x is 0 to 1;

y is 0 to 1;

z is 0 to 4;

$R^5$ is as defined above;

$R^{10}$ is hydrogen; -OH; aryl as defined above; Het, as defined above; $-NH_2$; $NR^{17}R^{18}$; $NHR^{18}$; $-N(R^{17}R^{18}R^{19})^+A^-$, where $R^{17}$ and $R^{19}$ are independently $C_1$-$C_4$ alkyl, $R^{18}$ is aryl, Het or $C_1$-$C_4$ alkyl optionally substituted with a substi tuent chosen from the group consisting of aryl, Het, OH, $-NH_2$, -NH-$C_1$-$C_4$ alkyl, -N($C_1$-$C_4$ alkyl)$_2$, $-CO_2H$, $-CO_2$-$C_1$-$C_4$ alkyl, $SO_3H$, -CO-NH-$SO_2$-$C_1$-$C_4$ alkyl, or -CO-NH-$SO_2$ aryl, and $A^-$ is a counterion selected from the group consisting of small, single negatively charged ions;

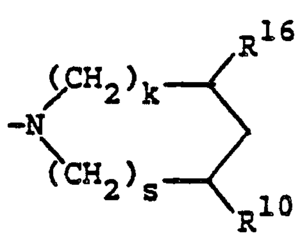

where k is 1 or 2; s is 0 or 1; $R^{16}$ is -H, -OH, $C_1$-$C_4$ alkyl, aryl, or aryloxy; and $R^{10}$ is as defined above;

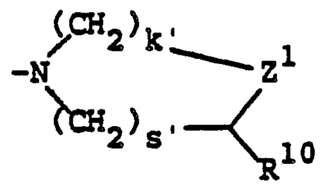

where k′ is 2 or 3; s′ is 0 or 1; $R^{10}$ is as defined above; and $Z^1$ is O, S, SO, $SO_2$, NH;

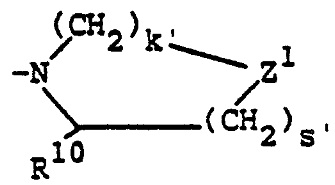

where k′, s′, $R^{10}$, and $Z^1$ are as defined above;

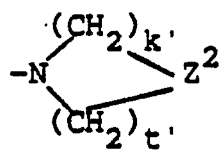

where t′ is 2 or 3; k′ is as defined above, and $Z^2$ is $NR^{18}$ or $N(R^{17}R^{18})^+A^-$, where $R^{17}$, $R^{18}$ and $A^-$ are as defined above;
and pharmaceutically-acceptable salts thereof.

In the peptides of the present invention, the A, B, E, G and J components have asymmetric centers and occur as racemates, racemic mixtures and as individual diastereomers, with all isomeric forms generally being included in the present invention.
In particular, asymmetric carbon atoms in the G substituent has either an S or an R configuration, except as noted in one alternate definition of G above, and all other asymmetric carbon atoms have an S configuration, with preferred chiralities indicated in the following further description.

When any variable (e.g., aryl, Het, $R^{1a}$, $R^{1b}$, $R^5$, $R^6$, $R^7$, $A^-$, r, etc.) occurs more than one time in any constituent or in formula I, its definition on each ocurrence is independent of its definition at every other occurrence.

As used herein, "alkyl" is intended to include both branched- and straight-chain saturated aliphatic hydrocarbon groups having the specified number of carbon atoms (Me is methyl, Et is ethyl, Pr is propyl, Bu is butyl); "alkoxy" represents an alkyl group of indicated number of carbon atoms attached through an oxygen bridge; and "$C_3$-$C_7$-cycloalkyl" is intended to include saturated ring groups, such as cyclopropyl, cyclobutyl, cyclopentyl, cyclohexyl (Cyh) and cycloheptyl. "Alkanoyl" is intended to include those alkylcarbonyl groups of specified number of carbon atoms, which are exemplified by formyl, acetyl, propanoyl and butanoyl; "alkenyl" is intended to include hydrocarbon chains groups of either a straight- or branched-configuration and one unsaturation, which may occur at any point along the chain, such as ethenyl, propenyl, butenyl, pentenyl, and the like, and includes E and Z forms, where applicable; and "arylalkyl" represents aryl groups as herein defined which are attached through a straight-or branched- chain alkyl group of specified number of carbon atoms, such as, for example, benzyl, phenethyl, 3,3-diphenylpropyl, 3-indolymethyl, and the like. "Halo", as used herein, means fluoro, chloro, bromo and iodo, and "counterion" is used to represent a small, single negatively-charged specie, such as chloride, bromide, hydroxide, acetate, perchlorate, benzoate, maleate, benzene sulfonate, tartrate, hemitartrate, and the like.

As used herein, with exceptions as noted, "aryl" is intended to mean phenyl (Ph) or naphthyl, which is optionally-substituted with one or two members independently selected from the group consisting of $C_1$-$C_4$ alkyl, amino, mono- or di-$C_1$-$C_4$-alkylamino, amino-$C_1$-$C_4$-alkyl, hydroxy-$C_1$-$C_4$-alkyl, phenyl-$C_1$-$C_4$-alkyl, mono- or di-$C_1$-$C_4$-alkylamino-$C_1$-$C_4$-alkyl, guanidyl, guanidyl-$C_1$-$C_4$-alkyl, hydroxyl, $C_1$-$C_4$-alkoxy, $CF_3$, halo, CHO, $CO_2H$, $CO_2$-$C_1$-$C_4$ alkyl, and $CO_2$-$C_1$-$C_4$ alkoxy. "Aroyl" is intended to include those aryl-carbonyl groups which are exemplified by benzoyl and naphthoyl.

The term "Het", as used herein, represents an unsubstituted or mono- or disubstituted 5- to 7-membered mono- or 7- to 10-membered bicyclic heterocyclic ring, where there are one or two heteroatoms which are independently selected from the group consisting of N, O, S, NO, SO, $SO_2$ and quaternized N, and the substituent(s) is/are independently selected from the group consisting of hydroxyl, thiol, $C_1$-$C_6$-alkyl, $CF_3$, $C_1$-$C_4$-alkoxy, halo, aryl, as defined above, aryl-$C_1$-$C_4$-alkyl, amino, mono- or di-$C_1$-$C_4$-alkylamino, amino-$C_1$-$C_4$-alkylamino, amino-$C_1$-$C_4$-alkyl, hydroxy-$C_1$-$C_4$-alkyl, di-$C_1$-$C_4$-alkylamino-$C_1$-$C_4$-alkyl, guanidyl,, guanidyl-$C_1$-$C_4$-alkyl, CHO, $CO_2H$, and $CO_2$-$C_1$-$C_4$-alkyl; or unsubstituted or mono- or disubstituted $C_1$-$C_4$-alkyl, wherein the substituent(s) is/are on the terminal carbon atom(s) and is/are chosen from the group consisting of $-CO_2R^{2a}$ and $-CONR^{2a}R^{2b}$, where $R^{2a}$ is as defined above and $R^{2b}$ has the same definition as $R^{2a}$ but is independently selected therefrom; and when r is 2, 3 or 4, alternatively $R^3$ is $C_1$-$C_4$-alkoxy, mono- or di-$C_1$-$C_4$-alkyl, $-NH_2$, or -OH.

The following additional abbreviations have also been used herein:

| Abbreviated Designation | Amino Acid/Residue |
|---|---|
| ACHPA | (3<u>S</u>,4<u>S</u>)-4-amino-5-cyclohexyl-3-hydroxypentanoic acid |
| Ala | D- or L-alanine |
| Arg | D- or L-arginine |
| Cal (Cha) | ß-cyclohexylalanine |
| Cys | D- or L-cysteine |
| Gly | glycine |
| His | D- or L-histidine |
| HPhe (HomoPhe) | D- or L-homologated phenylalanine |
| HomoACHPA | homologated ACHPA |
| Ile | D- or L-isoleucine |
| Leu | D- or L-leucine |
| Lys | D- or L-lysine |
| Met | D- or L-methionine |
| Nle | D- or L-norleucine |
| Nva | D- or L-norvaline |

| | |
|---|---|
| Orn | D- or L-ornithine |
| Phe | D- or L-phenylalanine |
| Ser | D- or L-serine |
| Sta | statine, (3S,4S)-4-amino-3-hydroxy-6-methylheptanoic acid |
| Thr | D- or L-threonine |
| Trp | L-tryptophan |
| Tyr | L-tyrosine |
| | **Protecting Group** |
| BOC (Boc) | t-butyloxycarbonyl |
| BOM | benzyloxymethyl |
| CBZ (Cbz) (Z) | benzyloxycarbonyl(carbobenzoxy) |
| DNP | 2,4-dinitrophenyl |
| EtOC | ethoxycarbonyl |
| IPOC | isopropoxycarbonyl |
| OMe | methoxy, except when it immediately follows an amino acid residue abbreviation, and it represents methyl ester |
| TBDMS | t-butyldimethylsilyl |
| | **Activating Group** |
| HBT(HOBt) | N-hydroxybenzotriazole |
| | **Condensing Agent** |
| DCCI (DCC) | N,N'-dicyclohexylcarbodiimide |
| | **Reagent** |
| $Et_3N$ (TEA) | triethylamine |
| $i-Pr_2NEt$ | diisopropylethylamine |
| TFA | trifluoroacetic acid |

| | Coupling Reagents |
|---|---|
| BOP reagent | benzotriazol-1-yloxytris(dimethyl-amino)phosphonium hexafluoro-phosphate |
| BOP-Cl | bis(2-oxo-3-oxazolidinyl)phosphinic cloride |
| | **Solvent** |
| AcOH (HOAc) | acetic acid |
| $CH_2Cl_2$ | methylene chloride |
| DMF | dimethylformamide |

The novel renin inhibitory peptides of the present invention may alternately be described in terms of common amino acid components and closely-related analogs thereof, wherein, in accordance with the usages of formula I:

A, G and J are as defined under Formula I;

B is Phe, Homophe, Tyr, HomoTyr, Trp, or HomoTrp; and

E is Ala, Leu, Ser, Thr, Phe, Tyr, Trp, His, (N-methyl)His, Lys, Orn, Arg, Cys, Nle, (N-methyl)Nle, Nva, or Met.

In the peptides of the instant invention, the double amino acid sequence of the endogenous human substrate, $Leu^{10}$-$Val^{11}$, is replaced by a G component statine analog/residue (particularly ACHPA or Cal[CH-(OH)$CH_2$]Val). This substitution results in an increase in metabolic stability and a better fit to the renin enzyme.

Also in these novel shortened peptides, amino-terminal substituted carbamoyl or acyl moieties are incorporated, which substantially improve water solubility and oral bioavailability of the renin inhibitory peptides. These peptides are therefore of lower molecular weight and different structure, with an enhanced potency and duration of action, than known peptides of this class.

It will be understood that closely-related analogs of the above common amino acids, for example, aliphatic amino acids in addition to Ala, Val, Leu, and Ile, such as α-aminobutyric acid (Abu), and substituted phenyl derivatives of Phe, are included in the broad description of the novel inhibitory peptides of the present invention represented by Formula I and related definitions.

Representative preferred renin-inhibitory peptides of the present invention include the following:

N-(2-t-butylsulfonylethyloxycarbonyl)-Phe-His-ACHPA-NH-2(S)methylbutyl;
N-(3-t-butylsulfonylpropyloxycarbonyl)-Phe-His-ACHPA-NH-2(S)methylbutyl;
N-(4-t-butylsulfonylbutyryl)-Phe-His-ACHPA-NH-2(S)methylbutyl;
N-(2-t-butylsulfonamidoethyloxycarbonyl)-Phe-His-ACHPA-NH-2(S)methylbutyl;
N-(3-t-butylsulfonamidopropyloxycarbonyl)-Phe-His-ACHPA-NH-2(S)methylbutyl;
N-(4-t-butylsulfonamidobutyryl)-Phe-His-ACHPA-NH-2(S)methylbutyl;
N-(2-ethoxycarbonylaminoethoxycarbonyl)-Phe-His-ACHPA-NH-2(S)methylbutyl;
N-(3-ethoxycarbonylaminopropyloxycarbonyl)-Phe-His-ACHPA-NH-2(S)methylbutyl;
N-(4-ethoxycarbonylaminobutyryl)-Phe-His-ACHPA-NH-2(S)-methylbutyl;
N-(2-t-butoxycarbonylaminoethoxycarbonyl)Phe-His-ACHPA-NH-2(S)methylbutyl;
N-(4-t-butoxycarbonylaminobutyryl)-Phe-His-ACHPA-NH-2(S)methylbutyl;
N-(3-t-butoxycarbonylaminopropyloxycarbonyl)-Phe-His-ACHPA-NH-2(S)methylbutyl;
N-(2-t-butylacetamidoethoxycarbonyl)-Phe-His-ACHPA-NH-2(S)methylbutyl;
N-(3-t-butylacetamidopropyloxycarbonyl)-Phe-His-ACHPA-NH-2(S)methylbutyl;
N-(4-t-butylacetamidobutyryl)-Phe-His-ACHPA-NH-2(S)methylbutyl;
N-[2-(3-amino-3-methylbutyramidoethoxycarbonyl]-Phe-His-ACHPA-NH-2(S)methylbutyl;
N-[3-(3-amino-3-methylbutyramidopropyloxycarbonyl]-Phe-His-ACHPA-NH-2(S)methylbutyl;
N-[4-(3-amino-3-methylbutyramidobutyryl]-Phe-His-ACHPA-NH-2(S)methylbutyl;
N-(2-L-prolinamidoethoxycarbonyl)-Phe-His-ACHPA-NH-2(S)methylbutyl;

N-(3-L-prolinamidopropyloxycarbonyl)-Phe-His-ACHPA-NH-2(S)methylbutyl;
N-(4-L-prolinamidobutyryl)-Phe-His-ACHPA-NH-2(S)methyl-butyl;
N-(piperidine-4-ethoxycarbonyl)-Phe-His-ACHPA-NH-2(S)methylbutyl;
N-[2-(piperidine-3-methoxycarbonylamino)ethoxycarbonyl]-Phe-His-ACHPA-NH-2(S)methylbutyl;
N-[3-(piperidine-3-methoxycarbonylamino)propyloxycarbonyl]-Phe-His-ACHPA-NH-2(S)methylbutyl;
N-[4-(piperidine-3-methoxycarbonylamino)butyryl]-Phe-His-ACHPA-NH-2(S)methylbutyl;
N-[2-(pyrrolidine-2-methoxycarbonyl)ethyoxycarbonyl]-Phe-His-ACHPA-NH-2(S)methylbutyl;
N-[3-(pyrrolidine-2-methoxycarbonyl)propyloxycarbonyl]-Phe-His-ACHPA-NH-2(S)methylbutyl;
N-[4-(pyrrolidine-2-methoxycarbonyl)butyryl]-Phe-His-ACHPA-NH-2(S)methylbutyl;
N-(2-t-butylsulfonylethyloxycarbonyl)-Phe-His-ACHPA-NH-(N-methyl-3-aminoquinuclidinyl) acetate;
N-(3-t-butylsulfonylpropyloxycarbonyl)-Phe-His-ACHPA-NH-(N-methyl-3-aminoquinuclidinyl) acetate;
N-(4-t-butylsulfonylbutyryl)-Phe-His-ACHPA-NH-(N-methyl-3-aminoquinuclidinyl) acetate;
N-(2-t-butylsulfonamidoethyloxycarbonyl)-Phe-His-ACHPA-NH-(N-methyl-3-aminoquinuclidinyl) acetate;
N-(3-t-butylsulfonamidopropyloxycarbonyl)-Phe-His-ACHPA-NH-(N-methyl-3-aminoquinuclidinyl) acetate;
N-(4-t-butylsulfonamidobutyryl)-Phe-His-ACHPA-NH-(N-methyl-3-aminoquinuclidinyl) acetate;
N-(2-ethoxycarbonylaminoethoxycarbonyl)-Phe-His-ACHPA-NH-(N-methyl-3-aminoquinuclidinyl) acetate;
N-(3-ethoxycarbonylaminopropyloxycarbonyl)-Phe His-ACHPA-NH-(N-methyl-3-aminoquinuclidinyl) acetate;
N-(4-ethoxycarbonylaminobutyryl)-Phe-His-ACHPA-NH-(N-methyl-3-aminoquinuclidinyl) acetate;
N-(2-t-butoxycarbonylaminoethoxycarbonyl)-Phe-His-ACHPA-NH-(N-methyl-3-aminoquinuclidinyl) acetate;
N-(4-t-butoxycarbonylaminobutyryl)-Phe-His-ACHPA-NH-(N-methyl-3-aminoquinuclidinyl) acetate;
N-(3-t-butoxycarbonylaminopropyloxycarbonyl)-Phe-His-ACHPA-NH-(N-methyl-3-aminoquinuclidinyl) acetate;
N-(2-t-butylacetamidoethoxycarbonyl)-Phe-His-ACHPA-NH-(N-methyl-3-aminoquinuclidinyl) acetate;
N-(3-t-butylacetamidopropyloxycarbonyl)-Phe-His-ACHPA-NH-(N-methyl-3-aminoquinuclidinyl) acetate;
N-(4-t-butylacetamidobutyryl)-Phe-His-ACHPA-NH-NH-(N-methyl-3-aminoquinuclidinyl) acetate;
N-[2-(3-amino-3-methylbutyramidoethoxycarbonyl]-Phe-His-ACHPA-NH-(N-methyl-3-aminoquinuclidinyl) acetate;
N-[3-(3-amino-3-methylbutyramidopropyloxycarbonyl]-Phe-His-ACHPA-NH-(N-methyl-3-aminoquinuclidinyl) acetate;
N-[4-(3-amino-3-methylbutyramidobutyryl]-Phe-His-ACHPA-NH-(N-methyl-3-aminoquinuclidinyl) acetate;
N-(2-L-prolinamidoethoxycarbonyl)-Phe-His-ACHPA-NH-(N-methyl-3-aminoquinuclidinyl) acetate;
N-(3-L-prolinamidopropyloxycarbonyl)-Phe His-ACHPA-NH-(N-methyl-3-aminoquinuclidinyl) acetate;
N-(4-L-prolinamidobutyryl)-Phe-His-ACHPA-NH-(N-methyl-3-aminoquinuclidinyl) acetate;
N-[2-(piperidine-3-methoxycarbonylamino)ethyoxycarbonyl]-Phe-His-ACHPA-NH-(N-methyl-3-aminoquinuclidinyl) acetate;
N-2-(pyrrolidine-2-methoxycarbonyl)-Phe-His-ACHPA-NH-(N-methyl-3-aminoquinuclidinyl) acetate;
N-4-(piperidine-3-methoxycarbonyl)-Phe-His-ACHPA-NH-(N-methyl-3-aminoquinuclidinyl) acetate;
N-[3-(piperidine-3-methoxycarbonylamino)propyloxycarbonyl]-Phe-His-ACHPA-NH-(N-methyl-3-aminoquinuclidinyl) acetate;
N-[4-(piperidine-3-methoxycarbonylamino)butyryl]-Phe-His-ACHPA-NH-(N-methyl-3-aminoquinuclidinyl) acetate;
N-[2-(pyrrolidine-2-methoxycarbonyl)ethyoxycarbonyl]-Phe-His-ACHPA-NH-(N-methyl-3-aminoquinuclidinyl) acetate;
N-[3-(pyrrolidine-2-methoxycarbonyl)propyloxycarbonyl]-Phe-His-ACHPA-NH-(N-methyl-3-aminoquinuclidinyl) acetate;
N-[4-(pyrrolidine-2-methoxycarbonyl)butyryl]-Phe-His-ACHPA-NH-(N-methyl-3-aminoquinuclidinyl)acetate
N-(2-t-butylsulfonylethyloxycarbonyl)-Phe-His-Cal[CH(OH)$CH_2$]Val-NH$(CH_2)_3CH_3$;
N-(3-t-butylsulfonylpropyloxycarbonyl)-Phe-His-Cal[CH(OH)$CH_2$]Val-NH$(CH_2)_3CH_3$
N-(4-t-butylsulfonylbutyryl)-Phe-His-Cal[CH(OH)$CH_2$]-Val-NH$(CH_2)_3CH_3$;
N-(2-t-butylsulfonamidoethyloxycarbonyl)-Phe-His-Cal[CH(OH)$CH_2$]Val-NH$(CH_2)_3CH_3$;
N-(3-t-butylsulfonamidopropyloxycarbonyl)-Phe-His Cal[CH(OH)$CH_2$]Val-NH$(CH_2)_3CH_3$;
N-(4-t-butylsulfonamidobutyryl)-Phe-His--Cal[CH(OH)$CH_2$]Val-NH$(CH_2)_3CH_3$;
N-(2-ethoxycarbonylaminoethoxycarbonyl)-Phe-His-Cal[CH-(OH)$CH_2$]Val-NH$(CH_2)_3CH_3$;
N-(3-ethoxycarbonylaminopropyloxycarbonyl)-Phe-His-Cal[CH(OH)$CH_2$]Val-NH$(CH_2)_3CH_3$;
N-(4-ethoxycarbonylaminobutyryl)-Phe-His-Cal[CH(OH)$CH_2$]Val-NH$(CH_2)_3CH_3$;
N-(2-t-butoxycarbonylaminoethoxycarbonyl)-Phe-His-Cal[CH(OH)$CH_2$]Val-NH$(CH_2)_3CH_3$;
N-(4-t-butoxycarbonylaminobutyryl)-Phe-His-Cal[CH(OH)-$CH_2$]Val-NH$(CH_2)_3CH_3$;
N-(3-t-butoxycarbonylaminopropyloxycarbonyl)-Phe-His-Cal[CH(OH)$CH_2$]Val-NH($CH_2CH_3$;

N-(2-t-butylacetamidoethoxycarbonyl)-Phe-His-Cal[$CH(OH)CH_2$]Val-$NH(CH_2)_3CH_3$;
N-(3-t-butylacetamidopropyloxycarbonyl)-Phe-His-ACHPA-Cal[$CH(OH)CH_2$]Val-$NH(CH_2)_3CH_3$);
N-(4-t-butylacetamidobutyryl)-Phe-His-Cal[$CH(OH)CH_2$]Val-$NH(CH_2)_3CH_3$);
N-[2-(3-amino-3-methylbutyramidoethoxycarbonyl]-Phe-His-Cal[$CH(OH)CH_2$]Val-$NH(CH_2)_3CH_3$);
N-[3-(3-amino-3-methylbutyramidopropyloxycarbonyl]-Phe-His-Cal[$CH(OH)CH_2$]Val-$NH(CH_2)_3CH_3$;
N-[4-(2-amino-2-methyl)propyloxycarbonylaminobutyryl]-Phe-His-Cal[$CH(OH)CH_2$]Val-$NH(CH_2)_3CH_3$;
N-(2-L-prolinamidoethoxycarbonyl)-Phe-His-Cal[$CH(OH)CH_2$]Val-$NH(CH_2)_3CH_3$;
N-(3-L-prolinamidopropyloxycarbonyl)-Phe-His-Cal[$CH(OH)CH_2$]Val-$NH(CH_2)_3CH_3$;
N-(4-L-prolinamidobutyryl)-Phe-His-Cal[$CH(OH)CH_2$]Val-$NH(CH_2)_3CH_3$;
N-[2-(piperidine-3-methoxycarbonylamino)ethyoxycarbonyl]-Phe-His-Cal[$CH(OH)CH_2$]Val-$NH(CH_2)_3CH_3$;
N-[3-(piperidine-3-methoxycarbonylamino)propyloxycarbonyl]-Phe-His-Cal[$CH(OH)CH_2$]Val-$NH(CH_2)_3CH_3$;
N-4-(piperidine-3-methoxycarbonyl)-Phe-His-Cal[$CH(OH)CH_2$]Val-$NH(CH_2)_3CH_3$;
N-2-(pyrrolidine-2-methoxycarbonyl)Phe-His-Cal[$CH(OH)CH_2$]Val-$NH(CH_2)_3CH_3$;
N-[4-(piperidine-3-methoxycarbonylamino)butyryl]-Phe-His-Cal[$CH(OH)CH_2$]Val-$NH(CH_2)_3CH_3$;
N-[2-(pyrrolidine-2-methoxycarbonyl)ethyoxycarbonyl]-Phe-His-Cal[$CH(OH)CH_2$]Val-$NH(CH_2)_3CH_3$;
N-[3-(pyrrolidine-2-methoxycarbonyl)propyloxycarbonyl]-Phe-His-Cal[$CH(OH)CH_2$]Val-$NH(CH_2)_3CH_3$;
N-[4-(pyrrolidine-2-methoxycarbonyl)butyryl]-Phe-His-Cal[$CH(OH)CH_2$]Val-$NH(CH_2)_3CH_3$
N-(2-t-butylsulfonylethyloxycarbonyl)-Phe-His-ACHPA-$NHCH_2CH(OH)CH_2N(CH_2CH_2)_2O$;
N-(3-t-butylsulfonylpropyloxycarbonyl)-Phe-His-ACHPA-$NHCH_2CH(OH)CH_2N(CH_2CH_2)_2O$;
N-(4-t-butylsulfonylbutyryl)-Phe-His-ACHPANHCH$_2$CH(OH)-$CH_2N(CH_2CH_2)_2O$;
N-(2-t-butylsulfonamidoethyloxycarbonyl)-Phe His-ACHPA-$NHCH_2CH(OH)CH_2N(CH_2CH_2)_2O$;
N-(3 t-butylsulfonamidopropyloxycarbonyl)-Phe-His-ACHPA-$NHCH_2CH(OH)CH_2N(CH_2CH_2)_2O$;
N-(4-t-butylsulfonamidobutyryl)-Phe-His-ACHPA-$NHCH_2$-CH-$(OH)CH_2N(CH_2CH_2)_2O$;
N-(2-ethoxycarbonylaminoethoxycarbonyl)-Phe-His-ACHPA-$NHCH_2CH(OH)CH_2N(CH_2CH_2)_2O$;
N-(3-ethoxycarbonylaminopropyloxycarbonyl)-Phe-His-ACHPA-$NHCH_2CH(OH)CH_2N(CH_2CH_2)_2O$;
N-(4-ethoxycarbonylaminobutyryl)-Phe-His-ACHPA-NH-$CH_2$CH-$(OH)CH_2N(CH_2CH_2)_2O$;
N-(2-t-butoxycarbonylaminoethoxycarbonyl)-Phe-His-ACHPA-$NHCH_2CH(OH)CH_2N(CH_2CH_2)_2O$;
N-(4-t-butoxycarbonylaminobutyryl)-Phe-His-ACHPA-$NHCH_2CH(OH)CH_2N(CH_2CH_2)_2O$;
N-(3-t-butoxycarbonylaminopropyloxycarbonyl)-Phe-His-ACHPA-$NHCH_2CH(OH)CH_2N(CH_2CH_2)_2O$;
N-(2-t-butylacetamidoethoxycarbonyl)-Phe-His-ACHPA-$NHCH_2CH(OH)CH_2N(CH_2CH_2)_2O$;
N-(3-t-butylacetamidopropyloxycarbonyl)-Phe-His-ACHPA-$NHCH_2CH(OH)CH_2N(CH_2CH_2)_2O$;
N-(4-t-butylacetamidobutyryl)-Phe-His-ACHPA-$NHCH_2CH(OH)CH_2N(CH_2CH_2)_2O$;
N-[2-(3-amino-3-methylbutyramidoethoxycarbonyl]-Phe-His-ACHPA-$NHCH_2CH(OH)CH_2N(CH_2CH_2)_2O$;
N-[2-(3-amino-3-methylbutyramidoethoxycarbonyl]-Phe-His-ACHPA-$NHCH_2CH(OH)CH_2N(CH_2CH_2)_2O$;
N-[3-(3-amino-3-methylbutyramidopropyloxycarbonyl]-Phe-His-ACHPA-$NHCH_2CH(OH)CH_2N(CH_2CH_2)_2O$;
N-[4-(3-amino-3-methylbutyramidoaminobutyryl]-Phe-His-ACHPA-$NHCH_2CH(OH)CH_2N(CH_2CH_2)_2O$;
N-(2-L-prolinamidoethoxycarbonyl)-Phe-His-ACHPA-$NHCH_2CH(OH)CH_2N(CH_2CH_2)_2O$;
N-(3-L-prolinamidopropyloxycarbonyl)-Phe-His-ACHPA-$NHCH_2CH(OH)CH_2N(CH_2CH_2)_2O$;
N-(4-L-prolinamidobutyryl)-Phe-His-ACHPA-$NHCH_2CH(OH)$-$CH_2N(CH_2CH_2)_2O$;
N-[2-(piperidine-3-methoxycarbonylamino)ethyoxycarbonyl]-Phe-His-ACHPA-$NHCH_2CH(OH)CH_2N(CH_2CH_2)$-$_2O$;
N-[3-(piperidine-3-methoxycarbonylamino)propyloxycarbonyl]-Phe-His-ACHPA-$NHCH_2CH(OH)CH_2N$-$(CH_2CH_2)_2O$;
N-4-(piperidine-3-methoxycarbonyl)-Phe-His-ACHPA-$NHCH_2CH(OH)CH_2N(CH_2CH_2)_2O$;
N-2-(pyrrolidine-2-methoxycarbonyl)Phe-His-ACHPA-$NHCH_2CH(OH)CH_2N(CH_2CH_2)_2O$;
N-[4-(piperidine-3-methoxycarbonylamino)butyryl]-Phe-His-ACHPA-$NHCH_2CH(OH)CH_2N(CH_2CH_2)_2O$;
N-[2-(pyrrolidine-2-methoxycarbonyl)ethyoxycarbonyl]-Phe-His-ACHPA-$NHCH_2CH(OH)CH_2N(CH_2CH_2)_2O$;
N-[3-(pyrrolidine-2-methoxycarbonyl)propyloxycarbonyl]-Phe-His-ACHPA-$NHCH_2CH(OH)CH_2N(CH_2CH_2)_2O$;
N-[4-(pyrrolidine-2-methoxycarbonyl)butyryl]-Phe-His-ACHPA-$NHCH_2CH(OH)CH_2N(CH_2CH_2)_2O$
N-(2-t-butylsulfonylethyloxycarbonyl)-Phe-Nle-ACHPA-NH-N-[(4-picolyl)-3-aminoquinuclidinyl]acetate;
N-(3-t-butylsulfonylpropyloxycarbonyl)-Phe-Nle-ACHPA-NH-N-[(4-picolyl)-3-aminoquinuclidinyl]acetate;
N-(4-t-butylsulfonylbutyryl)-Phe-Nle-ACHPA-NH-N-[(4-picolyl)-3-aminoquinuclidinyl]acetate;
N-(2-t-butylsulfonamidoethyloxycarbonyl)-Phe-Nle-ACHPA-NH-N-[(4-picolyl)-3-aminoquinuclidinyl]acetate;
N-(3-t-butylsulfonamidopropyloxycarbonyl)-Phe-Nle-ACHPA-NH-N-[(4-picolyl)-3-aminoquinuclidinyl]acetate;
N-(4-t-butylsulfonamidobutyryl)-Phe-Nle-ACHPA-NH-N-[(4-picolyl)-3-aminoquinuclidinyl]acetate;
N-(2-ethoxycarbonylaminoethoxycarbonyl)-Phe-Nle-ACHPA-NH-N-[(4-picolyl)-3-aminoquinuclidinyl]acetate;
N-(3-ethoxycarbonylaminopropyloxycarbonyl)-Phe-Nle-ACHPA-NH-N-[(4-picolyl)-3-aminoquinuclidinyl]-acetate;

N-(4-ethoxycarbonylaminobutyryl)-Phe-Nle-ACHPA-NH-N-[(4-picolyl)-3-aminoquinuclidinyl]acetate;
N-(2-t-butoxycarbonylaminoethoxycarbonyl)-Phe-Nle-ACHPA-NH-N-[(4-picolyl)-3-aminoqinuclidinyl]acetate;
N-(4-t-butoxycarbonylaminobutyryl)-Phe-Nle-ACHPA-NH-N-[(4-picolyl)-3-aminoquinuclidinyl]acetate;
N-(3-t-butoxycarbonylaminopropyloxycarbonyl)-Phe-Nle-ACHPA-NH-N-[(4-picolyl)-3-aminoquinuclidinyl]-acetate;
N-(2-t-butylacetamidoethoxycarbonyl)-Phe-Nle-ACHPA-NH-N-[(4-picolyl)-3-aminoquinuclidinyl]acetate;
N-(3-t-butylacetamidopropyloxycarbonyl)-Phe-Nle-ACHPA-NH-N-[(4-picolyl)-3-aminoquinuclidinyl]acetate;
N-(4-t-butylacetamidobutyryl)-Phe-Nle-ACHPA-NH-N-[(4-picolyl)-3-aminoquinuclidinyl]acetate;
N-[2-(3 amino-3-methylbutyramidoethoxycarbonyl]-Phe-Nle-ACHPA-NH-N-[(4-picolyl)-3-aminoquinuclidinyl]-acetate;
N-[3-(3-amino-3-methylbutyramidopropyloxycarbonyl]-Phe-Nle-ACHPA-NH-N-[(4-picolyl)-3-aminoquinuclidinyl]acetate;
N-[4-(3-amino-3-methylbutyramidobutyryl]-Phe-Nle-ACHPA-NH-N-[(4-picolyl)-3-aminoquinuclidinyl]acetate;
N-(2-L-prolinamidoethoxycarbonyl)-Phe-Nle-ACHPA-NH-N-[(4-picolyl)-3-aminoquinuclidinyl]acetate;
N-(3-L-prolinamidopropyloxycarbonyl)-Phe-Nle-ACHPA-NH-N-[(4-picolyl)-3-aminoquinuclidinyl]acetate;
N-(4-L-prolinamidobutyryl)-Phe-Nle-ACHPA-NH-N-[(4-picolyl)-3-aminoquinuclidinyl]acetate;
N-[2-(piperidine-3-methoxycarbonylamino)ethyoxycarbonyl]-Phe-Nle-ACHPA-NH-N-[(4-picolyl)-3-aminoquinuclidinyl]acetate;
N-4-(piperidine-3-methoxycarbonyl)-Phe-[Nle-ACHPA-NH-N-[(4-picolyl)-3-aminoquinuclidinyl]acetate;
N-[2-(pyrrolidine-2-methoxycarbonyl)-Phe-Nle-ACHPA-NH-N-[(4-picolyl)-3-aminoquinuclidinyl]acetate;
N-[3-(piperidine-3-methoxycarbonylamino)propyloxycarbonyl]-Phe-Nle-ACHPA-NH-N-[(4-picolyl)-3-aminoquinuclidinyl]acetate;
N-[4-(piperidine-3-methoxycarbonylamino)butyryl]-Phe-Nle-ACHPA-NH-N-[(4-picolyl)-3-aminoquinuclidinyl]-acetate;
N-[2-(pyrrolidine-2-methoxycarbonyl)ethyoxycarbonyl]-Phe-Nle-ACHPA-NH-N-[(4-picolyl)-3-aminoquinuclidinyl]acetate;
N-[3-(pyrrolidine-2-methoxycarbonyl)propyloxycarbonyl]-Phe-Nle-ACHPA-NH-N-[(4-picolyl)-3-aminoquinuclidinyl]acetate;
N-[4-(pyrrolidine-2-methoxycarbonyl)butyryl]-Phe-Nle-ACHPA-NH-N-[(4-picolyl)-3-aminoquinuclidinyl]-acetate;
N-(2-t-butylsulfonylethyloxycarbonyl)-Phe-His-ACHPA-NH-N-[(4-picolyl)-3-aminoquinuclidinyl]acetate;
N-(3-t-butylsulfonylpropyloxycarbonyl)-Phe-His-ACHPA-NH-N-[(4-picolyl)-3-aminoquinuclidinyl]acetate;
N-(4-t-butylsulfonylbutyryl)-Phe-His-ACHPA-NH-N-[(4-picolyl-)-3-aminoquinuclidinyl]acetate;
N-(2-t-butylsulfonamidoethyloxycarbonyl)-Phe-His-ACHPA-NH-N-[(4-picolyl)-3-aminoquinuclidinyl]acetate;
N-(3-t-butylsulfonamidopropyloxycarbonyl)-Phe-His-ACHPA-NH-N-[(4-picolyl)-3-aminoquinuclidinyl]acetate;
N-(4-t-butylsulfonamidobutyryl)-Phe-His-ACHPA-NH-N-[(4-picolyl)-3-aminoquinuclidinyl]acetate;
N-(2-ethoxycarbonylaminoethoxycarbonyl)-Phe-His-ACHPA-NH-N-[(4-picolyl)-3-aminoquinuclidinyl]acetate;
N-(3-ethoxycarbonylaminopropyloxycarbonyl)-Phe-His-ACHPA-NH-N-[(4-picolyl)-3-aminoquinuclidinyl]-acetate;
N-(4-ethoxycarbonylaminobutyryl)-Phe-His-ACHPA-NH-N-[(4-picolyl)-3-aminoquinuclidinyl]acetate;
N-(2-t-butoxycarbonylaminoethoxycarbonyl)-Phe-His-ACHPA-NH-N-[(4-picolyl)-3-aminoquinuclidinyl]acetate;
N-(4-t-butoxycarbonylaminobutyryl)-Phe-His-ACHPA-NH-N-[(4-picolyl)-3-aminoquinuclidinyl]acetate;
N-(3-t-butoxycarbonylaminopropyloxycarbonyl)-Phe-His-ACHPA-NH-N-[(4-picolyl)-3-aminoquinuclidinyl]-acetate;
N-(2-t-butylacetamidoethoxycarbonyl)-Phe-His-ACHPA-NH-N-[(4-picolyl)-3-aminoquinuclidinyl]acetate;
N-(3-t-butylacetamidopropyloxycarbonyl)-Phe-His-ACHPA-NH-N-[(4-picolyl)-3-aminoquinuclidinyl]acetate;
N-(4-t-butylacetamidobutyryl)-Phe-His-ACHPA-NH-N-[(4-picolyl)-3-aminoquinuclidinyl]acetate;
N-[2-(3-amino-3-methylbutyramidoethoxycarbonyl]-Phe-His-ACHPA-NH-N-[(4-picolyl)-3-aminoquinuclidinyl]-acetate;
N-[3-(3-amino-3-methylbutyramidopropyloxycarbonyl]-Phe-His-ACHPA-NH-N-[(4-picolyl)-3-aminoquinuclidinyl]acetate;
N-[4-(3-amino-3-methylbutyramidobutyryl]-Phe-His-ACHPA-NH-N-[(4-picolyl)-3-aminoquinuclidinyl]acetate;
N-(2-L-prolinamidoethoxycarbonyl)-Phe-His-ACHPA-NH-N-[(4-picolyl-3-aminoquinuclidinyl]acetate;
N-(3-L-prolinamidopropyloxycarbonyl)-Phe-His-ACHPA-NH-N-[(4-picolyl)-3-aminoquinuclidinyl]acetate;
N-(4-L-prolinamidobutyryl)-Phe-His-ACHPA-NH-N-[(4-picolyl)-3-aminoquinuclidinyl]acetate;
N-[2-(piperidine-3-methoxycarbonylamino)ethyoxycarbonyl]-Phe-His-ACHPA-NH-N-[(4-picolyl)-3-aminoquinuclidinyl]acetate;
N-4-(piperidine-3-methoxycarbonyl)-Phe-His-ACHPA-NH-N-[(4-picolyl)-3-aminoquinuclidinyl]acecate;

N-[2-(pyrrolidine-2-methoxycarbonyl)Phe-His-ACHPA-NH-N-[(4-picolyl)-3-aminoquinuclidinyl]acetate;
N-[3-(piperidine-3-methoxycarbonylamino)propyloxycarbonyl]-Phe-His-ACHPA-NH-N-[(4-picolyl)-3-aminoquinuclidinyl]acetate;
N-[4-(piperidine-3-methoxycarbonylamino)butyryl]-Phe-His-ACHPA-NH-N-[(4-picolyl)-3-aminoquinuclidinyl]-acetate;
N-[2-(pyrrolidine-2-methoxycarbonyl)ethoxycarbonyl]-Phe-His-ACHPA-NH-N-[(4-picolyl)-3-aminoquinuclidinyl]acetate;
N-[3-(pyrrolidine-2-methoxycarbonyl)propyloxycarbonyl]-Phe-His-ACHPA-NH-N-[(4-picolyl)-3aminoquinuclidinyl]acetate;
N-[4-(pyrrolidine-2-methoxycarbonyl)butyryl]-Phe-His-ACHPA-NH-N-[(4-picolyl)-3-aminoquinuclidinyl]acetate;
N-(2-t-butylsulfonylethyloxycarbonyl)-Phe-His-ACHPA-NH-[1-(4-hydroxybutyl)-quinuclidin-3-yl] acetate;
N-(3-t-butylsulfonylpropyloxycarbonyl)-Phe-His-ACHPA-NH-[1-(4-hydroxybutyl)-quinuclidin-3-yl] acetate;
N-(4-t-butylsulfonylbutyryl)-Phe-His-ACHPA-NH-[1-(4-hydroxybutyl)-quinuclidin-3-yl] acetate;
N-(2-t-butylsulfonamidoethyloxycarbonyl)-Phe-His-ACHPA-NH-[1-(4-hydroxybutyl)-quinuclidin-3-yl] acetate;
N-(3-t-butylsulfonamidopropyloxycarbonyl)-Phe-His-ACHPA-NH-[1-(4-hydroxybutyl)-quinuclidin-3-yl] acetate;
N-(4-t-butylsulfonamidobutyryl)-Phe-His-ACHPA-NH-[1-(4-hydroxybutyl)-quinuclidin-3-yl] acetate;
N-(2-ethoxycarbonylaminoethoxycarbonyl)-Phe-His-ACHPA-NH-[1-(4-hydroxybutyl)-quinuclidin-3-yl] acetate;
N-(3-ethoxycarbonylaminopropyloxycarbonyl)-Phe-His-ACHPA-NH-[1-(4-hydroxybutyl)-quinuclidin-3-yl] acetate;
N-(4-ethoxycarbonylaminobutyryl)-Phe-His-ACHPA-NH-[1-(4 hydroxybutyl)-quinuclidin-3-yl] acetate;
N-(2-t-butoxycarbonylaminoethoxycarbonyl)-Phe-His-ACHPA-NH-[1-(4-hydroxybutyl)-quinuclidin-3-yl] acetate;
N-(4-t-butoxycarbonylaminobutyryl)-Phe-His-ACHPA-NH-[1-(4-hydroxybutyl)-quinuclidin-3-yl] acetate;
N-(3-t-butoxycarbonylaminopropyloxycarbonyl)-Phe-His-ACHPA-NH-[1-(4-hydroxybutyl)-quinuclidin-3-yl] acetate;
N-(2-t-butylacetamidoethoxycarbonyl)-Phe-His-ACHPA-NH-[1-(4-hydroxybutyl)-quinuclidin-3-yl] acetate;
N-(3-t-butylacetamidopropyloxycarbonyl)-Phe-His-ACHPA-NH-[1-(4-hydroxybutyl)-quinuclidin-3-yl] acetate;
N-(4-t-butylacetamidobutyryl)-Phe-His-ACHPA-NH-[1-(4-hydroxybutyl)-quinuclidin-3-yl] acetate;
N-[2-(3-amino-3-methylbutyramidoethoxycarbonyl]-Phe-His-ACHPA-NH-[1-(4-hydroxybutyl)-quinuclidin-3-yl] acetate;
N-[3-(3-amino-3-ethylbutyramidopropyloxycarbonyl]-Phe-His-ACHPA-NH-[1-(4-hydroxybutyl)-quinuclidin-3-yl] acetate;
N-[4-(3-amino-3-methylbutyramidcbutyryl]-Phe-His-ACHPA-NH-[1-(4-hydroxybutyl)-quinuclidin-3-yl] acetate;
N-(2-L-prolinamidoethoxycarbonyl)-Phe-His-ACHPA-NH-[1-(4-hydroxybutyl)-quinuclidin-3-yl] acetate;
N-(3-L-prolinamidopropyloxycarbonyl)-Phe-His-ACHPA-NH-[1-(4-hydroxybutyl)-quinuclidin-3-yl] acetate;
N-(4-L-prolinamidobutyryl)-Phe-His-ACHPA-NH-[1-(4-hydroxybutyl)-quinuclidin-3-yl] acetate;
N-[2-(piperidine-3-methoxycarbonylamino)ethyoxycarbonyl]-Phe-His-ACHPA-NH-[1-(4-hydroxybutyl)-quinuclidin-3-yl] acetate;
N-4-(piperidine-3-methoxycarbonyl)-Phe-His-ACHPA-NH-[1-(4-hydroxybutyl)-quinuclidin-3-yl] acetate;
N-[2-(pyrrolidine-2-methoxycarbonyl)-Phe-His-ACHPA-NH-[1-(4-hydroxybutyl)-quinuclidin-3-yl] acetate;
N-[3-(piperidine-3-methoxycarbonylamino)propyloxycarbonyl]-Phe-His-ACHPA-NH-[1-(4-hydroxybutyl)-quinuclidin-3-yl] acetate;
N-[4-(piperidine-3-methoxycarbonylamino)butyryl]-Phe-His-ACHPA-NH-[1-(4-hydroxybutyl)-quinuclidin-3-yl] acetate;
N-[2-(pyrrolidine-2-methoxycarbonyl)ethyoxycarbonyl]-Phe-His-ACHPA-NH-[1-(4-hydroxybutyl)-quinuclidin-3-yl] acetate;
N-[3-(pyrrolidine-2-methoxycarbonyl)propyloxycarbonyl]-Phe-His-ACHPA-NH-[1-(4-hydroxybutyl)-quinuclidin-3-yl] acetate;
N-[4-(pyrrolidine-2-methoxycarbonyl)butyryl]-Phe-His-ACHPA-NH-[1-(4-hydroxybutyl)-quinuclidin-3-yl] acetate;
N-(2-t-butylsulfonylethyloxycarbonyl)-Phe-His-ACHPA-NH-(1-benzylquinuclidin-3-yl) acetate;
N-(3-t-butylsulfonylpropyloxycarbonyl)-Phe-His-ACHPA-NH-(1-benzylquinuclidin-3 yl) acetate;
N-(4-t-butylsulfonylbutyryl)-Phe-His-ACHPA-NH-(1-benzylquinuclidin-3-yl) acetate;
N-(2-t-butylsulfonamidoethyloxycarbonyl)-Phe-His-ACHPA-NH-(1-benzylquinuclidin-3-yl) acetate;
N-(3-t-butylsulfonamidopropyloxycarbonyl)-Phe-His-ACHPA-NH-(1-benzylquinuclidin-3-yl) acetate;
N-(4-t-butylsulfonamidobutyryl)-Phe-His-ACHPA-NH-(1-benzylquinuclidin-3-yl) acetate;
N-(2-ethoxycarbonylaminoethoxycarbonyl)-Phe-His-ACHPA-NH-(1-benzylquinuclidin-3-yl) acetate;
N-(3-ethoxycarbonylaminopropyloxycarbonyl)-Phe-His-ACHPA-NH-(1-benzylquinuclidin-3-yl) acetate;

N-(4-ethoxycarbonylaminobutyryl)-Phe-His-ACHPA-NH-(1-benzylquinuclidin-3-yl) acetate;
N-(2-t-butoxycarbonylaminoethoxycarbonyl)-Phe-His-ACHPA-NH-(1-benzylquinuclidin-3-yl) acetate;
N-(4-t-butoxycarbonylaminobutyryl)-Phe-His-ACHPA-NH-(1-benzylquinuclidin-3-yl) acetate;
N-(3-t-butoxycarbonylaminopropyloxycarbonyl)-Phe-His-ACHPA-NH-(1-benzylquinuclidin-3-yl) acetate;
N-(2-t-butylacetamidoethoxycarbonyl)-Phe-His-ACHPA-NH-(1-benzylquinuclidin-3-yl) acetate;
N-(3-t-butylacetamidopropyloxycarbonyl)-Phe-His-ACHPA-NH-(1-benzylquinuclidin-3-yl) acetate;
N-(4-t-butylacetamidobutyryl)-Phe-His-ACHPA-NH-(1-benzylquinuclidin-3-yl) acetate;
N-[2-(3-amino-3-methylbutyramidoethoxycarbonyl]-Phe-His-ACHPA-NH-(1-benzylquinuclidin-3-yl) acetate;
N-[3-(3-amino-3-methylbutyramidopropyloxycarbonyl]-Phe-His-ACHPA-NH-(1-benzylquinuclidin-3-yl) acetate;
N-[4-(3-amino-3-methylbutyramidobutyryl]-Phe-His-ACHPA-NH-(1-benzylquinuclidin-3-yl) acetate;
N-(3-L-prolinamidopropyloxycarbonyl)-Phe-His-ACHPA-NH-(1-benzylquinuclidin-3-yl) acetate;
N-(4-L-prolinamidobutyryl)-Phe-His-ACHPA-NH-(1-benzylquinuclidin-3-yl) acetate;
N-[2-(piperidine-3-methoxycarbonylamino)ethyoxycarbonyl]-Phe-His-ACHPA-NH-(1-benzylquinuclidin-3-yl) acetate;
N-[3-(piperidine-3-methoxycarbonylamino)propyloxycarbonyl]-Phe-His-ACHPA-NH-(1-benzylquinuclidin-3-yl) acetate;
N-[4-(piperidine-3-methoxycarbonylamino)butryl]-Phe-His-ACHPA-NH-(1-benzylquinuclidin-3-yl) acetate;
N-4-(piperidine-3-methoxycarbonyl)-Phe-His-ACHPA-NH-(1-benzylquinuclidin-3-yl) acetate;
N-2-(pyrrolidine-2-methoxycarbonyl)-Phe-His-ACHPA-NH-(1-benzylquinuclidin-3-yl) acetate;
N-[2-(pyrrolidine-2-methoxycarbonyl)ethyoxycarbonyl]-Phe-His-ACHPA-NH-(1-benzylquinuclidin-3-yl) acetate;
N-[3-(pyrrolidine-2-methoxycarbonyl)propyloxycarbonyl]-Phe-His-ACHPA-NH-(1-benzylquinuclidin-3-yl) acetate;
N-[4-(pyrrolidine-2-methoxycarbonyl)butyryl]-Phe-His-ACHPA-NH-(1-benzylquinuclidin-3-yl) acetate;
N-(2-t-butylsulfonylethyloxycarbonyl)-Phe-His-ACHPA-NH-[1-((3,5-dimethylisoxazol-4-yl)methyl)quinuclidin-3-yl]acetate;
N-(3-t-butylsulfonylpropyloxycarbonyl)-Phe-His-ACHPA-NH-[1-((3,5-dimethylisoxazol-4-yl)methyl)quinuclidin-3-yl] acetate;
N-(4-t-butylsulfonylbutyryl)-Phe-His-ACHPA-NH-[1-((3,5-dimethylisoxazol-4-yl)methyl)quinuclidin-3-yl] acetate;
N-(2-t-butylsulfonamidoethyloxycarbonyl)-Phe-His-ACHPA-NH-[1-((3,5-dimethylisoxazol-4-yl)methyl)-quinuclidin-3-yl] acetate;
N-(3-t-butylsulfonamidopropyloxycarbonyl)-Phe-His-ACHPA-NH-[1-((3,5-dimethylisoxazol-4-yl)methyl)-quinuclidin-3-yl] acetate;
N-(4-t-butylsulfonamidobutyryl)-Phe-His-ACHPA-NH-[1-((3,5-dimethylisoxazol-4-yl)methyl)quinuclidin-3-yl] acetate;
N-(2-ethoxycarbonylaminoethoxycarbonyl)-Phe-His-ACHPA-NH-[1-((3,5-dimethylisoxazol-4-yl)methyl)-quinuclidin-3-yl] acetate;
N-(3-ethoxycarbonylaminopropyloxycarbonyl)-Phe-His-ACHPA-NH-[1-((3,5-dimethylisoxazol-4-yl)methyl)-quinuclidin-3-yl] acetate;
N-(4-ethoxycarbonylaminobutyryl)-Phe-His-ACHPA-NH-[1-((3,5-dimethylisoxazol-4-yl)methyl)quinuclidin-3-yl] acetate;
N-(2-t-butoxycarbonylaminoethoxycarbonyl)-Phe-His-ACHPA-NH-[1-((3,5-dimethylisoxazol-4-yl)methyl)-quinuclidin-3-yl] acetate;
N-(4-t-butoxycarbonylaminobutyryl)-Phe-His-ACHPA-NH-[1-((3,5-dimethylisoxazol-4-yl)methyl)quinuclidin-3-yl] acetate;
N-(3-t-butoxycarbonylaminopropyloxycarbonyl)-Phe-His-ACHPA-NH-[1-((3,5-dimethylisoxazol-4-yl)methyl)-quinuclidin-3-yl] acetate;
N-(2-t-butylacetamidoethoxycarbonyl)-Phe-His-ACHPA-NH-[1-((3,5-dimethylisoxazol-4-yl)methyl)quinuclidin-3-yl] acetate;
N-(3-t-butylacetamidopropyloxycarbonyl)-Phe-His-ACHPA-NH-[1-((3,5 dimethylisoxazol-4-yl)methyl)-quinuclidin-3-yl] acetate;
N-(4-t-butylacetamidobutyryl)-Phe-His-ACHPA-NH-[1-((3,5-dimethylisoxazol-4-yl)methyl)quinuclidin-3-yl] acetate;
N-[2-(3-amino-3-methylbutyramidoethoxycarbonyl]-Phe-His-ACHPA-NH-[1-((3,5-dimethylisoxazol-4-yl)-methyl)quinuclidin-3-yl] acetate;
N-[3-(3-amino-3-methylbutyramidopropyloxycarbonyl]-Phe-His-ACHPA-NH-[1-((3,5-dimethylisoxazol-4-yl)-

methyl)quinuclidin-3-yl] acetate;
N-[4-(3-amino-3-methylbutyramidobutyryl]-Phe-His-ACHPA-NH-[1-((3,5-dimethylisoxazol-4-yl)methyl)-quinuclidin-3-yl] acetate;
N-(2-L-prolinamidoethoxycarbonyl)-Phe-His-ACHPA-NH-[1-((3,5-dimethylisoxazol-4 yl)methyl)quinuclidin-3-yl] acetate;
N-(3-L-prolinamidopropyloxycarbonyl)-Phe-His-ACHPA-NH-[1-((3,5-dimethylisoxazol-4-yl)methyl)quinuclidin-3-yl]acetate;
N-(4-L-prolinamidobutyryl)-Phe His-ACHPA-NH-[1-((3,5-dimethylisoxazol-4-yl)methyl)quinuclidin-3-yl] acetate;
N-[2-(piperidine-3-methoxycarbonylamino)ethyoxycarbonyl]-Phe-His-ACHPA-NH-[1-((3,5-dimethylisoxazol-4-yl)methyl)quinuclidin-3-yl] acetate;
N-4-(piperidine-3-methoxycarbonyl)-Phe-His-ACHPA-NH-[1-((3,5-dimethylisoxazol-4-yl)methyl)quinuclidin-3-yl] acetate;
N-2-(pyrrolidine-2-methoxycarbonyl)-Phe-His-ACHPA-NH-[1-((3,5-dimethylisoxazol-4-yl)methyl)quinuclidin-3-yl]acetate;
N-[3-(piperidine-3-methoxycarbonylamino)propyloxycarbonyl]-Phe-His-ACHPA-NH-[1-((3,5-dimethylisoxazol-4-yl)methyl)quinuclidin-3-yl] acetate;
N-[4-(piperidine-3-methoxycarbonylamino)butyryl]-Phe-His-ACHPA-NH-[1-((3,5-dimethylisoxazol-4-yl)-methyl)quinuclidin-3-yl] acetate;
N-[2-(pyrrolidine-2-methoxycarbonyl)ethyoxycarbonyl]Phe-His-ACHPA-NH-[1-((3,5-dimethylisoxazol-4-yl)-methyl)quinuclidin-3-yl] acetate;
N-[3-(pyrrolidine-2-methoxycarbonyl)propyloxycarbonyl]-Phe-His-ACHPA-NH-[1-((3,5-dimethylisoxazol-4-yl)-methyl)quinuclidin-3-yl] acetate;
N-[4-(pyrrolidine-2-methoxycarbonyl)butyryl]-Phe-His-ACHPA-NH [1-((3,5-dimethylisoxazol-4-yl)methyl)-quinuclidin-3-yl] acetate.

Representative particularly preferred renin inhibitory peptides of the present invention include the following:
N-(2-t-butoxycarbonylaminoethoxycarbonyl)-Phe-His-ACHPA-NH-2(S)methylbutyl;
N-(4-t-butoxycarbonylaminobutyryl)-Phe-His-ACHPA-NH-2(S)methylbutyl;
N-(2-t-butylacetamidoethoxycarbonyl)-Phe-His-ACHPA-NH-2(S)methylbutyl;
N-[2-(3-amino-3-methyl)propiomylamidoethoxycarbonyl]-Phe-His-ACHPA-NH-2(S)methylbutyl;
N-(piperidine-4-ethoxycarbonyl)-Phe-His-ACHPA-NH-2(S)methylbutyl;
N-[2-(piperidine-3-methoxycarbonylamino)ethoxycarbonyl]-Phe-His-ACHPA-NH-2(S)methylbutyl;
N-(2-t-butoxycarbonylaminoethoxycarbonyl)-Phe-Nle-ACHPA-NH-N-[(4-picolyl)-3-aminoquinuclidinyl]acetate;
N-(2-t-butylacetamidoethoxycarbonyl)-Phe-Nle-ACHPA-NH-N-[(4-picolyl)-3 aminoquinuclidinyl]acetate;
N-[2-(3-amino-3-methylbutyramidoethoxycarbonyl]-Phe-Nle-ACHPA-NH-N-[(4-picolyl)-3-aminoquinuclidinyl]-acetate;
N-(2-t-butoxycarbonylaminoethoxycarbonyl)-Phe-His-ACHPA-NH-N-[(4-picolyl)-3-aminoquinuclidinyl]acetate;
N-(2-t-butylacetamidoethoxycarbonyl)-Phe-His-ACHPA-NH-N-[(4-picolyl)-3-aminoquinuclidinyl]acetate;
N-[2-(3-amino-3-methylbutyramidoethoxycarbonyl]-Phe-His-ACHPA-NH-N-[(4-picolyl)-3-aminoquinuclidinyl]-acetate;
N-(2-t-butylsulfonylethyloxycarbonyl)-Phe-His-ACHPA-NH-(N-methyl-3-aminoquinuclidinyl) acetate;
N-(2-t-butoxycarbonylaminoethoxycarbonyl)-Phe-His-ACHPA-NH-(N-methyl-3-aminoquinuclidinyl) acetate;
N-(2-t-butylacetamidoethoxycarbonyl)-Phe-His-ACHPA-NH-(N-methyl-3-aminoquinuclidinyl) acetate;
N-[2-(3-amino-3-methylbutyramidoethoxycarbonyl]-Phe-His-ACHPA-NH-(N-methyl-3-aminoquinuclidinyl) acetate;
N-[2-(piperidine-3-methoxycarbonylamino)ethyoxycarbonyl]-Phe-His-ACHPA-NH-(N-methyl-3-aminoquinuclidinyl) acetate;
N-(2-t-butoxycarbonylaminoethoxycarbonyl)-Phe-His-Cal[$CH(OH)CH_2$]Val-NH$(CH_2)_3CH_3$;
N-(2-t-butylacetamidoethoxycarbonyl)-Phe-His-Cal[$CH(OH)CH_2$]Val-NH$(CH_2)_3CH_3$;
N-[2-(3-amino-3-methylbutyramidoethoxycarbonyl]-Phe-His-Cal[$CH(OH)CH_2$]Val-NH$(CH_2)_3CH_3$;
N-(2-t-butoxycarbonylaminoethoxycarbonyl)-Phe-His-ACHPA-NH$CH_2CH(OH)CH_2N(CH_2CH_2)_2O$;
N-(2-t-butylacetamidoethoxycarbonyl)-Phe-His-ACHPA-NH$CH_2CH(OH)CH_2N(CH_2CH_2)_2O$;
N-[2-(3-amino-3-methylbutyramidoethoxycarbonyl]-Phe-His-ACHPA-NH$CH_2CH(OH)CH_2N(CH_2CH_2)_2O$;
N-(2-t-butoxycarbonylaminoethoxycarbonyl)-Phe-His-ACHPA-NH-[1-(4-hydroxybutyl)-quinuclidin-3-yl] acetate;
N-(2-t-butylacetamidoethoxycarbonyl)-Phe-His-ACHPA-NH-[1-(4-hydroxybutyl)-quinuclidin-3-yl] acetate;
N-(2-t-butoxycarbonylaminoethoxycarbonyl)-Phe-His-ACHPA-NH-(1-benzylquinuclidin-3-yl) acetate;

N-(2-t-butylacetamidoethoxycarbonyl)-Phe-His-ACHPA-NH-(1-benzylquinuclidin-3-yl) acetate;
N-[2-(3-amino-3-methylbutyramidoethoxycarbonyl]-Phe-His-ACHPA-NH-(1-benzylquinuclidin-3-yl) acetate;
N-(2-t-butoxycarbonylaminoethoxycarbonyl)-Phe-His-ACHPA-NH-[1-((3,5-dimethylisoxazol-4-yl)methyl)-quinuclidin-3-yl] acetate;
N-(2-t-butylacetamidoethoxycarbonyl)-Phe-His-ACHPA-NH-[1-((3,5-dimethylisoxazol-4-yl)methyl)quinuclidin-3-yl] acetate;
N-[2-(3-amino-3-methylbutyramidoethoxycarbonyl]-Phe-His-ACHPA-NH-[1-((3,5-dimethylisoxazol-4-yl)-methyl)quinuclidin-3-yl] acetate;
N-2-(pyrrolidine-2-methoxycarbonyl)-Phe-His-ACHPA-NH-(N-methyl-3-aminoquinuclidinyl) acetate;
N-4-(piperidine-3-methoxycarbonyl)-Phe-His-ACHPA-NH-(N-methyl-3-aminoquinuclidinyl) acetate;
N-4-(piperidine-3-methoxycarbonyl)-Phe-His-Cal[CH(OH)$CH_2$]Val-NH$(CH_2)_3CH_3$;
N-2-(pyrrolidine-2-methoxycarbonyl)Phe-His-Cal[CH(OH)$CH_2$]Val-NH$(CH_2)_3CH_3$;
N-4-(piperidine-3-methoxycarbonyl)-Phe-His-ACHPA-NH$CH_2$CH(OH)$CH_2$N$(CH_2CH_2)_2$O;
N-2-(pyrrolidine-2-methoxycarbonyl)Phe-His-ACHPA-NH$CH_2$CH(OH)$CH_2$N$(CH_2CH_2)_2$O;
N-4-(piperidine-3-methoxycarbonyl)-Phe-His-ACHPA-NH-[1-(4-hydroxybutyl)-quinuclidin-3-yl] acetate;
N-2-(pyrrolidine-2-methoxycarbonyl)Phe-His-ACHPA-NH-[1-(4-hydroxybutyl)-quinuclidin-3-yl] acetate;
N-4-(piperidine-3-methoxycarbonyl)-Phe-His-ACHPA-NH-(1-benzylquinuclidin-3-yl) acetate;
N-2-(pyrrolidine-2-methoxycarbonyl)-Phe-His-ACHPA-NH-(1-benzylquinuclidin-3-yl) acetate;
N-4-(piperidine-3-methoxycarbonyl)-Phe-His-ACHPA-NH-[1-((3,5-dimethylisoxazol-4-yl)methyl)quinuclidin-3-yl] acetate;
N-2-(pyrrolidine-2-methoxycarbonyl)-Phe-His-ACHPA-NH-[1-((3,5-dimethylisoxazol-4-yl)methyl)quinuclidin-3-yl] acetate;

The pharmaceutically-acceptable salts of the peptides of Formula I (in the form of water- or oil-soluble or dispersible products) include the conventional non-toxic salts or the quaternary ammonium salts of these peptides, which are formed, e.g., from inorganic or organic acids or bases. Examples of such acid addition salts include acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenylpropionate, picrate, pivalate, propionate, succinate, tartrate, thiocyanate, tosylate, and undecanoate. Base salts include ammonium salts, alkali metal salts such as sodium and potassium salts, alkaline earth metal salts such as calcium and magnesium salts, salts with organic bases such as dicyclohexylamine salts, N-methyl-D-glucamine, and salts with amino acids such as arginine, lysine, and so forth. Also, the basic nitrogen-containing groups may be quaternized with such agents as lower alkyl halides, such as methyl, ethyl, propyl, and butyl chloride, bromides and iodides; dialkyl sulfates like dimethyl, diethyl, dibutyl; and diamyl sulfates, long chain halides such as decyl, lauryl, myristyl and stearyl chlorides, bromides and iodides, aralkyl halides like benzyl and phenethyl bromides and others.

Peptide renin inhibitors of Formula I may be prepared in accordance with well-known procedures fcr preparing peptides from their constitutent amino acids.

Structures and abbreviations for the G components of the renin-inhibitory peptides of the present invention include:

1.

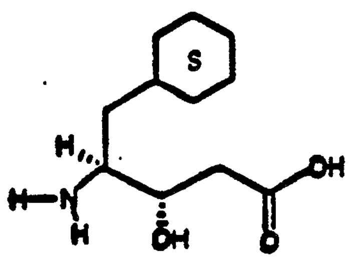

ACHPA

with BOC-ACHPA-OEt being prepared by the method described by Boger et al, J. Med. Chem 1985, 28, 1779-1790;

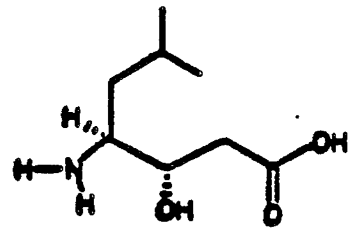

Statine (Sta)

2.

with BOC-Sta-OEt being prepared in accordance with the procedure described by Rich et al, J. Org. Chem., 43, 3624(1978); with efficient methods for preparing the 2-substituted statine component G (such as

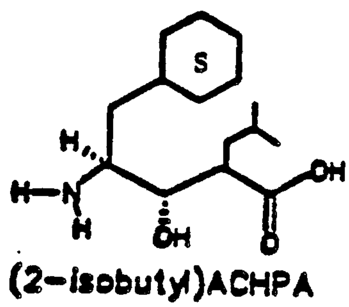

(2-isobutyl)ACHPA

3.

with (2-isobutyl)ACHPA and other 2-substituted statine components, in a suitably protected form, being described in Pub. Eur. Pat Appln. 157,409 (with other pertinent references including D. Veber et al, Biochem. Soc. Trans., 12, 956-959 (1984) and H. Stein et al, Fed. Proc. 45, 869, Abstract No 4151 (1986); and

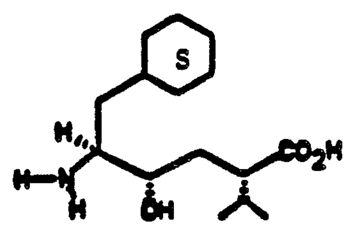

Cal[CH(OH)$CH_2$]Val

4.

Boc-Cal[CH(OH)$CH_2$]Val-OH lactone may be obtained from intermediates prepared using methods described by P. Buhlmayer et al in European Patent Application 0,184,550-A2. Synthetic routes to similar peptide bond isosteres are described in the following:

a. Szelke et al in Peptides, Structure and Function. Proceedings of the Eighth American Peptide Symposium (ed. V. J. Hruby and D. H. Rich) pp. 579-82. Pierce Chemical Co., Rockford, IL.

b. D. T. Pals et al in European Patent Application 0,173,481-A2.

c. B. E. Evans et al, J. Org. Chem., 50, 4615-1625 (1985).

d. A. H. Fray et al, J. Org. Chem., 51, 4828-4833 (1986).

e. M. Szelke et al, PCT Int. Appl. WO 84 03,044.

f. D. J. Kempf, J. Org. Chem., 51, 3921-3926 (1986). 5.

Efficient methods for preparing the 2-substituted statine component G in a suitably protected form are described in U.S. Serial No. 596,766, filed 4/4/84, now U.S. Pat. No. 4,663,310 (Attorney Docket No. Case

16970). Other pertinent references are D. Veber et al, Biochem. Soc. Trans., 12, 956-959 (1984) and H. Stein et al Fed. Proc. 45, 869, Abstract No 4151 (1986).

The peptide coupling reactions referred to below in Routes A, B, C, and D include those brought about by the action of DCC/HOBt (dicyclohexylcarbodiimide/N-hydroxybenzotriazole), DPPA (diphenylphorylazide), "BOP reagent" (benzotriazol-1-yloxytris(dimethylamino)phosphonium hexafluorophosphate), BOP-Cl (bis(2-oxo-3-oxazolidinyl)phosphinic chloride), DSO (N, N′-disuccinimidyl oxalate), and other well-known coupling reagents.

The amine components $R^{2c}R^{2d}NH$ referred to below are intended to represent all those encompassed by the definitions of T-U-J, and may include, for example, primary and secondary amines, amino acid amides or esters , or dipeptide amides or esters.

Methods used in the procedures below for quaternizing tertiary amines are well-known and include, for example, that of Chen et al., Can. J. Chem., 54, 3310-11 (1976). Methods used in the procedures below for preparing guanidines from primary and secondary amines are well-known and include, for example, that of Maryanoff, et al. J. Org. Chem., 51, 1882-4 (1986). Peptides of Formula I which contain ACHPA as a peptide bond mimic are prepared using the following procedures:

Route A:

Step A1:

BOC-ACHPA-OEt is treated with anhydrous TFA to remove the BOC protecting group, giving ACHPA-OEt.

Step A2:

Using standard methods, a dipeptide protected at the N-terminus with a BOC group (BOC-$AA^2$-$AA^1$) is coupled to ACHPA-OEt, giving a coupled product 3. Alternatively, BOC-$AA^1$ is coupled to ACHPA-OEt giving 1. The coupled product 1 is treated with anhydrous TFA to removed the BOC protecting group, and the resulting product 2 is coupled with BOC$AA^2$, giving 3.

Step A3:

The resulting coupled product 3 is treated with methanolic hydrazine, giving the corresponding hydrazide 4.

Step A4:

Hydrazide 4 is treated with acidic isoamyl nitrite, producing the coresponding acyl azide, which is treated in situ with an amine $R^{2c}R^{2d}NH$, giving coupled product 5. Alternatively, coupled product 3 as treated with sodium hydroxide in THF-$H_2O$ and the resulting carboxylic acid derivative coupled with $R^{2c}R^{2d}NH$, giving 5. Additional reactive functional groups in the amino component $R^{2c}R^{2d}NH$ are protected with protecting groups, such as CBZ for amino groups, benzyl ether for alcohols, and benzyl esters for carboxylic acids.

Step A5:

The N-terminal BOC protecting group is then removed from 5 by treatment with anhydrous TFA, and the resulting tripeptide analog 6 is treated first with carbonyldiimidazole and shortly thereafter with substituted alcohol component X′-(alkyl)-OH, producing carbamate 7. Alternatively, 6 may be treated with substituted acid X′-(alkyl)-$CO_2H$ and a coupling reagent such as DCC. Alternatively, the alcohol component X′-(alkyl)-OH is first treated with carbonyldiimidazole or with phosgene, and the resulting intermediate allowed to react with 6, producing carbamate 7. Additional reactive functional groups in the alcohol or acid

components X′-(alkyl)-OH and X′-(alkyl)-$CO_2H$ are protected with protecting groups as noted above for $R^{2c}R^{2d}NH$.

Step A5a:

Alternatively, compound 7 may be prepared using the following sequence: Compound 2 is coupled with X′-(alkyl)-OCO-$AA^2$, or X′-(alkyl)-CO-$AA^2$ producing carbamate or amide 3a. Compound 3a is then treated with methanolic hydrazine, giving hydrazide 4a. Hydrazide 4a is treated with acidic isoamyl nitrite, producing the corresponding acyl azide, which is treated in situ with an amine $R^{2c}R^{2d}NH$, giving 7. Additional reactive functional groups in the alcohol or acid components X′-(alkyl)-OH or X′-(alkyl)-$CO_2H$ are protected with protecting groups as noted above for $R^{2c}R^{2d}NH$. The X′-(alkyl)-OCO-$AA^2$ component is prepared by treatment of $AA^2$-O-t-Bu with carbonyldiimidazole and then with X′-(alkyl)-OH, giving X′-(alkyl)-OCO-$AA^2$-O-t-Bu. Treatment of this product with anhydrous TFA then gives X′-(alkyl)-OCO-$AA^2$, which is used as described above. The X′-(alkyl)-CO-$AA^2$ component is similarly made by combining X′-(alkyl)-$CO_2H$ and $AA^2$-O-t-Bu with DCC under coupling conditions to give X′-alkyl)-CO-$AA^2$-O-t-Bu. Treatment of this product with anhydrous TFA then gives X′-(alkyl)-CO-$AA^2$, which is used as described above.

Step A6:

During the steps above, reactive functional groups present in the side-chains of amino acid components or in the $R^{2c}R^{2d}NH$- element are protected with protecting groups. These may be CBZ groups for amines, benzyl ethers for alcohols, and benzyl esters for carboxylic acids. In the case of histidine, the BOC protecting may be used for protection of the side-chain imidazole ring; this group is removed in step A3 during the treatment with hydrazine, or alternatively in step A4 during treatment with sodium hydroxide. Thereafter the histidine imidazole ring may be left unprotected.

Step A7:

In cases where a quaternized amino group is to be introduced into the C-terminal $R^{2c}R^{2d}NH$-element of compound 8, one of the following procedures is followed to introduce the quaternized amine group:

Procedure 1:

A tertiary amine within the R1 or R2 group of $R^{2c}R^{2d}NH$ is quaternized by treatment of $R^{2c}R^{2d}NH$-BOC with an alkyl halide and $KHCO_3$ in methanol or ethanol, or with an alkyl halide in DMF. The resulting quaternary ammonium salt is then treated with anhydrous TFA to remove the BOC protecting group, and the resulting amine used in coupling step A4 above.

Procedure 2:

A compound 7, in which the $R^{2c}R^{2d}NH$-element contains a teriary amine, is prepared according to steps A1 to A6, and the tertiary amine is then quaternized by treatment with an alkyl halide in DMF. In this latter case, histidine, when present as the $AA^1$ element, must first be reprotected as the BOC derivative by treatment of 7 with di-t-butyldicarbonate. The BOC group is then removed after step A9 by treatment of 8 with $K_2CO_3$ in methanol or with anhydrous ammonia in DMF or methanol.

Step A8:

In cases where the $R^{2c}R^{2d}NH$- component of a compound 8 contains a guanidine group, compound 7 is prepared as described above. Step A7 may also be carried out if desired. Then an amine group present in the $R^{2c}R^{2d}NH$- component and hetetofore protected with a BOC group is liberated by treatment of 7 with anhydrous TFA. Then standard methods are used for introduction of the guanidine function.

Step A9:

Protecting groups are removed from 7 by hydrogenolysis, giving 8.

Step A10a:

Alcohol X′-(alkyl)-OH can be prepared from substituted or unsubstituted aminoethanol or aminopropanol by acylation with the appropriate carbamate, cambamyl chloride, carboxylic acid or acid chloride by the usual peptide coupling methods.

Step A10b:

Alcohol X′-(alkyl)-OH wherein X′ is O-$R^{1a}$ may be prepared from a protected alkyl diol bearing a free alcohol which can be alkylated or acylated by standard methods.

Step A10c:

Alcohol X′-(alkyl)-OH wherein X′ is $SR^{1a}$, $S(O)R^{1a}$, or $S(O)_2R^{1a}$ if not obtainable commercially can be made by the addition of a commercially available alkylthiol $R^{1a}SH$ to alcohols such as 2-chloro ethanol or 2-chloropropanol by standard methods of alkylation. Alternatively, alkyl thiol $R^{1a}SH$ may be added to ethylene oxide to give $R^{1a}S$-$CH_2$-$CH_2$-OH. The sulfide may be selectively oxidized to $R^{1a}SO$-$CH_2$-$CH_2$-OH, or $R^{1a}SO_2$-$CH_2$-$CH_2$-OH using 1 or 2 equivalents of an agent such as m-chloroperoxybenzoic acid. Alternatively, X′-(alkyl)-OCO-$AA^2$-O-t-Bu in which X′ is $R^{1a}S$ prepared according to Step A5a and methods cited here may be oxidized to sulfoxide or sulfone selectively by using 1 or 2 equivalents of m-chloroperoxybenzoic acid.

Step A10d:

Acids X′-(alkyl)-$CO_2H$ are prepared from the appropriate carboxylic esters by procedures similar to Steps A10a, A10b, or A10c.

| Compound | Formula |
|---|---|
| 1 | BOC-$AA^1$-ACHPA-OEt |
| 2 | $AA^1$-ACHPA-OEt |
| 3 | BOC-$AA^2$-$AA^1$-ACHPA-OEt |
| 3a | X′-(alkyl)-O-CO-$AA^2$-$AA^1$-ACHPA-OEt or X′-(alkyl)-CO-$AA^2$-$AA^1$-ACHPA-OEt |
| 4 | BOC-$AA^2$-$AA^1$-ACHPA-$NHNH_2$ |
| 4a | X′-(alkyl)-O-CO-$AA^2$-$AA^1$-ACHPA-$NHNH_2$ or X′-(alkyl)-CO-$AA^2$-$AA^1$-ACHPA-$NHNH_2$ |
| 5 | BOC-$AA^2$-$AA^1$-ACHPA-$NR^{2c}R^{2d}$ |
| 6 | $AA^2$-$AA^1$-ACHPA-$NR^{2c}R^{2d}$ |
| 7,8 | X′-(alkyl)-O-CO-$AA^2$-$AA^1$-ACHPA-$NR^{2c}R^{2d}$ or X′-(alkyl)-CO-$AA^2$-$AA^1$-ACHPA-$NR^{2c}R^{2d}$ |

Route B:

Step B1:

BOC-ACHPA-OEt is treated with sodium hydroxide in THF-$H_2O$, giving BOC-ACHPA.

Step B2:

BOC-ACHPA is coupled with an amine component $R^{2c}R^{2d}NH$, giving coupled product 9.

Step B3:

Compound 9 is treated with anhydrous TFA to remove the BOC protecting group, giving 10.

Step B4:

Compound 10 is coupled with a dipeptide protected at the N-terminus with a BOC protecting group (BOC-$AA^2$-$AA^1$), giving coupled product 12. Alternatively, 10 is coupled with BOC-$AA^1$, giving 11. Treatment of 11 with anhydrous TFA and coupling of the resulting product with BOC-$AA^2$ gives 12.

Step B5:

The N-terminal BOC protecting group is then removed from 12 by treatment with anhydrous TFA, and the resulting compound 13 is treated first with carbonyldiimidazole and shortly thereafter with an alcohol component $X'$-(alkyl)-OH, producing carbamate 7. Alternatively, the alcohol component $X'$-(alkyl)-OH is first treated with carbonyldiimidazole or with phosgene, and the resulting intermediate allowed to react with 13, producing carbamate 7. Acyl analog 7 is prepared by reaction of acid $X'$-(alkyl)-$CO_2H$ with 13 under standard peptide coupling conditions.

Step B5a:

Alternatively, compound 11 is treated with anhydrous TFA and the resulting intermediate is coupled to $X'$-(alkyl)-O-CO-$AA^2$, or $X'$-(alkyl)-CO-$AA^2$, as described in step A5a, to give compound 7.

Step B6:

As described above in route A, reactive functional groups in amino acid side chains of $AA^1$ and $AA^2$ and in the $X'$-(alkyl)-O or $X'$-(alkyl)-CO component are protected during the coupling steps above.

Step B7:

In cases where a quaternized amino group is to be introduced into the C-terminal $R^{2c}R^{2d}N$-element of compound 8, one of the following procedures is followed to introduce the quaternized amine group:

Procedure 1:

A tertiary amine within the R1 or R2 group of $R^{2c}R^{2d}NH$ is quaternized by treatment of $R^{2c}R^{2d}NH$-BOC with an alkyl halide and $KHCO_3$ in methanol or ethanol, or with an alkyl halide in DMF. The resulting quaternary ammonium salt is then treated with anhydrous TFA to remove the BOC protecting group, and the resulting amine used in coupling step B2 above.

Procedure 2:

A compound 7, in which the $R^{2c}R^{2d}N$-element contains a teriary amine, is prepared according to steps B1 to B6, and the tertiary amine is then quaternized by treatment with an alkyl halide in DMF. In this latter case, histidine, when present as the $AA^2$ element, must first be reprotected as the BOC derivative by treatment of 7 with di-t-butyldicarbonate. The BOC group is then removed after step B9 by treatment of 8 with $K_2CO_3$ in methanol, with anhydrous ammonia in DMF or methanol, or with anhydrous TFA.

Step B8:

In cases where the $R^{2c}R^{2d}N$- component of a compound 8 contains a guanidine group, compound 7 is prepared as described in steps B1 through B6 above. Step B7 is also carried out if desired. Then an amine group present in the $R^{2c}R^{2d}N$- component and hetetofore protected with a BOC group is liberated by treatment of 7 with anhydrous TFA. Then standard methods are used for introduction of the guanidine function.

Step B9:

The protecting groups are now removed from coupled product 7 by hydrogenolysis giving compound 8.

| Compound | Formula |
|---|---|
| 9 | BOC-ACHPA-$NR^{2c}R^{2d}$ |
| 10 | ACHPA-$NR^{2c}R^{2d}$ |
| 11 | $BOC_{AA}2_{\text{-ACHPA-NR}}2c_R2d$ |
| 12 | BOC-$AA^2$-$AA^1$-ACHPA-$NR^{2c}R^{2d}$ |
| 13 | $AA^2$-$AA^1$-ACHPA-$NR^{2c}R^{2d}$ |

Peptides of Formula I which contain statine are prepared as described above in routes A and B except that BOC-ACHPA-OEt is replaced with BOC-Sta-OEt. In this way, peptides such as 14 may be prepared.

| | |
|---|---|
| 14 | $X'$-(alkyl)-O-CO-$AA^2$-$AA^1$-Sta-$NR^{2c}R^{2d}$<br>or<br>$X'$-(alkyl)-CO-$AA^2$-$AA^1$-Sta-$NR^{2c}R^{2d}$ |

Peptides of Formula I which contain a 2-substituted statine or ACHPA element as a peptide bond mimic may be prepared as described above in Routes A and B except that BOC-ACHPA-OEt is replaced with a suitably protected 2-substituted ACHPA or 2-substituted statine derivative, for example BOC-(2-allyl)-ACHPA-OEt or BOC-(2Oisobutyl)Sta-OEt. In this way, peptides such as 15 may be prepared.

| | |
|---|---|
| 15 | $X'$-(alkyl)-O-CO-$AA^2$-$AA^1$-(2-isobutyl)ACHPA-$NR^{2c}R^{2d}$<br>or<br>$X'$-(alkyl)-CO-$AA^2$-$AA^1$-(2-isobutyl)ACHPA-$NR^{2c}R^{2d}$ |

Peptides of Formula I which contain a "reduced peptide bond" isostere such as Cal[$CH_2$NH]Val may also be prepared as described above in Routes A and B except that BOC-ACHPA-OEt is replaced with a suitably protected "reduced peptide bond" isostere, for example BOC-Cal[$CH_2$N(CBZ)]Val-OEt. In this case the CBZ protecting group of the isostere is removed by hydrogenolysis in step A9 or B9. In this way, peptides such as 16 may be prepared.

| | |
|---|---|
| 16 | X′-(alkyl)-O-CO-AA²-AA¹-Cal[$CH_2$NH]Val-$NR^{2c}R^{2d}$ or X′-(alkyl)-CO-AA²-AA¹-Cal[$CH_2$NH]Val-$NR^{2c}R^{2d}$ |

Peptides of Formula I which contain Cal[CH(OH)$CH_2$]Val as a peptide bond mimic are prepared as described below in Routes C and D.

Route C:

Step C1:

The BOC protecting group is removed from lactone 17 by treatment with anhydrous TFA, giving lactone 17a.

Step C2:

Lactone 17a is coupled with a BOC-protected amino acid (BOC-$AA^1$) giving 18a or with a N-terminal BOC-protected dipeptide (BOC-$AA^2$-$AA^1$) giving 18b. Compound 18b may also be prepared by treating 18a with anhydrous TFA, and coupling the product with BOC-$AA^2$. Compound 18b is treated with anhydrous TFA and the resulting product is treated first with carbonyldiimidazole and shortly thereafter with an alcohol component X′-(alkyl)-OH, producing carbamate 19a. Alternatively, the alcohol component X′-(alkyl)-OH is first treated with carbonyldiimidazole or with phosgene, and the resulting intermediate allowed to react with the product from TFA treatment (above), producing carbamate 19a The analogous acyl analogs 19b are prepared as described in Route A.

| | |
|---|---|
| 17 | BOC-Cal[CH(OH)$CH_2$]Val lactone |
| 17a | Cal[CH(OH)$CH_2$]Val lactone |
| 18a | BOC-$AA^1$-Cal[CH(OH)$CH_2$]Val lactone |
| 18b | BOC-$AA^2$-$AA^1$-Cal[CH(OH)$CH_2$]Val lactone |
| 19a | X′-(alkyl)-O-CO-$AA^2$-$AA^1$-Cal[CH(OH)$CH_2$]Val-lactone |
| 19b | X′-(alkyl)-CO-$AA^2$-$AA^1$-Cal[CH(OH)$CH_2$]Val lactone |

Step C3:

Lactone 19a (or 19b) is treated with aqueous potassium hydroxide to give the corresponding hydroxyacid. The hydroxyacid is treated with t-butyldimethylsilyl chloride and imidazole, then with HOAc in THF-H20, giving the protected hydroxyacid 20a (20b).

| | |
|---|---|
| 20a | X′-(alkyl)-0-CO-$AA^2$-$AA^1$-Cal[CH(OTBDMS)-$CH_2$]Val-OH |
| 20b | X′-(alkyl)-CO-$AA^2$-$AA^1$-Cal[CH(OTBDMS)$CH_2$]Val-OH |

Step C4:

20a (20b) is coupled with an amino component $R^{2c}R^{2d}NH$, giving 21a (21b). Additional reactive functional groups in the amino component $R^{2c}R^{2d}NH$ are protected with protecting groups such as CBZ for amino and guanidino groups, benzyl ethers for alcohols, and benzyl esters for carboxylic acids.

| | |
|---|---|
| 21a | X′-(alkyl)-O-CO-$AA^2$-$AA^1$-Cal[CH(OTBDMS)-$CH_2$]Val-$NR^{2c}R^{2d}$ |
| 21b | X′-(alkyl)-CO-$AA^2$-$AA^1$-Cal[CH(OTBDMS)$CH_2$]-Val-$NR^{2c}R^{2d}$ |

Step C5:

The silyl protecting group is removed from 21a (21b) by treatment with tetrabutylammonium fluoride in THF-DMF, giving 22a (22b).

| | |
|---|---|
| 22a(22c) | X′-(alkyl)-O-CO-$AA^2$-$AA^1$-Cal[CH(OH)$CH_2$]Val-$NR^{2c}R^{2d}$ |
| 22b(22d) | X′-(alkyl)-CO-$AA^2$-$AA^1$-Cal[CH(OH)$CH_2$]Val-$NR^{2c}R^{2d}$ |

Step C6:

It is understood that during the steps above, the reactive functional groups in the side-chains of the amino acids $AA^1$ and $AA^2$ are protected with protecting groups, which are removed during the hydrogenolysis of step C5. These protecting groups may be CBZ for amines and guanidines, and benzyl ethers for alcohols. When histidine is used as the $AA^1$ component, the side-chain imidazole ring may be protected during step C2 with a BOC protecting group. This protecting group is removed during treatment with TFA, and the imidazole ring left unprotected during subsequent N-terminal coupling or acylation reactions. Alternatively histidine may be protected with a DNP protecting group during step C2. This group is then removed in step C3 during the potassium hydroxide treatment.

Step C7:

In some cases a quaternized amino group is present in the C-terminal $R^{2c}R^{2d}N$- element of compound 22a (22b). In this case, a tertiary amine within $R^{2c}$ or $R^{2d}$ of $R^{2c}R^{2d}NH$ is quaternized by treatment of $R^{2c}R^{2d}N$-BOC with an alkyl halide and $KHCO_3$ in methanol or ethanol or with an alkyl halide in DMF. The resulting quaternary ammonium salt is then treated with anhydrous TFA to remove the BOC protecting group, and the resulting amine used in coupling step C4 above.

Alternatively, a compound 22a (22b) containing a tertiary amine in the $R^{2c}R^{2d}N$-element may be prepared as described above. If histidine is present as the $AA^1$ component, the imidazole ring is protected next by treatment with di-t-butyldicarbonate and $Et_3N$ in methanol or DMF. The tertiary amine present in the $R^{2c}R^{2d}N$-element is then quaternized by treatment with an alkyl halide in DMF.

Step C8:

In cases where a guanidine group is to be introduced into the $R^{2c}R^{2d}N$- component of an inhibitor of Formula I, compound 22a (22b) is prepared as described in steps C1 through C4 above. Step C7 may also be carried out if desired. Then an amine group present in the $R^{2c}R^{2d}N$- component and hetetofore protected with a BOC group is liberated by treatment of 22a (22b) with anhydrous TFA. Then standard methods are used for introduction of the guanidine function.

Step C9:

Protecting groups are removed from 22a (22b) by hydrogenolysis, giving 22c (22d).

Route D:

Step D1:

Lactone 17 is treated with potassium hydroxide to give the corresponding hydroxyacid 23. The hydroxyacid 23 is treated with t-butyldimethylsilyl chloride and imidazole, then with HOAc in THF-$H_2O$, giving the protected hydroxyacid 24.

| | |
|---|---|
| 17 | BOC-Cal[CH(OH)$CH_2$]Val lactone |
| 23 | BOC-Cal[CH(OH)$CH_2$]Val-OH |
| 24 | BOC-Cal[CH(OTBDMS)$CH_2$]Val-OH |

Step D2:

Protected hydroxyacid 24 is coupled with an amino component $R^{2c}R^{2d}NH$, giving 25. Additional reactive functional groups in the amino component $R^{2c}R^{2d}NH$ are protected with protecting groups such as CBZ for amino and guanidino groups, benzyl ethers for alcohols, and benzyl esters for carboxylic acids. Step D3: The BOC protecting group is removed from 25 by treatment with anhydrous TFA, giving 26.

| | |
|---|---|
| 25 | BOC-Cal[CH(OTBDMS)$CH_2$]Val-$NR^{2c}R^{2d}$ |
| 26 | Cal[CH(OTBDMS)$CH_2$]Val-$NR^{2c}R^{2d}$ |

Step D4:

Compound 26 is coupled with a BOC-protected amino acid (BOC-$AA^1$) or a dipeptide with an N-terminal BOC protecting group (BOC-$AA^2$-$AA^1$), giving coupled product 27.

| | |
|---|---|
| 27 | BOC-$AA^2$-$AA^1$-Cal[CH(OTBDMS)$CH_2$]Val-$NR^{2c}R^{2d}$ |

Step D5:

The N-terminal BOC protecting group of 27 is removed by treatment with anhydrous TFA in $CH_2Cl_2$, giving 28.

| | |
|---|---|
| 28 | $AA^2$-$AA^1$-Cal[CH(OTBDMS)$CH_2$]Val-$NR^{2c}R^{2d}$ |

Step D6:

Compound 28 is treated first with carbonyldiimidazole and shortly thereafter with an alcohol component $X'$-(alkyl)-OH, producing carbamate 29a. Alternatively, the alcohol component $X'$-(alkyl)-OH is first treated with carbonyldiimidazole or with phosgene, and the resulting intermediate allowed to react with 28, producingcarbamate 29a. Alternatively, acid $X'$-(alkyl)-$CO_2H$ may be coupled to 28 to give 29b. Additional reactive functional groups in the $X'$-(alkyl)-OH or $X'$-(alkyl)-CO elements are protected with protecting groups as described above.

| | |
|---|---|
| 29a | X′-(alkyl)-O-CO-AA$^2$-AA$^1$-Cal[CH(OTBDMS)$CH_2$]Val-NR$^{2c}$R$^{2d}$ |
| 29b | X′-(alkyl)-CO-AA$^2$-AA$^1$-Cal[CH(OTBDMS)$CH_2$]Val-NR$^{2c}$R$^{2d}$ |

Step D7:

The silyl protecting group is removed from 29a (29b) by treatment with fluoride, giving 22a (22b).

Step D8:

In some cases a quaternized amino group is present in the C-terminal $R^{2c}R^{2d}N$- element of compound 22a (22b). In this case, a tertiary amine within $R^{2c}$ or $R^{2d}$ of $R^{2c}R^{2d}NH$ is quaternized by treatment of $R^{2c}R^{2d}N$-BOC with an alkyl halide and $KHCO_3$ in methanol or ethanol or with an alkyl halide in DMF. The resulting quaternary ammonium salt is then treated with anhydrous TFA to remove the BOC protecting group, and the resulting amine used in coupling step D2 above.

Alternatively, a compound 22a (22b) containing a tertiary amine in the $R^{2c}R^{2d}N$-element may be prepared as described above. If histidine is present as the AA$^1$ component, the imidazole ring is protected next by treatment with di-t-butyldicarbonate and $Et_3N$ in methanol or DMF. The tertiary amine present in the $R^{2c}R^{2d}N$-element is then quaternized by treatment with an alkyl halide in DMF.

Step D9:

In cases where a guanidine group is to be introduced into the $R^{2c}R^{2d}N$- component of a compound of Formula I, compound 22a (22b) is prepared as described above. Step D8 may be carried out next if desired. Then an amine group present in the $R^{2c}R^{2d}N$- component and hetetofore protected with a BOC group is liberated by treatment of 22a (22b) with anhydrous TFA. Then standard methods are used for introduction of the guanidine function.

Step D10:

It is understoood that during the steps above, the reactive functional groups in the side-chains of amino acid components are protected with protecting groups, which are removed in the above hydrogenolysis step. These protecting groups may be CBZ fcr amines and guanidines, and benzyl ethers for alcohols. If histidine is present as AA$^1$, the side-chain imidazole ring is protected with a BOC group which is removed by TFA in step D3. Thereafter the side-chain of histidine is left unprotected. Alternatively, histidine with a DNP protecting group may be used. In this case, the DNP group is removed after step D11 by treatment of 22c (22d) with thiophenol.

Step D11:

Protecting groups are removed from 22a (22b) by hydrogenolysis, giving 22c (22d).

The novel peptides of the present invention possess a high degree of activity in treating renin-associated hypertension, hyperaldosteronism and/or congestive heart failure in humans, as well as in other warm-blooded animals such as mice, rats, horses, dogs and cats.

For these purposes, the peptides of the present invention may be administered orally, parenterally (including subcutaneous injections, intravenous, intramuscular, intrasternal injection or infusion techniques), by inhalation spray, or rectally, in dosage unit formulations containing conventional non-toxic pharmaceutically-acceptable carriers, adjuvants and vehicles.

Thus, in accordance with the present invention there is further provided a method of treating and a pharmaceutical composition for treating renin-associated hypertension, hyperaldosteronism, and/or congestive heart failure. This treatment involves administering to a patient in need of such treatment a pharmaceutical composition comprising a pharmaceutical carrier and a therapeutically-effective amount of a peptide of

the formula:

$$\begin{array}{c} R^7 \\ (CH_2)_r \quad O \\ A\text{-}B\text{-}E\text{-}N\text{-}CH\text{-}Q\text{-}CH\text{-}C\text{-}J \\ H \qquad R^4 \end{array} \quad (I),$$

wherein A, B, $R^{1a}$, $R^1$, $R^7$, Q, $R^4$, r and J are defined above, or a pharmaceutically-acceptable salt thereof.

These pharmaceutical compositions may be in the form of orally-administrable suspensions or tablets; nasal sprays; sterile injectable preparations, for example, as sterile injectable aqueous or oleagenous suspensions; or suppositories.

When administered orally as a suspension, these compositions may contain microcrystalline cellulose for imparting bulk, alginic acid or sodium alginate as a suspending agent, methylcellulose as a viscosity enhancer, and sweetners/flavoring agents known in the art. As immediate release tablets, these compositions may contain microcrystalline cellulose, dicalcium phosphate, starch, magnesium stearate and lactose and/or other excipients, binders, extenders, disintegrants, diluents and lubricants known in the art.

When administered by nasal aerosol or inhalation, these compositions may be prepared as solutions in saline, employing benzyl alcohol or other suitable preservatives, absorption promoters to enhance bioavailability, flourocarbons, and/or other solubilizing or dispersing agents known in the art.

The injectable solutions or suspensions may be formulated according to known art, using suitable non-toxic, parenterally-acceptable diluents or solvents, such as mannitol, 1,3-butanediol, water, Ringer's solution or isosteric sodium chloride solution, or suitable dispersing or wetting and suspending agents, such as sterile, bland, fixed oils, including synthetic mono- or diglycerides, and fatty acids, including oleic acid.

When rectally administered in the form of suppositories, these compositions may be prepared by mixing the drug with a suitable non-irritating excipient, such as cocoa butter, synthetic glyceride esters or polyethylene glycols, which are solid at ordinary temperatures, but liquify and/or dissolve in the rectal cavity to release the drug.

Dosage levels of the order of 0.02 to 2.0 grams-per-day are useful in the treatment of the above-indicated conditions, with oral doses two-to-five times higher. For example, renin-associated hypertension and hyperaldosteronism are effectively treated by the administration of from 10 to 50 milligrams of the compound per kilogram of body weight from one to three times per day. It will be understood, however, that the specific dose level and frequency of dosage for any particular patient will depend upon a variety of factors including the activity of the specific compound employed, the metabolic stability and length of action of that compound, the age, body weight, general health, sex, diet, mode and time of administration, rate of excretion, drug combination the severity of the particular condition, and the host undergoing therapy.

The present invention is also directed to combinations of the novel renin-inhibitory peptides of Formula I with one or more antihypertensive agents selected from the group consisting of diuretics, α-and/or β-adrenergic blocking agents. CNS-acting agents, adrenergic neuron blocking agents, vasodilators, angiotensin I converting enzyme inhibitors, calcium channel blockers, and other antihypertensive agents.

For example, the compounds of this invention can be given in combination with such compounds or salt or other derivative forms thereof as:

Diuretics:

acetazolamide; amiloride; bendroflumethiazide; benzthiazide; bumetanide; chlorothiazide; chlorthalidone; cyclothiazide; ethacrynic acid; furosemide; hydrochlorothiazide; hydroflumethiazide; indacrinone (racemic mixture, or as either the (+) or (-) enantiomer alone, or a manipulated ratio, e.g., 9:1 of said enantiomers, respectively); metolazone; methyclothiazide; muzolimine; polythiazide; quinethazone; sodium ethacrynate; sodium nitroprusside; spironolactone; ticrynafen; triamterene; trichlormethiazide;

α-Adrenergic Blocking Agents:

dibenamine; phentolamine; phenoxybenzamine; prazosin; tolazoline;

β-Adrenergic Blocking Agents:

atenolol; metoprolol; nadolol; propranolol; timolol;
((±)-2-[3-(tert-butylamino)-2-hydroxypropoxy]-2-furan-anilide) (ancarolol);
(2-acetyl-7-(2-hydroxy-3-isopropylaminopropoxy)benzofuran HCl) (befunolol);
((±)-1-(isopropylamino)-3-(p-(2-cyclcpropylmethoxyethyl)-phenoxy)-2-propranol HCl) (betaxolol);
(1-[(3,4-dimethoxyphenethyl)amino]-3-(m-tolyloxy)-2-propanol HCl) (bevantolol);
((±)-1-(4 ((2-isopropoxyethoxy)methyl)phenoxy)-3-isopropylamino-2-propanol)fumarate) (bisoprolol);
(4-(2-hydroxy-3-[4-(phenoxymethyl)-piperidino]propoxy)-indole);
(carbazolyl-4-oxy-5,2-(2-methoxyphenoxy)-ethylamino-2-propanol);
(1-((1,1-dimethylethyl)amino)-3-((2-methyl-1H-indol-4-yl)oxy)-2-propanol benzoate) (bopindolol);
(1-(2-exobicyclo[2.2.1]-hept-2-ylphenoxy)-3-[(1 methylethyl)-amino]-2-propanol HCl) (bornaprolol);
(o-[2-hydroxy-3-[(2-indol-3-yl-1,1-dimethylethyl)amino]propoxy]benzonitrile HCl) (bucindolol);
(α-[(tert.butylamino)methyl]-7-ethyl-2-benzofuran-methanol) (bufuralol);
(3-[3-acetyl-4-[3-(tert.butylamino)-2-hydroxypropyl]phenyl]-1,1-diethylurea HCl) (celiprolol);
((±)-2-[2-[3-[(1,1-dimethylethyl)amino]-2-hydroxypropoxy]phenoxy]-N-methylacetamide HCl) (cetamolol);
(2-benzimidazolyl-phenyl(2-isopropylaminopropanol));
((±)-3′-acetyl-4′-(2-hydroxy-3-isopropylaminopropoxy)acetanilide HCl) (diacetolol);
(methyl-4-[2-hydroxy-3-[(1-methylethyl)aminopropoxy]]benzenepropanoate HCl) (esmolol);
(erythro-DL-1-(7-methylindan-4-yloxy)-3-isopropylaminobutan-2-ol);
(1-(tert.butylamino)-3-[O-(2-propynyloxy)phenoxy]-2-propanol (pargolol);
(1-(tert.butylamino)-3-[o-(6-hydrazino-3-pyridazinyl)phenoxy]-2-propanol diHCl) (prizidilol);
((-)-2-hydroxy-5-[(R)-1-hydroxy-2-[(R)-(1-methyl-3-phenylpropyl)amino]ethyl]benzamide);
(4-hydroxy-9-[2-hydroxy-3-(isopropylamino)-propoxy]-7-methyl-5H-furo[3,2-g][1]-benzopyran-5-one) (iprocrolol);
((-)-5-(tert.butylamino)-2-hydroxypropoxy]-3,4-dihydro-1-(2H)-naphthalenone HCl) (levobunolol);
(4-(2-hydroxy-3-isopropylamino-propoxy)-1,2-benzisothiazole HCl);
(4-[3-(tert.butylamino)-2-hydroxypropoxy]-N-methylisocarbostyril HCl);
((±)-N-2-[4-(2-hydroxy-3-isopropyl aminopropoxy)phenyl]ethyl-N′-isopropylurea) (pafenolol);
(3-[[(2-trifluoroacetamido)ethyl]amino]-1-phenoxypropan-2-ol);
(N-(3-(o-chlorophenoxy)-2-hydroxypropyl)-N′-(4′-chloro-2,3-dihydro-3-oxo-5-pyridazinyl)ethylenediamine);
((±)-N-[3-acetyl-4-[2-hydroxy-3-[(1-methylethyl)amino]propoxy]phenyl]butanamide) (acebutolol);
((±)-4′-[3-(tert-butylamino)-2-hydroxypropoxy]spiro[cyclohexane-1,2′-indan]-1-one) (spirendolol);
(7-[3-[[2-hydroxy-3-[(2-methylindol-4-yl)oxy]propyl]amino]butyl]thiophylline) (teoprolol);
((±)-1-tert.butylamino-3-(thiochroman-8-yloxy)-2-propanol) (tertatolol);
((±)-1-tert.butylamino-3-(2,3-xylyloxy)-2-propanol HCl) (xibenolol);
(8-[3-(tert.butylamino)-2-hydroxypropoxy]-5-methylcoumarin) (bucumolol);
(2-(3-(tert.butylamino)-2-hydroxy-propoxy)benzonitrile HCl) (bunitrolol);
((±)-2′-[3-(tert-butylamino)-2-hydroxypropoxy-5′-fluorobutyrophenone) (butofilolol);
(1-(carbazol-4-yloxy)-3-(isopropylamino)-2-propanol) (carazolol);
(5-(3-tert.butylamino-2-hydroxy)propoxy-3,4-dihydrocarbostyril HCl) (carteolol);
(1-(tert.butylamino)-3-(2,5-dichlorophenoxy)-2-propanol) (cloranolol);
(1-(inden-4(or 7)-yloxy)-3-(isopropylamino)-2-propanol HCl) (indenolol);
(1-isopropylamino-3-[(2-methylindol-4-yl)oxy]-2-propanol) (mepindolol);
(1-(4-acetoxy-2,3,5-trimethylphenoxy)-3-isopropylaminopropan-2-ol) (metipranolol);
(1-(isopropylamino)-3-(o-methoxyphenoxy)-3-[(1-methylethyl)amino]-2-propanol) (moprolol);
((1-tert.butylamino)-3-[(5,6,7,8-tetrahydro-cis-6,7-dihydroxy 1-naphthyl)oxy]-2-propanol) (nadolol);
((S)-1-(2-cyclopentylphenoxy)-3-[(1.1-dimethylethyl)amino]-2-propanol sulfate (2:1)) (penbutolol);
(4′-[1-hydroxy-2-(amino)ethyl]methanesulfonanilide) (sotalol);
(2-methyl-3-[4-(2-hydroxy-3tert.butylaminopropoxy)phenyl]-7-methoxy-isoquinolin-1-(2H)-one);
(1-(4-(2-(4-fluorophenyloxy)ethoxy)phenoxy)-3-isopropylamino-2-propanol HCl);
((-)-p-[3-[(3,4-dimethoxyphenethyl)amino]-2-hydroxypropoxy]-β-methylcinnamonitrile) (pacrinolol);
((±)-2-(3′-tert.butylamino-2′-hydroxypropylthio)-4-(5′-carbamoyl-2′-thienyl)thiazole HCl) (arotinolol);
((±)-1-[p-[2-(cyclopropylmethoxy)ethoxy]phenoxy]-3-(isopropylamino)-2-propanol) (cicloprolol);
((±)-1-[(3-chloro-2-methylindol-4-yl)oxy]-3-[(2-phenoxyethyl)amino]-2-propanol) (indopanolol);
((±)-6-[[2-[[3-(p-butoxyphenoxy)-2-hydroxypropyl]amino]ethyl]amino]-1,3-dimethyluracil) (pirepolol);
(4-(cyclohexylamino)-1-(1-naphtholenyloxy)-2-butanol);
(1-phenyl-3-[2-[3-(2-cyanophenoxy)-2-hydroxypropyl]aminoethyl]hydantoin HCl);

(3,4-dihydro-8-(2-hydroxy-3-isopropylaminopropoxy)-3-nitroxy-2H-1-benzopyran) (nipradolol);

α- and β-Adrenergic Blocking Agents:

((±)-1-tert-butylamino)-3-[o-[2-(3-methyl-5-isoxazolyl)vinyl]phenoxy]-2-propanol) (isoxaprolol);
(1-isopropylamino-3-(4-(2-nitroxyethoxy)phenoxy)-2-propanol HCl);
(4-hydroxy-α-[[3-(4-methoxyphenyl)-1-methylpropyl]aminomethyl]-3-(methylsulfinyl)-benzmethanol HCl) (sulfinalol);
(5-[1-hydroxy-2-[[2-(o-methoxyphenoxy)ethyl]amino]ethyl]-2-methylbenzenesulfonamide HCl);
(5-[1-hydroxy-2-[(1-methyl-3-phenylpropyl)amino]ethyl]salicylamide HCl) (labetalol);
(1-((3-chloro-2-methyl-1H-indol-4-yl)oxy)-3-((2-phenoxyethyl)amino)-2-propanol-hydrogenmalonate) (ifendolol);
(4-(2-hydroxy-3-[(1-methyl-3-phenylpropyl)amino]propoxy)benzeneacetamide);
(1-[3-[[3-(1-naphthoxy)-2-hydroxypropyl]-amino]-3,3-dimethyl-propyl]-2-benzimidazolinone);
(3-(1-(2-hydroxy-2-(4-chlorophenylethyl)-4-piperidyl)-3,4-dihydroxy)quinoxolin-2(1H)-one);

CNS-Acting Agents:

clonidine; methyldopa;

Adrenergic Neuron Blocking Agents:

guanethidine; reserpine and other rauwolfia alkaloids such as rescinnamine;

Vasodilators:

diazoxide; hydralazine; minoxidil;

Angiotensin I Converting Enzyme Inhibitors:

1-(3-mercapto-2-methyl-1-oxcpropyl)-L-proline (captopril);
(1-(4-ethoxycarbonyl-2,4(R,R)-dimethylbutanoyl)indoline-2(S)-carboxylic acid);
(2-[2-[[1-ethoxycarbonyl)-3-phenyl-propyl]amino]-1-oxopropyl]-1,2,3,4-tetrahydro-3-isoquinolinecarboxylic acid);
((S)-1-[2-[[1-(ethoxycarbonyl)-3-phenylpropyl]amino]-1-oxopropyl]octahydro-1H-indole-2-carboxylic acid HCl);
(N-cyclopentyl-N-(3-(2,2-dimethyl-1-oxopropyl)thiol-2-methyl-1-oxopropyl)glycine) (pivalopril);
((2R,4R)-2-(2-hydroxyphenyl)-3-(3-mercaptopropionyl)-4-thiazolidinecarboxylic acid);
(1-(N-[1(S)-ethoxycarbonyl-3-phenylpropyl]-(S)-alanyl)cis,syn-octahydroindol-2(S)-carboxylic acid HCl);
((-)-(S)-1-[(S)-3-mercapto-2-methyl-1-oxopropyl]indoline-2-carboxylic acid);
([1(S),4S]-1-[3-(benzoylthio)-2-methyl-1-oxopropyl]-4-phenylthio-L-proline;
(3-([1-ethoxycarbonyl-3-phenyl-(1S)-propyl]amino)-2,3,4,5-tetrahydro-2-oxo-1-(3S)-benzazepine-1-acetic acid HCl);
(N-(2-benzyl-3-mercaptopropanoyl)-S-ethyl-L-cysteine) and the S-methyl analogue;
(N-(1(S)-ethoxycarbonyl-3-phenylpropyl)-L-alanyl-L-proline maleate) (enalapril);
N-[1-(S)-carboxy-3-phenylpropyl]-L-alanyl-1-proline;
$N^2$-[1-(S)-carboxy-3-phenylpropyl]-L-lysyl-L-proline (lisinopril);

Calcium Channel Blockers:

α-[3-[[2-(3,4-dimethoxyphenyl)ethyl]methylamino]propyl]-3,4-dimethoxy-α-(1-methylethyl)benzeneacetonitrile (verapamil);
1,4-dihydro-2,6-dimethyl-4-(2-nitrophenyl)-3,5-pyridinedicarboxylic acid dimethyl ester (nifedipine);
2-(2,2-dicyclohexylethyl)piperidine (perhexiline);
N-(1-methyl-2-phenylethyl)-phenylbenzenepropanamine (prenylamine);
3-(aminosulfonyl)-4-chloro-N-(2,3 dihydro-2-methyl-1H-indol-1-yl)benzamide (indapamide);
(2′-(2-diethylaminoethoxy)-3-phenylpropiophenone (etafenone);
(4-[4,4-bis-(4-fluorophenyl)butyl]-N-(2,6-dimethylphenyl)-1-piperazineacetamide) (lidoflazine);

(2-(N-benzyl-N-methylamino)ethylmethyl-2,6-dimethyl-4-(m-nitrophenyl)-1,4-dihydro-3,5-pyridinedicarboxylate HCl) (nicardipine);
(N-(3,4-dimethoxyphenethyl)-2-(3,4-dimethoxyphenyl)-N-methyl-m-dithiane-2-propylamine-1,1,3,3-tetraoxide) (tiapamil);
(5,6-dimethoxy-2-(3-[(α-(3,4-dimethoxy)phenylethyl)methylamino]propyl)phthalimidine) (falipamil);
(β-[(2-methylpropoxy)methyl]-N-phenyl-N-phenylmethyl-1-pyrrolidineethanamine HCl monohydrate) (bepridil);
((+)-cis-3-(acetyloxy)-5-[2-(dimethylamino)ethyl]-2,3-dihydro-2-(4-methoxyphenyl)-1,5-benzothiazepin-4-(5H)-one) (diltiazem);
((E)-1-[bis-(p-fluorophenyl)methyl]-4-cinnamylpiperazine di HCl) (flunarizine);
(5-[(3,4-dimethoxyphenethyl)methylamino]-2-isopropyl-2-(3,4,5-trimethoxyphenyl)valeronitrile (gallopamil);
(ethylmethyl(2,3-dichlorophenyl)-1,4-dihydro-2,6-dimethyl-3,5-pyridinedicarboxylate (felodipine);
(isopropyl-2-methoxyethyl-1,4-dihydro-2,6-dimethyl-4-(3-nitrophenyl)-3,5-pyridinecarboxylate) (nimodipine);
(3-ethyl-5-methyl-1,4-dihydro-2,6-dimethyl-4-(3-nitrophenyl)-3,5-pyridine-dicarboxylate) (nitrendipine);

Other Antihypertensive Agents:

aminophylline; cryptenamine acetates and tannates; deserpidine; meremethoxylline procaine; pargyline; trimethaphan camsylate; and the like, as well as admixtures and combinations thereof.

Typically, the individual daily dosages for these combinations can range from about one-fifth of the minimally-recommended clinical dosages to the maximum recommended levels for the entities when they are given alone. Coadministration is most readily accomplished by combining the active ingredients into a suitable unit dosage form containing the proper dosages of each. Other methods of coadministration are, of course, possible.

The renin-inhibitory novel peptides of the present invention may also be utilized in in vivo or in vitro diagnostic methods for the purpose of establishing the significance of renin as a causative or contributory factor in hypertension, hyperaldosteronism or congestive heart failure in a particular patient.

In the in vivo method, a novel peptide of the present invention is administered to a patient, preferably by intravenous injection, although parenteral administration is also suitable, at a hypotensive dosage level in a single dose of from 0.1 to 10 mg per kg of body weight, and the resulting transitory fall in blood pressure, if it occurs, indicates supranormal plasma renin levels.

In vitro methods which may be employed involve incubating a body fluid, preferably plasma, with a novel peptide of the present invention according to methods described in Boger et al., J. Med. Chem., 1985, 28, 1789-1790.

Pepstatin may be employed in the methods described above as an active control (see, e.g., U.S. Patent Nos. 3,784,686 and 3,873,681 for a description of the use of pepstatin in diagnostic methods of this type).

The following examples are intended to be representative and not limiting.

## Example 1

N-[(2-t-butoxycarbonylamino)-ethoxycarbonyl]-L-phenylalanine methyl ester

3.60g (16.7mmol) L-phenylalanine methyl ester hydrochloride (Sigma) was suspended in 5mL dry DMF at 0° and treated with 3.36g (27.5mmol,1.6eq) 4-dimethylaminopyridine followed by 4.43g (27.3mmol,1.6eq) N,N′-carbonyldiimidazole. After 30min. at 0° and 1h. at room temperature, a solution of 4.162g (25.8mmol,1.5eq) BOC-aminoethanol (Sigma) in 3mL dry DMF was added. The mixture was heated at 90° for 2h. then cooled and diluted into 100mL ethyl acetate. The solution was washed with 0.1N HCl (4x), 5% $NaHCO_3$ (1x) and brine (1x); dried over $MgSO_4$, filtered and volatiles were removed in vacuo. The crude product was purified by medium pressure liquid chromatography, eluting with hexanes/ethyl acetate (1:1) to afford 4.42g (12.1mmol,72%) of the title compound as a viscous oil. NM, (300MHz, $CDCl_3$): 7.1 7.3 (m,5H), 5.35 (br d,9Hz,1H), 4.90 (br s, 1H), 4.58 (g,8Hz,1H), 4.05 (t,6Hz,2H), 3.68 (s,3H), 3.28 (m,2H), 3.05 (m,2H), 1.40 (s,9H). FAB-MS: 367 (M+H,25%), 267 (M-BOC+H,100%).

## Example 2

N-[(2-t-butylacetylamino)-ethoxycarbonyl]-L-phenylalanine

2.576g (7.04mmol) of N-[(2-t-butoxycarbonylamino)-ethoxycarbonyl]-L-phenylalanine methyl ester, prepared as described in Example 1, was dissolved in 3mL $CH_2Cl_2$ at room temperature and treated with 4mL of HCl-saturated methanol solution. After 3 hours at room temperature, all volatiles were removed in vacuo and the residue dried under high vacuum overnight.

The crude prcduct from above was re-dissolved in 10mL dry $CH_2Cl_2$, cooled to 0° and treated with 2.58g (21.2mmol, 3eq) 4-dimethylamino-pyridine (Aldrich) followed by 1.03mL (.998g, 7.41mmol, 1.05eq) t-butylacetyl chloride (Aldrich). The mixture was maintained at 0° for 2 hours then warmed to room temperature and continued for another hour. The mixture was diluted with 100mL ether, washed with 1N HCl, 5% $NaHCO_3$, and brine; then dried over $MgSO_4$, filtered and stripped. The crude product was purified by medium pressure liquid chromatography, eluting with EtOAc, to afford 1.92g (5.27mmol, 75%) of the product as a colorless oil that slowly solidified on standing.

The product obtained above (1.92g, 5.27mmol) was dissolved in 5mL absolute methanol, cooled to 0° and treated with 5.5ml of 1.0M NaOH (5.5mmol,1.05eq). After 30min. at 0°, the mixture was warmed to room temperature and stirred another hour. The reaction mixture was diluted with 20mL distilled water and washed with ether (3x). The aqueous layer was cooled to 0° and acidified (pH 2, paper) by the dropwise addition of 6N HCl. The mixture was extracted with ethyl acetate (4x); the combined extracts washed with brine, dried over $MgSO_4$, filtered and solvent removed in vacuo to afford 1.75g (5.00mmol, 95%) of the title compound as a white solid. Rf ($CHCl_3$/$CH_3OH$/$H_2O$/HOAc; 100:20:3:.5): .32. NMR (300MHz, $CD_3OD$): 7.2 (m,5H), 4.39 (dd,1H), 4.02 (m,2H), 3.36(t,2H,7Hz), 3.18(dd,1H), 2.92(dd,1H), 2.03(s,2H), 0.98(s,9H). FAB-MS: 351 (M+H, 100%).

## Example 3

N-[(2-ethoxycarbonylamino)-ethoxycarbonyl]-L-phenylalanine

The title compound was synthesized from N-[(2-t-butoxycarbonylamino)-ethoxycarbonyl]-L-phenylalanine methyl ester (Example 1) and ethyl chloroformate according to the procedure described in Example 2. NMR (300MHz, $CD_3OD$): 7.2 (m,5H), 4.30 (m,1H), 4.0 (m,4H), 3.3 (m,3H), 2.95 (dd,1H), 1.22 (t,6Hz,3H). FAB-MS: 325 (M+H,100%).

## Example 4

N-[3-(dimethylamino)-1-propyloxycarbonyl]-L-phenylalanine

2.00g (6.85mmol) L-phenylalanine benzyl ester hydrochloride (Fluka) was suspended in 5mL dry DMF at 0° and treated with 1.67g ( 13.7mmol, 2eq) 4-dimethylaminopyridine followed by 1.222g (7.54mmol,1.1eq) N,N′-carbonyldiimidazole. After 30min. at 0° and 1h. at room temperature, 1.62mL of 3-dimethylamino-1-propanol (1.41g, 13.7mmol, 2eq) was added and the mixture heated to 85° for 6hr. The mixture was then cooled and added to 100mL EtOAc, washed with pH 7.0 phosphate buffer (4x), brine (1x), dried over $MgSO_4$, filtered and volatiles were removed in vacuo. The crude product was purified by medium pressure liquid chromatography, eluting with $CHCl_3$/MeOH/$NH_4OH$ (800:50:3) to afford 1.23g (3.20mmol,47%) of the product as a viscous oil.

The product obtained above (ca. 0.5g) was dissolved in 4mL EtOAc and several drops of concentrated hydrochloric acid added. The solution was hydrogenated over 100mg of 10% Pd/C at 40psi overnight. The mixture was filtered through Celite and the filtrate evaporated to afford the product as a pale yellow gum. NMR (200MHz, $CD_3OD$): 7.2 (m,5H), 4.35 (m,1H), 4.07 (m,2H), 3.1 (m,4H), 2.83 (s,6H), 2.0 (m,2H). FAB-MS: 295 (M+H,100%).

## Example 5

N-[(2-acetylamino)-ethoxycarbonyl]-L-phenylalanine

The title compound was synthesized from N-[(2-t-butoxycarbonylamino)-ethoxycarbonyl]-L-phenylalanine methyl ester (Example 1) and acetic anhydride according to the procedure described in Example 2. NMR (300MHz, $CD_3OD$): 7.2 (m,5H), 4.40 (dd,1H), 4.0 (m,2H), 3.35 (t,6Hz,2H), 3.18 (dd,1H), 2.92 (dd,1H), 1.90 (s,3H). FAB-MS: 295 (M+H,100%).

## Example 6

N-[N-t-butylacetyl-B-alanyl]-L-phenylalanine $t\text{-}BuCH_2C(O)NHCH_2CH_2C(O)\text{-}Phe$

370mg (1.0mmol) N-[N-Cbz-B-alanyl]-L-phenylalanine (Sigma) was suspended in 5mL dry $CH_2Cl_2$ in a clean, scratch-free round bottom flask. Ethereal diazomethane was added slowly until all solids had dissolved and a yellow color persisted. Excess diazomethane was destroyed in situ by the dropwise addition of glacial acetic acid until all color had disappeared. The mixture was filtered and stripped of volatiles under vacuum.

The crude methyl ester from above was dissolved in 5mL MeOH and 0.5mL concentrated HCl added. The mixture was hydrogenated over 75mg of 10% Pd/C at 40psi overnight. The catalyst was filtered off through a layer of Celite and the filtrate stripped of solvent under vacuum to afford 273mg (.96mmol) of product.

This material was taken up in 3mL $CH_2Cl_2$, cooled to 0° under nitrogen, and treated with .159mL (154mg,1.15mmol,1.2eq) t-butylacetyl chloride (Aldrich) and 350mg (2.86mmol,3eq) DMAP. The mixture was stirred at 0° for one hour then added to 50mL EtOAc, washed with 1N HCl (3x) and brine (1x), dried over $MgSO_4$.

## Example 7

Preparation of BOC-Phe-His-ACHPA-NH-2(S)-MeBu

To a solution of 6.13 g (10.0 mmole) of BOC-Phe-His-ACHPA-$NHNH_2$ in 70 ml DMF at -30°C was added 6 M HCl until the pH was adjusted to 2.0. Isoamyl nitrite (1.47 ml, 11.0 mmole) was added and the reaction mixture stirred for 3 hours while monitoring for the presence of excess oxidising agent with starch/iodine paper. Full conversion to the acyl azide was monitored by TLC (85:14:1 $CHCl_3$ MeOH, $NH_4OH$). 2(S)-methylbutyl amine (1.26 ml, 11.0 mmol) was then added followed by dropwise addition of diisopropyl ethyl amine until the pH of the reaction mixture had reached 9.0. The solution was stored in a cold room for 2 days at 0°C. The solution was concentrated in ininin vacuo and the residue taken up in 25 ml of $CH_2Cl_2$, washed with water (3x5 ml) and saturated NaCl (1x5 ml) and dried over $MgSO_4$. The solution was filtered, and concentrated in vacuo and the residue applied to a 2.0"x 7' column packed with LH20 eluting with MeOH. The first material to elute corresponded to the desired product (4.88 g, 7.3 mmole, 73%). Fast atom bombardment (FAB) showed M++H=669, M+=668 which fits for $C_{36}H_{56}N_6O_6$.

## Example 8

$BOCNHCH_2CH_2OC(O)$-Phe-His-ACHPA-2(S)-methylbutylamide

200mg (.26mmol) BOC-Phe-(BOC)His-ACHPA-2(S)-methylbutylamide (prepared as described in Example 7) was dissolved in 1.5mL MeOH and treated with 1.5mL of HCl-saturated MeOH. After 1 hour at room temperature, an additional 1mL of HCl/MeOH was added and the reaction continued at room temperature for another hour at which time all volatiles were removed under vacuum and the residue further evacuated overnight.

N-t-BOC-Aminoethanol (Sigma; 57mg, .35mmol, 1.4eq) was dissolved in 2mL $CH_2Cl_2$ and cooled to -10° under nitrogen. N,N′-carbonyldiimidazole (65mg,.40mmol, 1.5eq) was added and the mixture stirred at -10° for 1.5hrs. The product from above was dissolved in 2mL $CH_2Cl_2$ and .11mL (80mg, .79mmol, 3eq) of triethylamine added. This mixture was added to the BOC-aminoethanol/CDI mixture at -10° and the resulting mixture allowed to warm gradually to room temperature. The mixture was stirred at room temperature for 72hrs.

The mixture was diluted into 50mL ether and washed with pH 7.0 phosphate buffer (2x) and brine. The ether layer was dried over $MgSO_4$, filtered and stripped. The title compound was isolated as a white powder. Partial NMR (300MHz, $CD_3OD$): 7.60 (s,1H), 7.25 (m,5H), 6.91 (s,1H), 4.57 (t,6Hz,1H), 4.32 (dd,1H), 3.95 (m,4H), 2.20 (d,7Hz,2H), 1.43 (s,9H). FAB-MS: 756 (M+H,100%).

## Example 9

t-$BuCH_2CONHCH_2CH_2OC(O)$-Phe-His-ACHPA-2(S)-methylbutylamide

156mg (.227mmol) of BOC(DNP)His-ACHPA-2(S)-methylbutylamide was dissolved in 4mL $CH_2Cl_2$ at room temperature and treated with 5mL HCl-saturated MeOH for 3 hrs. at which time all volatiles were removed under vacuum. The residue was further evacuated on a vacuum line for 3 hrs.

The product from above was suspended in 5mL dry $CH_2Cl_2$ at 0° and treated with 87mg (.249mmol, 1.1eq) N-[(2-t-butylacetylamino)-ethoxycarbonyl]-L-phenylalanine (prepared as described in Example 2), .20mL diisopropylethylamine (148mg, 1.15mmol, 5eq) and finally, 150mg benzotriazol-1-yloxytris-(dimethylamino)phosphonium hexafluorophosphate ("BOP" reagent, Aldrich, .34mmole 1.5eq). The mixture was stirred at 0° for 3 hrs., then warmed to room temperature and stirred an additional hour.

The mixture was diluted into 50mL EtOAc; washed with 0.1N HCl (2x), 5% $NaHCO_3$ (3x) and brine (1x). The organic layer was dried over $MgSO_4$, filtered and solvents removed under vacuum. The crude product was purified by medium pressure liquid chromatography, eluting with EtOAc/MeOH/MeCN (9:1:1), to afford 112mg (.122mmol, 54%) of product as a pale yellow solid.

The intermediate obtained above (112mg, .122mmol) was dissolved in 3mL $CHCl_3$ and treated with .1mL thiophenol (.11g, .97mmol, 8eq) at room temperature for 18hrs. All volatiles were removed under vacuum and the residue partially purified by LH-20 chromatography, eluting with MeOH. Final purification by reverse phase HPLC ($C_{18}$), eluting with MeOH/$H_2O$, afforded 22mg (.029mmol, 24%) of the title compound as a white powder. Partial NMR (300MHz, $CD_3OD$): 7.63 (s,1H), 7.2 (m,5H), 6.92 (s,1H), 4.58 (t,6Hz,1H), 4.30 (dd,1H), 4.0 (m,4H), 2.20 (d,6Hz,2H), 2.05 (s,2H), 1.00 (s,9H). FAB-MS: 754 (M+H,100%).

## Example 10

$EtO_2CNH(CH_2)_2OC(O)$-Phe-His-ACHPA-2(S)-methylbutylamide

The title compound was synthesized according to the procedure described in Example 9 from N-[(2-ethoxycarbonylamino)-ethoxycarbonyl]-L-phenylalanine (Example 3). Partial NMR (300MHz,$CD_3OD$): 8.25 (s,1H), 7.23 (m,5H), 7.15 (s,1H), 4.61 (t,7Hz,1H), 4.29 (dd,1H), 4.05 (g,6Hz,2H), 3.93 (m,2H), 2.22 (d,7Hz,2H), 1.20 (t,6Hz,3H), 0.9 (m,ca.6H). FAB MS: 728 (M+H,100%).

## Example 11

$Me_2N(CH_2)_3OC(O)$-Phe-His-ACHPA-2(S)-methylbutylamide

The title compound was synthesized according to the procedure described in Example 9 from N-[3-(dimethylamino)-1-propyloxycarbonyl]-L-phenylalanine (Example 4). Rf ($CHCl_3/MeOH/H_2O/HOAc$; 100:20:3:.5): .46. Partial NMR (300MHz,$CD_3OD$): 7.60 (s,1H), 7.25 (m,5H), 6.90 (s,1H), 4.57 (t,7Hz,1H), 4.33 (dd,1H), 3.9 (m,3H), 2.38 (s,6H), 2.21 (d,6Hz,2H), 0.9 (m,ca.6H). FAB-MS: 698 (M+H,100%).

## Example 12

$H_2NCH_2CH_2OC(O)$-Phe-His-ACHPA-2(S)-methylbutylamide hydrochloride

17mg (.023mmol) $BOCNH(CH_2)_2OC(O)$-Phe-His-ACHPA-2(S)-methylbutylamide, as prepared in Example 8 was dissolved in 1mL MeOH and treated with 1.5mL of HCl-saturated MeOH at room temperature for one hour. All volatiles were removed under vacuum to afford the title compound as a white, glassy solid. Partial NMR (300MHz,$CD_3OD$): 8.82 (s,1H), 7.40 (s,1H), 7.27 (m,5H), 4.72 (m,1H), 4.25 (m,3H), 3.98 (m,2H), 2.35 (br d,6Hz,2H), 0.9 (m,ca.6H). FAB-MS: 656 (M+H,100%).

## Example 13

$CH_3SO_2CH_2CH_2OC(O)$-Phe-His-ACHPA-2(S)-methylbutylamide

The title compound was synthesized according to the procedure described in Example 9 from N-[(2-methanesulfonyl)ethoxycarbonyl]-L-phenylalanine (Sigma). Partial NMR (300MHz,$CD_3OD$): 7.76 (s,1H), 7.25 (m,5H), 6.96 (s,1H), 4.60 (dd,1H), 4.38 (m,3H), 3.92 (m,2H), 2.94 (s,3H), 2.20 (d,6Hz,2H), 0.9 (m,ca.6H). FAB-MS: 719 (M+H,100%).

## Example 14

$CbzNHCH_2CH_2C(O)$-Phe-His-ACHPA-2(S)-methylbutylamide

The title compound was synthesized according to the procedure described in Example 9 from (N-Cbz-B-alanyl)-L-phenylalanine (Sigma). Partial NMR (300MHz,$CD_3OD$): 8.76 (s,1H), 7.3 (m,11H), 5.05 (s,2H), 4.64 (dd,1H), 4.49 (dd,1H), 3.95 (m,2H), 2.38 (t,6Hz,2H), 2.24 (d,6Hz,2H), 0.9 (m,ca.6H). FAB-MS: 774 (M+H,100%).

## Example 15

t-$BuCH_2C(O)NHCH_2CH_2C(O)$-Phe-His-ACHPA-2(S)-methylbutylamide

The title compound was synthesized according to the procedure described in Example 9 from (N-t-butylacetyl-B-alanyl)-L-phenylalanine (Example 6). Partial NMR (300MHz,$CD_3OD$): 8.81 (s,1H), 7.39 (s,1H), 7.23 (m,5H), 4.64 (dd,1H), 4.48 (dd,1H), 3.95 (m,2H), 2.38 (t,6Hz,2H), 2.25 (d,7Hz,2H), 2.00 (s,2H), 0.97 (s,9H), 0.90 (m,ca.6H). FAB-MS: 738 (M+H,100%).

## Example 16

t-BuCH$_2$CONHCH$_2$CH$_2$OCO-Phe-Nle-ACHPA-NH-[1-(4-picolyl)quinuclidin-3-yl]acetate

t-BuCH$_2$CONHCH$_2$CH$_2$OCO-Phe-Nle-ACHPA-OEt, was prepared according to procedures outlined in Example 9 except that norleucine was used in place of histidine and was converted to the hydrazide by stirring with excess hydrazine in ethanol. 0.289 gm (0.332 mmole) of the above hydrazide in 5 ml of DMF was cooled to -30°C under $N_2$. The pH was adjusted to 0.5-1.5 with a freshly prepared solution of HCl in THF (0.512 ml of a 6.49M solution, 3.32 mmole). To this mixture was added isoamyl nitrite (0.049 ml, 0.365 mmole) in four portions over 2 hours. After 2 more hours at -30°C a cooled slurry of 3-(S)-aminoquinuclidene•2HCl (0.087 gm, 0.438 mmole) and $Et_3N$ (0.6 ml) was then added and stirred for 17 hours at -10°C. The reaction mixture was then evaporated in vacuo and the residue was partitioned between $H_2O$ and BuOH. The BuOH fraction was evaporated in vacuo and purified by chromatography (silica gel, $CHCl_3$:MeOH:$NH_4OH$, 80:20:2) to give t-BuCH$_2$CONHCH$_2$CH$_2$OCO-Phe-Nle-ACHPA-NH-3(S)-aminoquinuclidene amide (0.17 gm, 65%). $^1H$ NMR consistent with structure. FAB-MS: m/e = 793 (M+H). Quaternization of the compound prepared above was accomplished by stirring it (0.17 gm, 0.221 mmole) with 4-picolylchloride hydrochloride (0.072 gm. 0.442 mmole) and $Et_3N$ (0.061 ml) in $CHCl_3$ (0.4 ml) for 3 days. The reaction mixture was evaporated in vacuo and the resulting HCl salt was exchanged with acetate using BioRad AG3 X4A (0.3 gm) and HOAc as eluent. The resulting product was purified by chromatography (silica gel, $CHCl_3$:MeOH:$NH_4OH$, 80:30:3) giving the title compound (0.14 gm, 70%). $^1H$ NMR consistent with structure. FAB-MS: m/e = 860 ($M^+$). El. An.
Calc: N, 10.16; C, 62.18; H, 8.55.
Found: N, 9.88; C, 62.21; H, 8.61.

## Example 17

N-[2-(2-amino-2-methyl)propionamidoethozycarbonyl]-Phe-His-ACHPA-NH-2(S)methylbutyl

A. Methyl-3-benzyloxycarbonylamino-3-methylbutanoate

A solution of methyl 3-carboxy-3-methylbutyrate (11 gm, 0.068 mol), prepared by the selective esterification of the diacid MeOH, $H_2SO_4$ cat.), diphenylphosphorylazide (DPPA) (16 ml, 0.075 mol), and 9.8 ml of $Et_3N$ in 120 ml of benzene was refluxed for 0.75 hour at which time was added 14 ml of benzylalcohol. The reaction mixture was allowed to reflux for 15 hours, whereupon, it was cooled to room temperature and washed with $H_2O$, and a saturated solution of $NaHCO_3$. The organic fraction was dried over $MgSO_4$, filtered, and evaporated in vacuo. The residue was chromatographed (silica, hexane:ethyl acetate, 3:1) to give 7.4 gm of the title compound. FAB-MS, m/e = 266 (M+1).

B. CbzNH-C(CH$_3$)$_2$CH$_2$C(O)-NHCH$_2$CH$_2$OC(O)-Phe-OH

.550 g (2.07 mmol) of methyl 3-(carbobenzyloxyamino)-3-methylbutanoate, prepared as described in example A, dissolved in 5 ml absolute methanol was treated with 2.5 ml of 1.0M NaOH (2.5 mmol, 1.2 eq) at room temperature for 2 hours. The mixture was diluted with 20 ml distilled water and washed twice with ether. The aqueous layer was separated, cooled to 0°, acidified by dropwise addition of 6N HCl and extracted with ethyl acetate (5 x 10 ml). The combined extracts were washed with brine, dried over $MgSO_4$, filtered and volatiles removed under vacuum to afford the carboxylic acid that was used without purification.

.760 g (2.07 mmol, 1.0 eq) of N-[(2-t-butoxycarbonylamino)ethoxycarbonyl]-L-phenylalanine methyl ester, prepared was dissolved in 3 ml methylene chloride and treated with 3 ml of HCl-saturated methanol. After 2 hours at room temperature, all volatiles were removed under vacuum and the amine further evacuated under high vacuum for 14 hours.

The carboxylic acid prepared above was dissolved in 3 ml methylene chloride and added to the amine with the aid of 5 ml of methylene chloride. The solution was then treated with 1.80 ml (1.35 g, 10.4 mmol, 5

eq) diisopropylethyl amine and 1.37 g (3.1 mmol, 1.5 eq) benzotriazol-1-yloxytris(dimethylamino)-phosphonium hexafluorophosphate. After 2 hours, the mixture was diluted with 30 ml ether, filtered and washed with 5% citric acid (2x), 5% $NaHCO_3$ (2x), and brine (1x). The solution was dried over $MgSO_4$, filtered and stripped leaving a colorless residue that was purified by medium pressure liquid chromatography, eluting with ethyl acetate/hexane (2:1) to afford 916 mg (1.84 mmol, 89%) of the methyl ester of the title compound as a colorless oil.

A solution of 916 mg (1.84 mmol) of the compound prepared above in 3 ml absolute methanol at room temperature was treated with 2.4 ml of 1.0M NaOH (2.4 mmol, 1.3 eq). After 30 minutes, the mixture was diluted with 20 ml distilled water and washed with ether (3x). The aqueous layer was separated, cooled to 0°, acidified with 6N HCl and extracted with ether (5x). The combined extracts were washed with 1N HCl (1x) and brine (1x), then dried over $MgSO_4$, filtered and stripped under vacuum to afford 858 mg (1.77 mmol, 96%) of the title compound as a white powder. NMR (300 MHz, $CD_3OD$): 7.3 (m, 10H), 5.08 (s, 2H), 4.45 (m, 1H), 4.05 (m, 2H), 3.45 (m, 2H), 3.21 (dd, 1H), 2.95 (dd, 1H), 2.57 (br s, 2H), 1.4 (br s, 6H). FAB-MS: 486 (M+H, 100%).

C. BOC(BOM)His-ACHPA-2(S)-methylbutyl amide

1.38 g (3.59 mmol) BOC-ACHPA-2(S)-methylbutyl amide in 5 ml methylene chloride was treated with 5 ml of HCl-saturated methanol solution for 2 hours at room temperature. All volatiles were removed in vacuo, and the residue further evacuated for 14 hours.

The amine salt from above was redissolved in 3 ml methylene chloride cooled to 0°, and treated with 1.42 g (3.79 mmol, 1.05 eq) BOC(BOM)His dicyclohyxylamine salt (BACHEM), followed by 3.14 ml (2.33 g, 18.0 mmol, 5 eq) diisopropylethyl amine and finally, 2.38 g (5.38 mmol, 1.5 eq) benzotriazol-1-yloxytris-(dimethylamino)phosphonium hexafluorophosphate. The mixture was maintained at 0° for 2 hours then warmed to room temperature for 1 hour.

The mixture was added to 50 ml ether and washed with 5% citric acid (3x), 5% $NaHCO_3$ (2x) and brine; then dried over , filtered and evacuated in vacuo. Puridication by medium pressure liquid chromatography, eluting with ethyl acetate/aceto nitrile/methanol (9:1:1), afforded the title compound as a white powder (2.09 g, 3.26 mmol, 91%). Partial NMR (300 MHz, $CD_3OD$): 7.78 (s, 1H), 7.4 (m, 5H), 6.93 (s, 1H), 5.54 (br s, 2H), 4.57 (br s, 2H), 4.43 (dd, 1H), 3.97 (t, 2H), 2.20 (d, 2H), 1.45 (s, 9H), .95 (m, 6H). FAB-MS: 642 (M+H, 100%).

D. N-[2-(2-amino-2-methyl)propionamidoethoxycarbonyl]-Phe-His-ACHPA-NH-2(S)methylbutyl

317 mg (.495 mmol) BOC(BOM)His-ACHPA-2(S)-methylbutylamide (Example 16), in 2 ml methylene chloride was treated with 1 ml anhydrous trifluoroacetic acid at room temperature for three hcurs. All volatiles were removed under vacuum and the residue placed under vacuum overnight.

The compound from above was redissolved in 3 ml methylene chloride, cooled to 0°, and treated with 264 mg (.54 mmol, 1.1 eq) of CbzNH-$C(CH_3)_2CH_2$(O)-$NHCH_2CH_2OC$(O)-Phe-OH (prepared as described in Example 14), followed by 0.43 ml (320 mg, 2.47 mmol, 5 eq) diisopropylethyl amine and 330 mg of benzotriazol-1-yloxytris(dimethylamino)phosphonium hexafluorophosphate (.75 mmol, 1.5 eq). The mixture was maintained at 0° for 2 hours, then warmed to room temperature for one hour. The reaction mixture was added to 30 ml ether and washed with 5% citric acid (2x), 5% $NaHCO_3$ (2x) and brine. The mixture was dried over $MgSO_4$, filtered and evacuated under vacuum to afford an oil that was purified by medium pressure liquid chromatography, eluting with ethyl acetate/acetonitrile/methanol (9:1:.5). The product (286 mg, .283 mmol, 57%) was obtained as a colorless solid FAB-MS; 1009 (M+H, 100%).

The intermediate from above (286 mg, .283 mmol) in 80% aqueous acetic acid was hydrogenated over 100 mg of 30% palladium on carbon for 14 hours at 40 psi. The mixture was filtered and the filtrate concentrated under vacuum. Purification by HPLC (RP, C-18) eluting with .1% aqueous trifluoroacetic acid/methanol (gradient; 50% MeOH to 80% MeOH over 10 minutes) afforded 105 mg (.12 mmol, 42%) of the title compound as a white powder. Partial NMR (300 MHz, $CD_3OD$): 8.86 (s, 1H), 7.44 (s, 1H), 7.3 (m, 5H), 4.73 (dd, 1H), 4.33 (dd, 1H), 4.0 (m, 4H), 2.51 (s, 2H), 2.29 (m, 2H), 1.40 (s, 3H), 1.39 (s, 3H), .95 (m, 6H). FAB-MS: 755 (M+H, 100%).

## Claims

1. A peptide of the formula:
A-B E-G J,
wherein:
A is X′-(alkyl) and bound to the "B" moiety by a linkage comprising carbonyl:

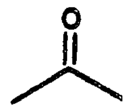

or oxycarbonyl:

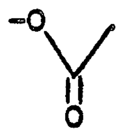

wherein:
(alkyl) is a 2 to 4 carbon chain in which 1 or 2 of the carbon atoms can each be mono- or disubstituted by $C_1$-$C_2$ alkyl;
X′ is independently selected from

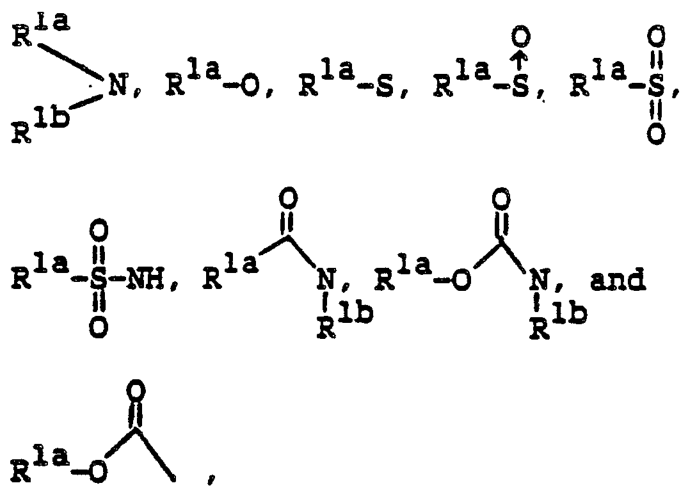

where $R^{1a}$ is hydrogen; $C_1$-$C_6$ alkyl, optionally substituted by amino, mono- or di-$C_1$-$C_4$-alkylamino; $C_3C_6$ cycloalkyl; $C_3$-$C_6$ cycloalkyl $C_1$-$C_4$ alkyl; or heterocyclyl or heterocyclyl-$C_1$-$C_4$ alkyl where the heterocyclyl group is selected from pyrrolidine, pyrazolidine, piperidine, and piperazine, and the heterocyclyl group is optionally mono$R^{1b}$ substituted by $C_1$-$C_4$ alkyl; and
is hydrogen or $C_1$-$C_4$ alkyl;
B is

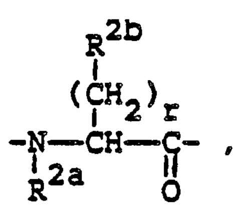

where
r is 1-to-4;
$R^{2a}$ is hydrogen or $C_1$-$C_4$ alkyl; and

$R^{2b}$ is hydrogen; indolyl; or aryl, wherein aryl is unsubstituted or mono- or disubstituted phenyl or naphthyl, where the substituent(s) is/are independently selected from the group consisting of $C_1$-$C_4$-alkyl, amino, mono- or di-$C_1$-$C_4$-alkylamino, amino $C_1$-$C_4$-alkyl, hydroxy-$C_1$-$C_4$-alkyl, phenyl-$C_1$-$C_4$-alkyl, mono- or di-$C_1$-$C_4$-alkylamino-$C_1$-$C_4$-alkyl, guanidyl, guanidyl-$C_1$-$C_4$-alkyl, hydroxyl, $C_1$-$C_4$-alkoxy, $CF_3$, halo, CHO, $CO_2H$, $CO_2$-$C_1$-$C_4$-alkyl, and $CO_2$-$C_1$-$C_4$-alkoxy;
or

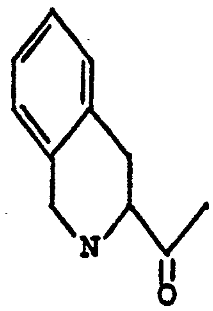
;

E is

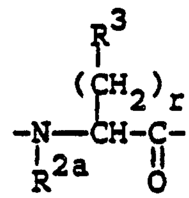
,

where
r is as defined above; and
$R^3$ is hydrogen; aryl, as defined above; Het, wherein Het is an unsubstituted or mono- or disubstituted 5- to 7-membered mono- or 7- to 10-membered bicyclic heterocyclic ring, where there are one or two heteroatoms which are independently selected from the group consisting of N, O, S, NO, SO, $SO_2$ and quaternized N, and the substituent(s) is/are independently selected from the group consisting of hydroxyl, thiol, $C_1$-$C_6$-alkyl, $CF_3$, $C_1$-$C_4$-alkoxy, halo, aryl, as defined above, aryl-$C_1$-$C_4$-alkyl, amino, mono- or di-$C_1$-$C_4$-alkylamino, amino-$C_1$-$C_4$-alkylamino, amino-$C_1$-$C_4$-alkyl, hydroxy-$C_1$-$C_4$-alkyl, di-$C_1$-$C_4$-alkylamino-$C_1$-$C_4$-alkyl, guanidyl, guanidyl-$C_1$-$C_4$alkyl, CHO, $CO_2H$, and $CO_2$-$C_1$-$C_4$-alkyl; or unsubstituted or mono- or disubstituted $C_1$-$C_4$-alkyl, wherein the substituent(s) is/are on the terminal carbon atom(s) and is/are chosen from the group consisting of $-CO_2R^{2a}$ and $-CONR^{2a}R^{2b}$, where $R^{2a}$ is as defined above and $R^{2b}$ the same definition as $R^{2a}$ but is independently selected therefrom; and when r is 2, 3 or 4, alternatively $R^3$ is $C_1$-$C_4$-alkoxy, mono or di-$C_1$-$C_4$-alkyl, $-NH_2$, or -OH;
G is

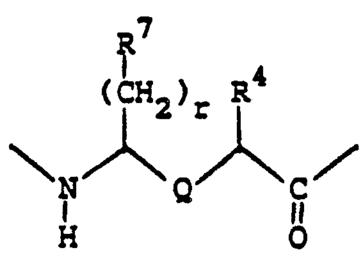
,

where
$R^4$ is hydrogen; $C_1$-$C_6$-alkyl; $C_2$-$C_6$-alkenyl; mono- or disubstituted $C_2$-$C_6$-alkyl, wherein the substituent(s) is/are on the final 1 or/and 2 carbon atoms of the alkyl chain and is/are independently selected from the group consisting of hydroxyl, carboxy, $CO_2$-$C_1$-$C_4$-alkyl, amino, mono- or di-$C_1$-$C_4$-alkylamino, aryl, as defined above, and unsubstituted or mono- or disubstituted $C_3$-$C_7$-cycloalkyl, wherein the substituent(s)

is/are independently selected from the group consisting of $C_1$-$C_4$-alkyl, trifluoromethyl, hydroxy, $C_1$-$C_4$-alkoxy, and halo; $R^7$ is hydrogen; $C_3$-$C_6$-alkyl; aryl, as defined above; or unsubstituted or mono- or disubstituted $C_3$-$C_7$-cycloalkyl, wherein the substituent(s) is/are independently selected from the group consisting of $C_1$-$C_4$-alkyl, trifluoromethyl, hydroxy, $C_1$-$C_4$-alkoxy, and halo;

Q is

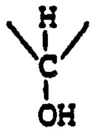 or 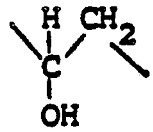

and

r is as defined above; and

J is -Y-$(CH_2)_x$-[CH($R^5$)]$_y$-$(CH_2)_z$-$R^{10}$

Y is O, NH, or N-$C_1$-$C_4$ alkyl;

x is 0 to 1;

y is 0 to 1;

z is 0 to 4;

$R^5$ is as defined above;

$R^{10}$ is hydrogen; -OH; aryl as defined above; Het, as defined above; $NH_2$; $NR^{17}R^{18}$; $-NHR^{18}$; $-N(R^{17}R^{18}R^{19})^+A^-$, where $R^{17}$ and $R^{19}$ are independently $C_1$-$C_4$ alkyl, $R^{18}$ is aryl, Het or $C_1$-$C_4$ alkyl optionally substituted with a substituent chosen from the group consisting of aryl, Het, OH, $-NH_2$ -NH-$C_1$-$C_4$ alkyl, -N($C_1$-$C_4$ alkyl)-$_2$, $-CO_2H$, $-CO_2$-$C_1$-$C_4$ alkyl, $SO_3H$, -CO-NH-$SO_2$-$C_1$-$C_4$ alkyl, or -CO-NH-$SO_2$ aryl, and $A^-$ is a counterion selected from the group consisting of small, single negatively charged ions;

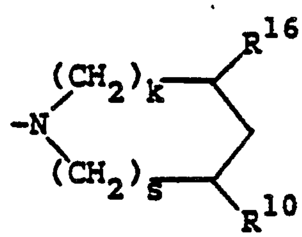

where k′ is 1 or 2; s′ is 0 or 1; $R^{16}$ is -H, -OH, $C_1$-$C_4$ alkyl, aryl, or aryloxy; and $R^{10}$ is as defined above;

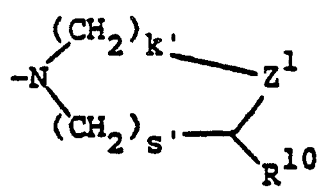

where k is 2 or 3; s' is 0 or 1; $R^{10}$ is as defined above; and $Z^1$ is O, S, SO′, $SO_2$, NH;

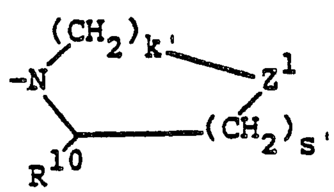

where k′, s′, $R^{10}$, and $Z^1$ are as defined above;

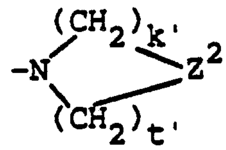

where t′ is 2 or 3; k′ is as defined above, and $Z^2$ is $NR^{18}$ or $N(R^{17}R^{18})^+A^-$, where $R^{17}$, $R^{18}$ and $A^-$ are as defined above;
and pharmaceutically-acceptable salts thereof.

2. A peptide according to Claim 1, wherein for B, r is 1 or 2; and $R^3$ is aryl substituted by $C_1$- $C_4$-alkyl, $C_1$-$C_4$-alkoxy, hydroxyl, halo or $CF_3$; and for G, r is 1; and $R^4$ is $C_1$-$C_4$ -alkyl, $C_2$-$C_4$-alkenyl, or $C_2$-$C_4$- alkyl monosubstituted by hydroxyl, amino, or mono- or di-$C_1$-$C_4$-alkylamino.

3. A peptide according to Claim 1, which is a member selected from the group consisting of:
N-(2-t-butylsulfonylethyloxycarbonyl)-Phe-His-ACHPA-NH-2(S)methylbutyl;
N-(3-t-butylsulfonylpropyloxycarbonyl)-Phe-His-ACHPA-NH-2(S)methylbutyl;
N-(4-t-butylsulfonylbutyryl)-Phe-His-ACHPA-NH-2(S)methylbutyl;
N-(2-t-butylsulfonamidoethyloxycarbonyl)-Phe-His-ACHPA-NH-2(S)methylbutyl;
N-(3-t-butylsulfonamidopropyloxycarbonyl)-Phe-His-ACHPA-NH-2(S)methylbutyl;
N-(4-t-butylsulfonamidobutyryl)-Phe-His-ACHPA-NH-2(S)methylbutyl;
N-(2-ethoxycarbonylaminoethoxycarbonyl)-Phe-His-ACHPA-NH-2(S)methylbutyl;
N-(3-ethoxycarbonylaminopropyloxycarbonyl)-Phe-His-ACHPA-NH-2(S)methylbutyl;
N-(4-ethoxycarbonylaminobutyryl)-Phe-His-ACHPA-NH-2(S)-methylbutyl;
N-(2-t-butoxycarbonylaminoethoxycarbonyl)-Phe His-ACHPA-NH-2(S)methylbutyl;
N-(4-t-butoxycarbonylaminobutyryl)-Phe-His-ACHPA-NH-2(S)methylbutyl;
N-(3-t-butoxycarbonylaminopropyloxycarbonyl)-Phe-His-ACHPA-NH-2(S)methylbutyl;
N-(2-t-butylacetamidoethoxycarbonyl)-Phe-His-ACHPA-NH-2(S)methylbutyl;
N-(3-t-butylacetamidopropyloxycarbonyl)-Phe-His-ACHPA-NH-2(S)methylbutyl;
N-(4-t-butylacetamidobutyryl)-Phe-His-ACHPA-NH-2(S)methylbutyl;
N-[2-(3-amino-3-methylbutyramidoethoxycarbonyl]-Phe-His-ACHPA-NH-2(S)methylbutyl;
N-[3-(3-amino-3-methylbutyramidopropyloxycarbonyl]-Phe-His-ACHPA-NH-2(S)methylbutyl;
N-[4-(3-amino-3-methylbutyramidobutyryl]-Phe-His-ACHPA-NH-2(S)methylbutyl;
N-(2-L-prolinamidoethoxycarbonyl)-Phe-His-ACHPA-NH-2(S)methylbutyl;
N-(3-L-prolinamidopropyloxycarbonyl)-Phe-His-ACHPA-NH-2(S)methylbutyl;
N-(4-L-prolinamidobutyryl)-Phe-His-ACHPA-NH-2(S)methyl-butyl;
N-(piperidine-4-ethoxycarbonyl)-Phe-His-ACHPA-NH-2(S)methylbutyl;
N-[2-(piperidine-3-methoxycarbonylamino)ethoxycarbonyl]-Phe-His-ACHPA-NH-2(S)methylbutyl;
N-[3-(piperidine-3-methoxycarbonylamino)propyloxycarbonyl]-Phe-His-ACHPA-NH-2(S)methylbutyl;
N-[4-(piperidine-3-methoxycarbonylamino)butyryl]-Phe-His-ACHPA-NH-2(S)methylbutyl;
N-[2-(pyrrolidine-2-methoxycarbonyl)ethyoxycarbonyl]-Phe-His-ACHPA-NH-2(S)methylbutyl;
N-[3-(pyrrolidine-2-methoxycarbonyl)propyloxycarbonyl]-Phe-His-ACHPA-NH-2(S)methylbutyl;
N-[4-(pyrrolidine-2-methoxycarbonyl)butyryl]-Phe-His-ACHPA-NH-2(S)methylbutyl;
N-(2-t-butylsulfonylethyloxycarbonyl)-Phe-His-ACHPA-NH-(N-methyl-3-aminoquinuclidinyl) acetate;
N-(3-t-butylsulfonylpropyloxycarbonyl)-Phe-His-ACHPA-NH-(N-methyl-3-aminoquinuclidinyl) acetate;
N-(4-t-butylsulfonylbutyryl)-Phe-His-ACHPA-NH-(N-methyl-3-aminoquinuclidinyl) acetate;
N-(2-t-butylsulfonamidoethyloxycarbonyl)-Phe-His-ACHPA-NH-(N-methyl-3-aminoquinuclidinyl) acetate;
N-(3-t-butylsulfonamidopropyloxycarbonyl)-Phe-His-ACHPA-NH-(N-methyl-3-aminoquinuclidinyl) acetate;
N-(4-t-butylsulfonamidobutyryl)-Phe-His-ACHPA-NH-(N-methyl-3-aminoquinuclidinyl) acetate;
N-(2-ethoxycarbonylaminoethoxycarbonyl)-Phe-His-ACHPA-NH-(N-methyl-3-aminoquinuclidinyl) acetate;
N-(3-ethoxycarbonylaminopropyloxycarbonyl)-Phe-His-ACHPA-NH-(N-methyl-3-aminoquinuclidinyl) acetate;
N-(4-ethoxycarbonylaminobutyryl)-Phe-His-ACHPA-NH-(N-methyl-3-aminoquinuclidinyl) acetate;
N-(2-t-butoxycarbonylaminoethoxycarbonyl)-Phe-His-ACHPA-NH-(N-methyl-3-aminoquinuclidinyl) acetate;
N-(4-t-butoxycarbonylaminobutyryl)-Phe-His-ACHPA-NH-(N-methyl-3-aminoquinuclidinyl) acetate;
N-(3-t-butoxycarbonylaminopropyloxycarbonyl)-Phe-His-ACHPA-NH-(N-methyl-3-aminoquinuclidinyl) acetate;
N-(2-t-butylacetamidoethoxycarbonyl)-Phe-His-ACHPA-NH-(N-methyl-3-aminoquinuclidinyl) acetate;
N-(3-t-butylacetamidopropyloxycarbonyl)-Phe-His-ACHPA-NH-(N-methyl-3-aminoquinuclidinyl) acetate;
N-(4-t-butylacetamidobutyryl)-Phe-His-ACHPA-NH-NH-(N-methyl-3-aminoquinuclidinyl) acetate;

N-[2-(3-amino-3-methylbutyramidoethoxycarbonyl]-Phe-His-ACHPA-NH-(N-methyl-3-aminoquinuclidinyl) acetate;
N-[3-(3-amino-3-methylbutyramidopropyloxycarbonyl]-Phe-His-ACHPA-NH-(N-methyl-3-aminoquinuclidinyl) acetate;
N-[4-(3-amino-3-methylbutyramidobutyryl]-Phe-His-ACHPA-NH-(N-methyl-3-aminoquinuclidinyl) acetate;
N-(2-L-prolinamidoethoxycarbonyl)-Phe-His-ACHPA-NH-(N-methyl-3-aminoquinuclidinyl) acetate;
N-(3-L-prolinamidopropyloxycarbonyl)-Phe-His-ACHPA-NH-(N-methyl-3-aminoquinuclidinyl) acetate;
N-(4-L-prolinamidobutyryl)-Phe-His-ACHPA-NH-(N-methyl-3-aminoquinuclidinyl) acetate;
N-[2-(piperidine-3-methoxycarbonylamino)ethyoxycarbonyl]-Phe-His-ACHPA-NH-(N-methyl-3-aminoquinuclidinyl) acetate;
N-2-(pyrrolidine-2-methoxycarbonyl)-Phe-His-ACHPA-NH-(N-methyl-3-aminoquinuclidinyl) acetate;
N-4-(piperidine-3-methoxycarbonyl)-Phe-His-ACHPA-NH-(N-methyl-3-aminoquinuclidinyl) acetate;
N-[3-(piperidine-3-methoxycarbonylamino)propyloxycarbonyl]-Phe-His-ACHPA-NH-(N-methyl-3-aminoquinuclidinyl) acetate;
N-[4-(piperidine-3-methoxycarbonylamino)butyryl]-Phe-His-ACHPA-NH-(N-methyl-3-aminoquinuclidinyl) acetate;
N-[2-(pyrrolidine-2-methoxycarbonyl)ethyoxycarbonyl]-Phe-His-ACHPA-NH-(N-methyl-3-aminoquinuclidinyl) acetate;
N-[3-(pyrrolidine-2-methoxycarbonyl)propyloxycarbonyl]-Phe-His-ACHPA-NH-(N-methyl-3-aminoquinuclidinyl) acetate;
N-[4-(pyrrolidine-2-methoxycarbonyl)butyryl]-Phe-His-ACHPA-NH-(N-methyl-3-aminoquinuclidinyl)acetate
N-(2-t-butylsulfonylethyloxycarbonyl)-Phe-His-Cal[CH(OH)$CH_2$]Val-NH$(CH_2)_3CH_3$;
N-(3-t-butylsulfonylpropyloxycarbonyl)-Phe-His-Cal[CH(OH)$CH_2$]Val-NH$(CH_2)_3CH_3$
N-(4-t-butylsulfonylbutyryl)-Phe-His-Cal[CH(OH)$CH_2$]Val-NH$(CH_2)_3CH_3$;
N-(2-t-butylsulfonamidoethyloxycarbonyl)-Phe-His-Cal[CH(OH)$CH_2$]Val-NH$(CH_2)_3CH_3$;
N-(3-t-butylsulfonamidopropyloxycarbonyl)-Phe-His-Cal[CH(OH)$CH_2$]Val-NH$(CH_2)_3CH_3$;
N-(4-t-butylsulfonamidobutyryl)-Phe-His--Cal[CH(OH)$CH_2$]Val-NH$(CH_2)_3CH_3$;
N-(2-ethoxycarbonylaminoethoxycarbonyl)-Phe-His-Cal[CH-(OH)$CH_2$]Val-NH$(CH_2)_3CH_3$;
N-(3-ethoxycarbonylaminopropyloxycarbonyl)-Phe-His-Cal[CH(OH)$CH_2$]Val-NH$(CH_2)_3CH_3$;
N-(4-ethoxycarbonylaminobutyryl)-Phe-His-Cal[CH(OH)$CH_2$]Val-NH$(CH_2)_3CH_3$;
N-(2-t-butoxycarbonylaminoethoxycarbonyl)-Phe-His-Cal[CH(OH)$CH_2$]Val-NH$(CH_2)_3CH_3$;
N-(4-t-butoxycarbonylaminobutyryl)-Phe-His-Cal[CH(OH)-$CH_2$]Val-NH$(CH_2)_3CH_3$;
N-(3-t-butoxycarbonylaminopropyloxycarbonyl)-Phe-His-Cal[CH(OH)$CH_2$]Val-NH$(CH_2)_3CH_3$;
N-(2-t-butylacetamidoethoxycarbonyl)-Phe-His-Cal[CH(OH)$CH_2$]Val-NH$(CH_2)_3CH_3$
N-(3-t-butylacetamidopropyloxycarbonyl)-Phe His-ACHPA-Cal[CH(OH)$CH_2$]Val-NH$(CH_2)_3CH_3$;
N-(4-t-butylacetamidobutyryl)-Phe-His-Cal[CH(OH)$CH_2$]Val-NH$(CH_2)_3CH_3$;
N-[2-(3-amino-3-methylbutyramidoethoxycarbonyl]-Phe-His-Cal[CH(OH)$CH_2$]Val-NH$(CH_2)_3CH_3$;
N-[3-(3-amino-3-methylbutyramidopropyloxycarbonyl]-Phe-His-Cal[CH(OH)$CH_2$]Val-NH$(CH_2)_3CH_3$;
N-[4-(2-amino-2-methyl)propyloxycarbonylaminobutyryl]-Phe-His-Cal[CH(OH)$CH_2$]Val-NH$(CH_2)_3CH_3$;
N-(2-L-prolinamidoethoxycarbonyl)-Phe-His-Cal[CH(OH)$CH_2$]Val-NH$(CH_2)_3CH_3$;
N-(3-L-prolinamidopropyloxycarbonyl)-Phe-His-Cal[CH(OH)$CH_2$]Val-NH$(CH_2)_3CH_3$;
N-(4-L-prolinamidobutyryl)-Phe-His-Cal[CH(OH)$CH_2$]Val-NH$(CH_2)_3CH_3$;
N-[2-(piperidine-3-methoxycarbonylamino)ethyoxycarbonyl]-Phe-His-Cal[CH(OH)$CH_2$]Val-NH$(CH_2)_3CH_3$;
N-[3-(piperidine-3-methoxycarbonylamino)propyloxycarbonyl]-Phe-His-Cal[CH(OH)$CH_2$]Val-NH$(CH_2)_3CH_3$;
N-4-(piperidine-3-methoxycarbonyl)-Phe-His-Cal[CH(OH)$CH_2$]Val-NH$(CH_2)_3CH_3$;
N-2-(pyrrolidine-2-methoxycarbonyl)Phe-His-Cal[CH(OH)$CH_2$]Val-NH$(CH_2)_3CH_3$;
N-[4-(piperidine-3-methoxycarbonylamino)butyryl]-Phe-His-Cal[CH(OH)$CH_2$]Val-NH$(CH_2)_3CH_3$;
N-[2-(pyrrolidine-2-methoxycarbonyl)ethyoxycarbonyl]-Phe-His-Cal[CH(OH)$CH_2$]Val-NH$(CH_2)_3CH_3$;
N-[3-(pyrrolidine-2-methoxycarbonyl)propyloxycarbonyl]-Phe-His-Cal[CH(OH)$CH_2$]Val-NH$(CH_2)_3CH_3$;
N-[4-(pyrrolidine-2-methoxycarbonyl)butyryl]-Phe-His-Cal[CH(OH)$CH_2$]Val-NH$(CH_2)_3CH_3$;
N-(2-t-butylsulfonylethyloxycarbonyl)-Phe-His-ACHPA-$NHCH_2CH(OH)CH_2N(CH_2CH_2)_2O$;
N-(3-t-butylsulfonylpropyloxycarbonyl)-Phe-His-ACHPA-$NHCH_2CH(OH)CH_2N(CH_2CH_2)_2O$;
N-(4-t-butylsulfonylbutyryl)-Phe-His-ACHPA-$NHCH_2CH(OH)$-$CH_2N(CH_2CH_2)_2O$;
N-(2-t-butylsulfonamidoethyloxycarbonyl)-Phe-His-ACHPA-$NHCH_2CH(OH)CH_2N(CH_2CH_2)_2O$;
N-(3-t-butylsulfonamidopropyloxycarbonyl)-Phe-His-ACHPA-$NHCH_2CH(OH)CH_2N(CH_2CH_2)_2O$;
N-(4-t-butylsulfonamidobutyryl)-Phe-His-ACHPA-$NHCH_2CH$-$(OH)CH_2N(CH_2CH_2)_2O$;
N-(2-ethoxycarbonylaminoethoxycarbonyl)-Phe-His-ACHPA-$NHCH_2CH(OH)CH_2N(CH_2CH_2)_2O$;
N-(3-ethoxycarbonylaminopropyloxycarbonyl)-Phe-His-ACHPA-$NHCH_2CH(OH)CH_2N(CH_2CH_2)_2O$;

N-(4-ethoxycarbonylaminobutyryl)-Phe-His-ACHPA-NHCH$_2$CH-(OH)CH$_2$N(CH$_2$CH$_2$)$_2$O;
N-(2-t-butoxycarbonylaminoethoxycarbonyl)-Phe-His-ACHPA-NHCH$_2$CH(OH)CH$_2$N(CH$_2$CH$_2$)$_2$O;
N-(4-t-butoxycarbonylaminobutyryl)-Phe-His-ACHPA-NHCH$_2$CH(OH)CH$_2$N(CH$_2$CH$_2$)$_2$O;
N-(3-t-butoxycarbonylaminopropyloxycarbonyl)-Phe-His-ACHPA-NHCH$_2$CH(OH)CH$_2$N(CH$_2$CH$_2$)$_2$O;
N-(2-t-butylacetamidoethoxycarbonyl)-Phe-His-ACHPA-NHCH$_2$CH(OH)CH$_2$N(CH$_2$CH$_2$)$_2$O;
N-(3-t-butylacetamidopropyloxycarbonyl)-Phe-His-ACHPA-NHCH$_2$CH(OH)CH$_2$N(CH$_2$CH$_2$)$_2$O;
N-(4-t-butylacetamidobutyryl)-Phe-His-ACHPA-NHCH$_2$CH(OH)CH$_2$N(CH$_2$CH$_2$)$_2$O;
N-[2-(3-amino-3-methylbutyramidoethoxycarbonyl]-Phe-His-ACHPA-NHCH$_2$CH(OH)CH$_2$N(CH$_2$CH$_2$)$_2$O;
N-[2-(3-amino-3-methylbutyramidoethoxycarbonyl]-Phe-His-ACHPA-NHCH$_2$CH(OH)CH$_2$N(CH$_2$CH$_2$)$_2$O;
N-[3-(3-amino-3-methylbutyramidopropyloxycarbonyl]-Phe-His-ACHPA-NHCH$_2$CH(OH)CH$_2$N(CH$_2$CH$_2$)$_2$O;
N-[4-(3-amino-3-methylbutyramidoaminobutyryl]-Phe-His-ACHPA-NHCH$_2$CH(OH)CH$_2$N(CH$_2$CH$_2$)$_2$O;
N-(2-L-prolinamidoethoxycarbonyl)-Phe-His-ACHPA-NHCH$_2$CH(OH)CH$_2$N(CH$_2$CH$_2$)$_2$O;
N-(3-L-prolinamidopropyloxycarbonyl)-Phe-His-ACHPA-NHCH$_2$CH(OH)CH$_2$N(CH$_2$CH$_2$)$_2$O;
N-(4-L-prolinamidobutyryl)-Phe-His-ACHPA-NHCH$_2$CH(OH)-CH$_2$N(CH$_2$CH$_2$)$_2$O;
N-[2-(piperidine-3-methoxycarbonylamino)ethyoxycarbonyl]-Phe-His-ACHPA-NHCH$_2$CH(OH)CH$_2$N(CH$_2$CH$_2$)-$_2$O;
N-[3-(piperidine-3-methoxycarbonylamino)propyloxycarbonyl]-Phe-His-ACHPA-NHCH$_2$CH(OH)CH$_2$N-(CH$_2$CH$_2$)$_2$O;
N-4-(piperidine-3-methoxycarbonyl)-Phe-His-ACHPA-NHCH$_2$CH(OH)CH$_2$N(CH$_2$CH$_2$)$_2$O;
N-2-(pyrrolidine-2-methoxycarbonyl)Phe-His-ACHPA-NHCH$_2$CH(OH)CH$_2$N(CH$_2$CH$_2$)$_2$O;
N-[4-(piperidine-3-methoxycarbonylamino)butyryl]-Phe-His-ACHPA-NHCH$_2$CH(OH)CH$_2$N(CH$_2$CH$_2$)$_2$O;
N-[2-(pyrrolidine-2-methoxycarbonyl)ethyoxycarbonyl]-Phe-His-ACHPA-NHCH$_2$CH(OH)CH$_2$N(CH$_2$CH$_2$)$_2$O;
N-[3-(pyrrolidine-2-methoxycarbonyl)propyloxycarbonyl]-Phe-His-ACHPA-NHCH$_2$CH(OH)CH$_2$N(CH$_2$CH$_2$)$_2$O;
N-[4-(pyrrolidine-2-methoxycarbonyl)butyryl]-Phe-His-ACHPA-NHCH$_2$CH(OH)CH$_2$N(CH$_2$CH$_2$)$_2$O;
N-(2-t-butylsulfonylethyloxycarbonyl)-Phe-Nle-ACHPA-NH-N-[(4-picolyl)-3-aminoquinuclidinyl]acetate;
N-(3-t-butylsulfonylpropyloxycarbonyl)-Phe-Nle-ACHPA-NH-N-[(4-picolyl)-3-aminoquinuclidinyl]acetate;
N-(4-t-butylsulfonylbutyryl)-Phe-Nle-ACHPA-NH-N-[(4-picolyl)-3-aminoquinuclidinyl]acetate;
N-(2-t-butylsulfonamidoethyloxycarbonyl)-Phe-Nle-ACHPA-NH-N-[(4-picolyl)-3-aminoquinuclidinyl]acetate;
N-(3-t-butylsulfonamidoproxyloxycarbonyl)-Phe-Nle-ACHPA-NH-N-[(4-picolyl)-3-aminoquinuclidinyl]acetate;
N-(4-t-butylsulfonamidobutyryl)-Phe-Nle-ACHPA-NH-N-[(4-picolyl)-3-aminoquinuclidinyl]acetate;
N-(2-ethoxycarbonylaminoethoxycarbonyl)-Phe-Nle-ACHPA-NH-N-[(4-picolyl)-3-aminoquinuclidinyl]acetate;
N-(3-ethoxycarbonylaminopropyloxycarbonyl)-Phe-Nle-ACHPA-NH-N-[(4-picolyl)-3-aminoquinuclidinyl]-acetate;
N-(4-ethoxycarbonylaminobutyryl)-Phe-Nle-ACHPA-NH-N-[(4 picolyl)-3-aminoquinuclidinyl]acetate;
N-(2-t-butoxycarbonylaminoethoxycarbonyl)-Phe-Nle-ACHPA-NH-N-[(4-picolyl)-3-aminoquinuclidinyl]acetate;
N-(4-t-butoxycarbonylaminobutyryl)-Phe-Nle-ACHPA-NH-N-[(4-picolyl)-3-aminoquinuclidinyl]acetate;
N-(3-t-butoxycarbonylaminopropyloxycarbonyl)-Phe-Nle-ACHPA-NH-N-[(4-picolyl)-3-aminoquinuclidinyl]-acetate;
N-(2-t-butylacetamidoethoxycarbonyl)-Phe-Nle-ACHPA-NH-N-[(4-picolyl)-3-aminoquinuclidinyl]acetate;
N-(3-t-butylacetamidopropyloxycarbonyl)-Phe-Nle-ACHPA-NH-N-[(4-picolyl)-3-aminoquinuclidinyl]acetate;
N-(4-t-butylacetamidobutyryl)-Phe-Nle-ACHPA-NH-N-[(4-picolyl)-3-aminoquinuclidinyl]acetate;
N-[2-(3-amino-3-methylbutyramidoethoxycarbonyl]-Phe-Nle-ACHPA-NH-N-[(4-picolyl)-3-aminoquinuclidinyl]-acetate;
N-[3-(3-amino-3-methylbutyramidopropyloxycarbonyl]-Phe-Nle-ACHPA-NH-N-[(4-picolyl)-3-aminoquinuclidinyl]acetate;
N-[4-(3-amino-3-methylbutyramidobutyryl]-Phe-Nle-ACHPA-NH-N-[(4-picolyl)-3-aminoquinuclidinyl]acetate;
N-(2-L-prolinamidoethoxycarbonyl)-Phe-Nle-ACHPA-NH-N-[(4-picolyl)-3-aminoquinuclidinyl]acetate;
N-(3-L-prolinamidopropyloxycarbonyl)-Phe-Nle-ACHPA-NH-N-[(4-picolyl)-3-aminoquinuclidinyl]acetate;
N-(4-L-prolinamidobutyryl)-Phe-Nle-ACHPA-NH-N-[(4-picolyl)-3-aminoquinuclidinyl]acetate;
N-[2-(piperidine-3-methoxycarbonylamino)ethyoxycarbonyl]-Phe-Nle-ACHPA-NH-N-[(4-picolyl)-3-aminoquinuclidinyl]acetate;
N-4-(piperidine-3-methoxycarbonyl)-Phe-Nle-ACHPA-NH-N-[(4-picolyl)-3-aminoquinuclidinyl]acetate;
N-[2-(pyrrolidine-2-methoxycarbonyl)-Phe-Nle-ACHPA-NH-N-[(4-picolyl)-3-aminoquinuclidinyl]acetate;
N-[3-(piperidine-3-methoxycarbonylamino)propyloxycarbonyl]-Phe-Nle-ACHPA-NH-N-[(4-picolyl)-3-aminoquinuclidinyl]acetate;
N-[4-(piperidine-3-methoxycarbonylamino)butyryl]-Phe-Nle-ACHPA-NH-N-[(4-picolyl)-3 aminoquinuclidinyl]-acetate;
N-[2-(pyrrolidine-2-methoxycarbonyl)ethyoxycarbonyl]-Phe-Nle-ACHPA-NH-N-[(4-picolyl)-3-aminoquinuclidinyl]acetate;
N-[3-(pyrrolidine-2-methoxycarbonyl)propyloxycarbonyl]-Phe-Nle-ACHPA-NH-N-[(4-picolyl)-3-aminoquinuclidinyl]acetate;
N-[4-(pyrrolidine-2-methoxycarbonyl)butyryl]-Phe-Nle-ACHPA-NH-N-[(4-picolyl)-3-aminoquinuclidinyl]-acetate;
N-(2-t-butylsulfonylethyloxycarbonyl)-Phe-His-ACHPA-NH-N-[(4-picolyl)-3-aminoquinuclidinyl]acetate;
N-(3-t-butylsulfonylpropyloxycarbonyl)-Phe-His-ACHPA-NH-N-[(4-picolyl)-3-aminoquinuclidinyl]acetate;
N-(4-t-butylsulfonylbutyryl)-Phe-His-ACHPA-NH-N-[(4-picolyl)-3-aminoquinuclidinyl]acetate;
N-(2-t-butylsulfonamidoethyloxycarbonyl)-Phe-His-ACHPA-NH-N-[(4-picolyl)-3-aminoquinuclidinyl]acetate;
N-(3-t-butylsulfonamidopropyloxycarbonyl)-Phe-His-ACHPA-NH-N-[(4-picolyl)-3-aminoquinuclidinyl]acetate;
N-(4-t-butylsulfonamidobutyryl)-Phe-His-ACHPA-NH-N-[(4-picolyl)-3-aminoquinuclidinyl]acetate;
N-(2-ethoxycarbonylaminoethoxycarbonyl)-Phe-His-ACHPA-NH-N-[(4-picolyl)-3-aminoquinuclidinyl]acetate;
N-(3-ethoxycarbonylaminopropyloxycarbonyl)-Phe-His-ACHPA-NH-N-[(4-picolyl)-3-aminoquinuclidinyl]-acetate;
N-(4-ethoxycarbonylaminobutyryl)-Phe-His-ACHPA-NH-N-[(4-picolyl)-3-aminoquinuclidinyl]acetate;
N-(2-t-butoxycarbonylaminoethoxycarbonyl)-Phe-His-ACHPA-NH-N-[(4-picolyl)-3-aminoquinuclidinyl]acetate;
N-(4-t-butoxycarbonylaminobutyryl)-Phe-His-ACHPA-NH-N-[(4-picolyl)-3-aminoquinuclidinyl]acetate;
N-(3-t-butoxycarbonylaminopropyloxycarbonyl)-Phe-His-ACHPA-NH-N-[(4-picolyl)-3-aminoquinuclidinyl]-acetate;
N-(2-t-butylacetamidoethoxycarbonyl)-Phe-His-ACHPA-NH-N-[(4-picolyl)-3-aminoquinuclidinyl]acetate;
N-(3-t-butylacetamidopropyloxycarbonyl)-Phe-His-ACHPA-NH-N-[(4-picolyl)-3-aminoquinuclidinyl]acetate;
N-(4-t-butylacetamidobutyryl)-Phe-His-ACHPA-NH-N-[(4-picolyl)-3-aminoquinuclidinyl]acetate;
N-[2-(3-amino-3-methylbutyramidoethoxycarbonyl]-Phe-His-ACHPA-NH-N-[(4-picolyl)-3-aminoquinuclidinyl]-acetate;
N-[3-(3-amino-3-methylbutyramidopropyloxycarbonyl]-Phe-His-ACHPA-NH-N-[(4-picolyl)-3-aminoquinuclidinyl]acetate;
N-[4-(3-amino-3-methylbutyramidobutyryl]-Phe-His-ACHPA-NH-N-[(4-picolyl)-3-aminoquinuclidinyl]acetate;
N-(2-L-prolinamidoethoxycarbonyl)-Phe-His-ACHPA-NH-N-[(4-picolyl)-3-aminoquinuclidinyl]acetate;
N-(3-L-prolinamidopropyloxycarbonyl)-Phe-His-ACHPA-NH-N-[(4-picolyl)-3-aminoquinuclidinyl]acetate;
N-(4-L-prolinamidobutyryl)-Phe-His-ACHPA-NH-N-[(4-picolyl)-3-aminoquinuclidinyl]acetate;
N-[2-(piperidine-3-methoxycarbonylamino)ethyoxycarbonyl]-Phe-His-ACHPA-NH-N-[(4-picolyl)-3-aminoquinuclidinyl]acetate;
N-4-(piperidine-3-methoxycarbonyl)-Phe-His-ACHPA-NH-N-[(4-picolyl)-3-aminoquinuclidinyl]acetate;
N-[2-(pyrrolidine-2-methoxycarbonyl)Phe-His-ACHPA-NH-N-[(4-picolyl)-3-aminoquinuclidinyl]acetate;
N-[3-(piperidine-3-methoxycarbonylamino)propyloxycarbonyl]-Phe-His-ACHPA-NH-N-[(4-picolyl)-3-aminoquinuclidinyl]acetate;
N-[4-(piperidine-3-methoxycarbonylamino)butyryl]-Phe-His-ACHPA-NH-N-[(4-picolyl)-3-aminoquinuclidinyl]-acetate;
N-[2-(pyrrolidine-2-methoxycarbonyl)ethyoxycarbonyl]-Phe-His-ACHPA-NH-N-[(4-picolyl)-3-aminoquinuclidinyl]acetate;
N-[3-(pyrrolidine-2-methoxycarbonyl)propyloxycarbonyl]-Phe-His-ACHPA-NH-N-[(4-picolyl)-3-aminoquinuclidinyl]acetate;
N-[4-(pyrrolidine-2-methoxycarbonyl)butyryl]-Phe-His-ACHPA-NH-N-[(4-picolyl)-3-aminoquinuclidinyl]acetate;
N-(2-t-butylsulfonylethyloxycarbonyl)-Phe-His-ACHPA-NH-[1-(4-hydroxybutyl)-quinuclidin-3-yl] acetate;
N-(3-t-butylsulfonylpropyloxycarbonyl)-Phe-His-ACHPA-NH-[1-(4-hydroxybutyl)-quinuclidin-3-yl] acetate;
N-(4-t-butylsulfonylbutyryl)-Phe-His-ACHPA-NH-[1-(4-hydroxybutyl)-quinuclidin-3-yl] acetate;
N-(2-t-butylsulfonamidoethyloxycarbonyl)-Phe-His-ACHPA-NH-[1-(4-hydroxybutyl)-quinuclidin-3-yl] acetate;
N-(3-t-butylsulfonamidopropyloxycarbonyl)-Phe-His-ACHPA-NH-[1-(4-hydroxybutyl)-quihuclidin-3-yl] acetate;
N-(4-t-butylsulfonamidobutyryl)-Phe-His-ACHPA-NH-[1-(4-hydroxybutyl)-quinuclidin-3-yl] acetate;
N-(2-ethoxycarbonylaminoethoxycarbonyl)-Phe-His-ACHPA-NH-[1-(4-hydroxybutyl)-quinuclidin-3-yl] acetate;
N-(3-ethoxycarbonylaminopropyloxycarbonyl)-Phe-His-ACHPA-NH-[1-(4-hydroxybutyl)-quinuclidin-3-yl] acetate;
N-(4-ethoxycarbonylaminobutyryl)-Phe-His-ACHPA-NH-[1-(4-hydroxybutyl)-quinuclidin-3-yl] acetate;
N-(2-t-butoxycarbonylaminoethoxycarbonyl)-Phe-His-ACHPA-NH-[1-(4 hydroxybutyl)-quinuclidin-3-yl] acetate;
N-(4-t-butoxycarbonylaminobutyryl)-Phe-His-ACHPA-NH-[1-(4-hydroxybutyl)-quinuclidin-3-yl] acetate;
N-(3-t-butoxycarbonylaminopropyloxycarbonyl)-Phe-His-ACHPA-NH-[1-(4-hydroxybutyl)-quinuclidin-3-yl] acetate;

N-(2-t-butylacetamidoethoxycarbonyl)-Phe-His-ACHPA-NH-[1-(4-hydroxybutyl)-quinuclidin-3-yl] acetate;
N-(3-t-butylacetamidopropyloxycarbonyl)-Phe-His-ACHPA-NH-[1-(4-hydroxybutyl)-quinuclidin-3-yl] acetate;
N-(4-t-butylacetamidobutyryl)-Phe-His-ACHPA-NH-[1-(4-hydroxybutyl)-quinuclidin-3-yl] acetate;
N-[2-(3-amino-3-methylbutyramidoethoxycarbonyl]-Phe-His-ACHPA-NH-[1-(4-hydroxybutyl)-quinuclidin-3-yl] acetate;
N-[3-(3-amino-3-methylbutyramidopropyloxycarbonyl]-Phe-His-ACHPA-NH-[1-(4-hydroxybutyl)-quinuclidin-3-yl] acetate;
N-[4-(3-amino-3-methylbutyramidobutyryl]-Phe-His-ACHPA-NH-[1-(4-hydroxybutyl)-quinuclidin-3-yl] acetate;
N-(2-L-prolinamidoethoxycarbonyl)-Phe-His-ACHPA-NH-[1-(4-hydroxybutyl)-quinuclidin-3-yl] acetate;
N-(3-L-prolinamidopropyloxycarbonyl)-Phe-His-ACHPA-NH-[1-(4-hydroxybutyl)-quinuclidin-3-yl] acetate;
N-(4-L-prolinamidobutyryl)-Phe-His-ACHPA-NH-[1-(4-hydroxybutyl)-quinuclidin-3-yl] acetate;
N-[2-(piperidine-3-methoxycarbonylamino)ethyoxycarbonyl]-Phe-His-ACHPA-NH-[1-(4-hydroxybutyl)-quinuclidin-3-yl] acetate;
N-4-(piperidine-3-methoxycarbonyl)-Phe-His-ACHPA-NH-[1-(4-hydroxybutyl)-quinuclidin-3-yl] acetate;
N-[2-(pyrrolidine-2-methoxycarbonyl)-Phe-His-ACHPA-NH-[1-(4-hydroxybutyl)-quinuclidin-3-yl] acetate;
N-[3-(piperidine-3-methoxycarbonylamino)propyloxycarbonyl]-Phe-His-ACHPA-NH-[1-(4-hydroxybutyl)-quinuclidin-3-yl] acetate;
N-[4-(piperidine-3-methoxycarbonylamino)butyryl]-Phe-His-ACHPA-NH-[1-(4-hydroxybutyl)-quinuclidin-3-yl] acetate;
N-[2-(pyrrolidine-2-methoxycarbonyl)ethyoxycarbonyl]-Phe-His-ACHPA-NH-[1-(4-hydroxybutyl)-quinuclidin-3-yl] acetate;
N-[3-(pyrrolidine-2-methoxycarbonyl)propyloxycarbonyl]-Phe-His-ACHPA-NH-[1-(4-hydroxybutyl)-quinuclidin-3-yl] acetate;
N-[4-(pyrrolidine-2-methoxycarbonyl)butyryl]-Phe-His-ACHPA-NH-[1-(4-hydroxybutyl)-quinuclidin-3-yl] acetate;
N-(2-t-butylsulfonylethyloxycarbonyl)-Phe-His-ACHPA-NH-(1-benzylquinuclidin-3-yl) acetate;
N-(3-t-butylsulfonylpropyloxycarbonyl)-Phe-His-ACHPA-NH-(1-benzylquinuclidin-3-yl) acetate;
N-(4-t-butylsulfonylbutyryl)-Phe-His-ACHPA-NH-(1-benzylquinuclidin-3-yl) acetate;
N-(2-t-butylsulfonamidoethyloxycarbonyl)-Phe-His-ACHPA-NH-(1-benzylquinuclidin-3-yl) acetate;
N-(3-t-butylsulfonamidopropyloxycarbonyl)-Phe-His-ACHPA-NH-(1-benzylquinuclidin-3-yl) acetate;
N-(4-t-butylsulfonamidobutyryl)-Phe-His-ACHPA-NH-(1-benzylquinuclidin-3-yl) acetate;
N-(2-ethoxycarbonylaminoethoxycarbonyl)-Phe-His-ACHPA-NH-(1-benzylquinuclidin-3-yl) acetate;
N-(3-ethoxycarbonylaminopropyloxycarbonyl)-Phe-His-ACHPA-NH-(1-benzylquinuclidin-3-yl) acetate;
N-(4-ethoxycarbonylaminobutyryl)-Phe-His-ACHPA-NH-(1-benzylquinuclidin-3-yl) acetate;
N-(2-t-butoxycarbonylaminoethoxycarbonyl)-Phe-His-ACHPA-NH-(1-benzylquinuclidin-3-yl) acetate;
N-(4-t-butoxycarbonylaminobutyryl)-Phe-His-ACHPA-NH-(1-benzylquinuclidin-3-yl) acetate;
N-(3-t-butoxycarbonylaminopropyloxycarbonyl)-Phe-His-ACHPA-NH-(1-benzylquinuclidin-3-yl) acetate;
N-(2-t-butylacetamidoethoxycarbonyl)-Phe-His-ACHPA-NH-(1-benzylquinuclidin-3-yl) acetate;
N-(3-t-butylacetamidopropyloxycarbonyl)-Phe-His-ACHPA-NH-(1-benzylquinuclidin-3-yl) acetate;
N-(4-t-butylacetamidobutyryl)-Phe-His-ACHPA-NH-(1-benzylquinuclidin-3-yl) acetate;
N-[2-(3-amino-3-methylbutyramidoethoxycarbonyl]-Phe-His-ACHPA-NH-(1-benzylquinuclidin-3-yl) acetate;
N-[3-(3-amino-3-methylbutyramidopropyloxycarbonyl]-Phe-His-ACHPA-NH-(1-benzylquinuclidin-3-yl) acetate;
N-[4-(3-amino-3-methylbutyramidobutyryl]-Phe-His-ACHPA-NH-(1-benzylquinuclidin-3-yl) acetate;
N-(3-L-prolinamidopropyloxycarbonyl)-Phe-His-ACHPA-NH-(1-benzylquinuclidin-3-yl) acetate;
N-(4-L-prolinamidobutyryl)-Phe-His-ACHPA-NH-(1-benzylquinuclidin-3-yl) acetate;
N-[2-(piperidine-3-methoxycarbonylamino)ethyoxycarbonyl]-Phe-His-ACHPA-NH-(1-benzylquinuclidin-3-yl) acetate;
N-[3-(piperidine-3-methoxycarbonylamino)propyloxycarbonyl]-Phe-His-ACHPA-NH-(1-benzylquinuclidin-3-yl) acetate;
N-[4-(piperidine-3-methoxycarbonylamino)butyryl]-Phe-His-ACHPA-NH-(1-benzylquinuclidin-3-yl) acetate;
N-4-(piperidine-3-methoxycarbonyl)-Phe-His-ACHPA-NH-(1-benzylquinuclidin-3-yl) acetate;
N-2-(pyrrolidine-2-methoxycarbonyl)-Phe-His-ACHPA-NH-(1-benzylquinuclidin-3-yl) acetate;
N-[2-(pyrrolidine-2-methoxycarbonyl)ethyoxycarbonyl]-Phe-His-ACHPA-NH-(1-benzylquinuclidin-3-yl) acetate;
N-[3-(pyrrolidine-2-methoxycarbonyl)propyloxycarbonyl]-Phe-His-ACHPA-NH-(1-benzylquinuclidin-3-yl) acetate;
N-[4-(pyrrolidine-2-methoxycarbonyl)butyryl]-Phe-His-ACHPA-NH-(1-benzylquinuclidin-3-yl) acetate;

N-(2-t-butylsulfonylethyloxycarbonyl)-Phe-His-ACHPA-NH-[1-((3,5-dimethylisoxazol-4-yl)methyl)quinuclidin-3-yl]acetate;
N-(3-t-butylsulfonylpropyloxycarbonyl)-Phe-His-ACHPA-NH-[1-((3,5-dimethylisoxazol-4-yl)methyl)quinuclidin-3-yl] acetate;
N-(4-t-butylsulfonylbutyryl)-Phe-His-ACHPA-NH-[1-((3,5-dimethylisoxazol-4-yl)methyl)quinuclidin-3-yl] acetate;
N-(2-t-butylsulfonamidoethyloxycarbonyl)-Phe-His-ACHPA-NH-[1-((3,5-dimethylisoxazol-4-yl)methyl)-quinuclidin-3-yl] acetate;
N-(3-t-butylsulfonamidopropyloxycarbonyl)-Phe-His-ACHPA-NH-[1-((3,5-dimethylisoxazol-4-yl)methyl)-quinuclidin-3-yl] acetate;
N-(4-t-butylsulfonamidobutyryl)-Phe-His-ACHPA-NH-[1-((3,5-dimethylisoxazol-4-yl)methyl)quinuclidin-3-yl] acetate;
N-(2-ethoxycarbonylaminoethoxycarbonyl)-Phe-His-ACHPA-NH-[1-((3,5-dimethylisoxazol-4-yl)methyl)-quinuclidin-3-yl] acetate;
N-(3-ethoxycarbonylaminopropyloxycarbonyl)-Phe-His-ACHPA-NH-[1-((3,5-dimethylisoxazol-4-yl)methyl)-quinuclidin-3-yl] acetate;
N-(4-ethoxycarbonylaminobutyryl)-Phe-His-ACHPA-NH-[1-((3,5-dimethylisoxazol-4-yl)methyl)quinuclidin-3-yl] acetate;
N-(2-t-butoxycarbonylaminoethoxycarbonyl)-Phe-His-ACHPA-NH [1-((3,5-dimethylisoxazol-4-yl)methyl)-quinuclidin-3-yl] acetate;
N-(4-t-butoxycarbonylaminobutyryl)-Phe-His-ACHPA-NH-[1-((3,5-dimethylisoxazol-4-yl)methyl)quinuclidin-3-yl] acetate;
N-(3-t-butoxycarbonylaminopropyloxycarbonyl)-Phe-His-ACHPA-NH-[1-((3,5-dimethylisoxazol-4-yl)methyl)-quinuclidin-3-yl] acetate;
N-(2-t-butylacetamidoethoxycarbonyl)-Phe-His-ACHPA-NH-[1-((3,5-dimethylisoxazol-4-yl)methyl)quinuclidin-3-yl] acetate;
N-(3,5-butylacetamidopropyloxycarbonyl)-Phe-His-ACHPA-NH-[1-((3,5-dimethylisoxazol-4-yl)methyl)-quinuclidin-3-yl] acetate;
N-(4-t-butylacetamidobutyryl)-Phe-His-ACHPA-NH-[1-((3,5-dimethylisoxazol-4-yl)methyl)quinuclidin-3-yl] acetate;
N-[2-(3-amino-3-methylbutyramidoethoxycarbonyl]-Phe-His-ACHPA-NH-[1-((3,5-dimethylisoxazol-4-yl)-methyl)quinuclidin-3-yl] acetate;
N-[3-(3-amino-3-methylbutyramidopropyloxycarbonyl]-Phe-His-ACHPA-NH-[1-((3,5-dimethylisoxazol-4-yl)-methyl)quinuclidin-3-yl] acetate;
N-[4-(3-amino-3-metylbutyramidobutyryl]-Phe-His-ACHPA-NH-[1-((3,5-dimethylisoxazol-4-yl)methyl)-quinuclidin-3-yl] acetate;
N-(2-L-prolinamidoethoxycarbonyl)-Phe-His-ACHPA-NH-[1-((3,5-dimethylisoxazol-4-yl)methyl)quinuclidin-3-yl] acetate;
N-(3-L-prolinamidopropyloxycarbonyl)-Phe-His-ACHPA-NH-[1-((3,5-dimethylisoxazol-4-yl)methyl)quinuclidin-3-yl]acetate;
N-(4-L-prolinamidobutyryl)-Phe-His-ACHPA-NH-[1-((3,5-dimethylisoxazol-4-yl)methyl)quinuclidin-3-yl] acetate;
N-[2-(piperidine-3-methoxycarbonylamino)ethyoxycarbonyl]-Phe-His-ACHPA-NH-[1-((3,5-dimethylisoxazol-4-yl)methyl)quinuclidin-3-yl] acetate;
N-4-(piperidine-3-methoxycarbonyl)-Phe-His-ACHPA-NH-[1-((3,5-dimethylisoxazol-4-yl)methyl)quinuclidin-3-yl] acetate;
N-2-(pyrrolidine-2-methoxycarbonyl)-Phe-His-ACHPA-NH-[1-((3,5-dimethylisoxazol-4-yl)methyl)quinuclidin-3-yl]acetate;
N-[3-(piperidine-3-methoxycarbonylamino)propyloxycarbonyl]-Phe-His-ACHPA-NH-[1-((3,5-dimethylisoxazol-4-yl)methyl)quinuclidin-3-yl] acetate;
N-[4-(piperidine-3-methoxycarbonylamino)butyryl]-Phe-His-ACHPA-NH-[1-((3,5-dimethylisoxazol-4-yl)-methyl)quinuclidin-3-yl] acetate;
N-[2-(pyrrolidine-2-methoxycarbonyl)ethyoxycarbonyl]-Phe-His-ACHPA-NH-[1-((3,5-dimethylisoxazol-4-yl)-methyl)quinuclidin-3-yl] acetate;
N-[3-(pyrrolidine-2-methoxycarbonyl)propyloxycarbonyl]-Phe-His-ACHPA-NH-[1-((3,5-dimethylisoxazol-4-yl)-methyl)quinuclidin-3-yl] acetate;
N-[4-(pyrrolidine-2-methoxycarbonyl)butyryl]-Phe-His-ACHPA-NH-[1-((3,5-dimethylisoxazol-4-yl)methyl)-quinuclidin-3-yl] acetate.

4. A peptide according to claim 3, which is a member selected from the group consisting of:
N-(2-t-butoxycarbonylaminoethoxycarbonyl)-Phe-His-ACHPA-NH-2(S)methylbutyl;
N-(4-t-butoxycarbonylaminobutyryl)-Phe-His-ACHPA-NH-2(S)methylbutyl;
N-(2-t-butylacetamidoethoxycarbonyl)-Phe-His-ACHPA-NH-2(S)methylbutyl;
N-[2-(3-amino-3-methyl)propiomylamidoethoxycarbonyl]-Phe-His-ACHPA-NH-2(S)methylbutyl;
N-(piperidine-4-ethoxycarbonyl)-Phe-His-ACHPA-NH-2(S)methylbutyl;
N-[2-(piperidine-3-methoxycarbonylamino)ethoxycarbonyl]-Phe-His-ACHPA-NH-2(S)methylbutyl;
N-(2-t-butoxycarbonylaminoethoxycarbonyl)-Phe-Nle-ACHPA-NH-N-[(4-picolyl)-3-aminoquinuclidinyl]acetate;
N-(2-t-butylacetamidoethoxycarbonyl)-Phe-Nle-ACHPA-NH-N-[(4-picolyl)-3-aminoquinuclidinyl]acetate;
N-[2-(3-amino-3-methylbutyramidoethoxycarbonyl]-Phe-Nle-ACHPA-NH-N-[(4-picolyl)-3-aminoquinuclidinyl]-acetate;
N-(2-t-butoxycarbonylaminoethoxycarbonyl)-Phe-His-ACHPA-NH-N-[(4-picolyl)-3-aminoquinuclidinyl]acetate;
N-(2-t-butylacetamidoethoxycarbonyl)-Phe-His-ACHPA-NH-N-[(4-picolyl)-3-aminoquinuclidinyl]acetate;
N-[2-(3-amino-3-methylbutyramidoethoxycarbonyl]-Phe-His-ACHPA-NH-N-[(4-picolyl)-3-aminoquinuclidinyl]-acetate;
N-(2-t-butylsulfonylethyloxycarbonyl)-Phe-His-ACHPA-NH-(N-methyl-3-aminoquinuclidinyl) acetate;
N-(2-t-butoxycarbonylaminoethoxycarbonyl)-Phe-His-ACHPA-NH-(N-methyl-3-aminoquinuclidinyl) acetate;
N-(2-t-butylacetamidoethoxycarbonyl)-Phe-His-ACHPA-NH-(N-methyl-3-aminoquinuclidinyl) acetate;
N-[2-(3-amino-3-methylbutyramidoethoxycarbonyl]-Phe-His-ACHPA-NH-(N-methyl-3-aminoquinuclidinyl) acetate;
N-[2-(piperidine-3-methoxycarbonylamino)ethyoxycarbonyl]-Phe-His-ACHPA-NH-(N-methyl-3-aminoquinuclidinyl) acetate;
N-(2-t-butoxycarbonylaminoethoxycarbonyl)-Phe-His-Cal[$CH(OH)CH_2$]Val-$NH(CH_2)_3CH_3$;
N-(2-t-butylacetamidoethoxycarbonyl)-Phe-His-Cal[$CH(OH)CH_2$]Val-$NH(CH_2)_3CH_3$;
N-[2-(3-amino-3-methylbutyramidoethoxycarbonyl]-Phe-His-Cal[$CH(OH)CH_2$]Val-$NH(CH_2)_3CH_3$;
N-(2-t-butoxycarbonylaminoethoxycarbonyl)-Phe-His-ACHPA-$NHCH_2CH(OH)CH_2N(CH_2)_3CH_3$;
N-(2-t-butylacetamidoethoxycarbonyl)-Phe-His-ACHPA-$NHCH_2CH(OH)CH_2N(CH_2CH_2)_2O$;
N-[2-(3-amino-3-methylbutyramidoethoxycarbonyl]-Phe-His-ACHPA-NHCH-$CH(OH)CH_2N(CH_2CH_2)_2O$;
N-(2-t-butoxycarbonylaminoethoxycarbonyl)-Phe-His-ACHPA-NH-[1-(4-hydroxybutyl)-quinuclidin-3-yl] acetate;
N-(2-t-butylacetamidoethoxycarbonyl)-Phe-His-ACHPA-NH-[1-(4-hydroxybutyl)-quinuclidin-3-yl] acetate;
N-(2-t-butoxycarbonylaminoethoxycarbonyl)-Phe-His-ACHPA-NH-(1-benzylquinuclidin-3-yl) acetate;
N-(2-t-butylacetamidoethoxycarbonyl)-Phe-His-ACHPA-NH-(1-benzylquinuclidin-3-yl) acetate;
N-[2-(3-amino-3-methylbutyramidoethoxycarbonyl]-Phe-His-ACHPA-NH-(1-benzylquinuclidin-3-yl) acetate;
N-(2-t-butoxycarbonylaminoethoxycarbonyl)-Phe-His-ACHPA-NH-[1-((3,5-dimethylisoxazol-4-yl)methyl)-quinuclidin-3-yl] acetate;
N-(2-t-butylacetamidoethoxycarbonyl)-Phe-His-ACHPA-NH-[1-((3,5-dimethylisoxazol-4-yl)methyl)quinuclidin-3-yl] acetate;
N-[2-(3-amino-3-methylbutyramidoethoxycarbonyl]-Phe-His-ACHPA-NH-[1-((3,5-dimethylisoxazol-4-yl)-methyl)--quinuclidin-3-yl] acetate;
N-2-(pyrrolidine-2-methoxycarbonyl)-Phe-His-ACHPA-NH-(N-methyl-3-aminoquinuclidinyl) acetate;
N-4-(piperidine-3-methoxycarbonyl)-Phe-His-ACHPA-NH-(N-methyl-3-aminoquinuclidinyl) acetate;
N-4-(piperidine-3-methoxycarbonyl)-Phe-His-Cal[$CH(OH)CH_2$]Val-$NH(CH_2)_3CH_3$;
N-2-(pyrrolidine-2-methoxycarbonyl)Phe-His-Cal[$CH(OH)CH_2$]Val-$NH(CH_2)_3CH_3$;
N-4-(piperidine-3-methoxycarbonyl)-Phe-His-ACHPA-$NHCH_2CH(OH)CH_2N(CH_2CH_2)_2O$;
N-2-(pyrrolidine-2-methoxycarbonyl)Phe-His-ACHPA-$NHCH_2CH(OH)CH_2N(CH_2CH_2)_2O$;
N-4-(piperidine-3-methoxycarbonyl)-Phe-His-ACHPA-NH-[1-(4-hydroxybutyl)-quinuclidin-3-yl] acetate;
N-2-(pyrrolidine-2-methoxycarbonyl)Phe-His-ACHPA-NH-[1-(4-hydroxybutyl)-quinuclidin-3-yl] acetate;
N-4-(piperidine-3-methoxycarbonyl)-Phe-His-ACHPA-NH-(1-benzylquinuclidin-3-yl) acetate;
N-2-(pyrrolidine-2-methoxycarbonyl)-Phe-His-ACHPA-NH-(1-benzylquinuclidin-3-yl) acetate;
N-4-(piperidine-3-methoxycarbonyl)-Phe-His-ACHPA-NH-[1-((3,5-dimethylisoxazol-4-yl)methyl)quinuclidin-3-yl] acetate;
N-2-(pyrrolidine-2-methoxycarbonyl)-Phe-His-ACHPA-NH-[1-((3,5-dimethylisoxazol-4-yl)methyl)quinuclidin-3-yl] acetate.

5. A pharmaceutical composition for renin-associated hypertension or congestive heart failure comprising a pharmaceutical carrier and a therapeutically-effective amount of a peptide according to Claim 1.

6. A pharmaceutical composition according to Claim 5, also comprising an adjuvant.

7. A pharmaceutical composition according to Claim 5, also comprising one or more compounds

selected from the group consisting of:

Diuretics:

acetazolamide; amiloride; bendroflumethiazide; benzthiazide; bumetanide; chlorothiazide; chlorthalidone; cyclothiazide; ethacrynic acid; furosemide; hydrochlorothiazide; hydroflumethiazide; indacrinone (racemic mixture, or as either the (+) or (-) enantiomer alone, or a manipulated ratio, e.g., 9:1 of said enantiomers, respectively); metolazone; methyclothiazide; muzolimine; polythiazide; quinethazone; sodium ethacrynate; sodium nitroprusside; spironolactone; ticrynafen; triamterene; trichlormethiazide;

α-Adrenergic Blocking Agents:

dibenamine; phentolamine; phenoxybenzamine; prazosin; tolazoline;

β-Adrenergic Blocking Agents:

atenolol; metoprolol; nadolol; propranolol; timolol;
((±)-2-[3-(tert-butylamino)-2-hydroxypropoxy]-2-furan-anilide) (ancarolol);
(2-acetyl-7-(2-hydroxy-3-isopropylaminopropoxy)benzofuran HCl) (befunolol);
((±)-1-(isopropylamino)-3-(p-(2-cyclopropylmethoxyethyl)-phenoxy)-2-propranol HCl) (betaxolol);
(1-[(3,4-dimethoxyphenethyl)amino]-3-(m-tolyloxy)-2-propanol HCl) (bevantolol);
((±)-1-(4-((2-isopropoxyethoxy)methyl)phenoxy)-3-isopropylamino-2-propanol)fumarate) (bisoprolol);
(4-(2-hydroxy-3-[4-(phenoxymethyl)-piperidino]propoxy)-indole);
(carbazolyl-4-oxy-5,2-(2-methoxyphenoxy)-ethylamino-2-propanol);
(1-((1,1-dimethylethyl)amino)-3-((2-methyl-1H-indol-4-yl)oxy)-2-propanol benzoate) (bopindolol);
(1-(2-exobicyclo[2.2.1]-hept-2-ylphenoxy)-3-[(1-methylethyl)-amino]-2-propanol HCl) (bornaprolol);
(o-[2-hydroxy-3-[(2-indol-3-yl-1,1-dimethylethyl)amino]propoxy]benzonitrile HCl) bucindolol);
(α-[(tert.butylamino)methyl]-7-ethyl-2-benzofuran-methanol) (bufuralol);
(3-[3-acetyl-4-[3-(tert.butylamino)-2-hydroxypropylphenyl]-1,1-diethylurea HCl) (celiprolol);
((±)-2-[2-[3-[(1,1-dimethylethyl)amino]-2-hydroxypropoxy]phenoxy]-N-methylacetamide HCl) (cetamolol);
(2-benzimidazolyl-phenyl(2-isopropylaminopropanol));
((±)-3′-acetyl-4′-(2-hydroxy-3-isopropylaminopropoxy)acetanilide HCl) (diacetolol);
(methyl-4-[2-hydroxy-3-[(1 methylethyl)aminopropoxy]]benzenepropanoate HCl) (esmolol):
(erythro-DL-1-(7-methylindan-4-yloxy)-3-isopropylaminobutan-2-ol);
(1-(tert.butylamino)-3-[O-(2-propynyloxy)phenoxy]-2-propanol (pargolol);
(1-(tert.butylamino)-3-[o-(6-hydrazino-3-pyridazinyl)phenoxy]-2-propanol diHCl) (prizidilol);
((-)-2 hydroxy-5-[(R)-1-hydroxy-2-[(R)-(1-methyl-3-phenylpropyl)amino]ethyl]benzamide);
(4-hydroxy-9-[2-hydroxy-3-(isopropylamino)-propoxy]-7-methyl-5H-furo[3,2-g][1]-benzopyran-5-one) (iprocrolol);
((-)-5-(tert.butylamino)-2-hydroxypropoxy]-3,4-dihydro-1-(2H)-naphthalenone HCl) (levobunolol);
(4-(2-hydroxy-3-isopropylamino-propoxy)-1,2-benzisothiazole HCl);
(4-[3-(tert.butylamino)-2-hydroxypropoxy]-N-methylisocarbostyril HCl);
((±)-N-2-[4-(2-hydroxy-3-isopropyl aminopropoxy)phenyl]ethyl-N′-isopropylurea) (pafenolol);
(3-[[(2-trifluoroacetamido)ethyl]amino]-1-phenoxypropan-2-ol);
(N-(3-(o-chlorophenoxy)-2-hydroxypropyl)-N′-(4′chloro-2,3-dihydro-3-oxo-5-pyridazinyl)ethylenediamine);
((±)-N-[3-acetyl-4-[2-hydroxy-3-[(1-methylethyl)amino]propoxy]phenyl]butanamide) (acebutolol);
((±)-4′-[3-(tert-butylamino)-2-hydroxypropoxy]spiro[cyclohexane-1,2′-indan]-1′-one) (spirendolol);
(7-[3-[[2-hydroxy-3-[(2-methylindol-4-yl)oxy]propyl]amino]butyl]thiophylline) (teoprolol);
((±)-1-tert.butylamino-3-(thiochroman-8-yloxy)-2-propanol) (tertatolol);
((±)-1-tert.butylamino-3-(2,3-xylyloxy)-2-propanol HCl) (xibenolol);
(8-[3-(tert.butylamino)-2-hydroxypropoxy]-5-methylcoumarin) (bucumolol);
(2-(3-(tert.butylamino)-2-hydroxy propoxy)benzonitrile HCl) (bunitrolol);
((+)-2′-[3-(tert-butylamino)-2-hydroxypropoxy-5′-fluorobutyrophenone) (butofilolol);
(1-(carbazol-4-yloxy)-3-(isopropylamino)-2-propanol)(carazolol);
(5-(3-tert.butylamino-2-hydroxy)propoxy-3,4-dihydrocarbostyril HCl) (carteolol);
(1-(tert.butylamino)-3-(2,5-dichlorophenoxy)-2-propanol) (cloranolol);
(1-(inden-4(or-7)-yloxy)-3-(isopropylamino)-2-propanol HCl) (indenolol);
(1-isopropylamino-3-[(2-methylindol-4-yl)oxy]-2-propanol) (mepindolol);

(1-(4-acetoxy-2,3,5-trimethylphenoxy)-3-isopropylaminopropan-2-ol) (metipranolol);
(1-(isopropylamino)-3-(o-methoxyphenoxy)-3-[(1-methylethyl)amino]-2-propanol) (moprolol);
((1-tert.butylamino)-3-[(5,6,7,8-tetrahydro-cis-6,7-dihydroxy-1-naphthyl)oxy-2-propanol) (nadolol);
((S)-1-(2-cyclopentylphenoxy)-3-[(1,1-dimethylethyl)amino]-2-propanol sulfate (2:1)) (penbutolol);
(4′-[1-hydroxy-2-(amino)ethyl]methanesulfonanilide) (sotalol);
(2-methyl-3-[4-(2-hydroxy-3-tert.butylaminopropoxy)phenyl]-7-methoxy-isoquinolin-1-(2H)-one);
(1-(4-(2-(4-fluorophenyloxy)ethoxy)phenoxy)-3-isopropylamino-2-propanol HCl);
((-)-p-[3-[(3,4-dimethoxyphenethyl)amino]-2-hydroxypropoxy]-β-methylcinnamonitrile) (pacrinolol);
((±)-2-(3′-tert.butylamino-2′-hydroxypropylthio)-4-(5′-carbamoyl-2′-thienyl)thiazole HCl) (arotinolol);
((±)-1-[p-[2-(cyclopropylmethoxy)ethoxy]phenoxy]-3-(isopropylamino)-2-propanol) (cicloprolol);
((±)-1-[(3-chloro-2methylindol-4-yl)oxy]-3-[(2-phenoxyethyl)amino]-2-propanol) (indopanolol);
((±)-6-[[2-[[3-(p-butoxyphenoxy)-2-hydroxypropyl]amino]ethyl]amino]-1,3-dimethyluracil) (pirepolol);
(4-(cyclohexylamino)-1-(1-naphtholenyloxy)-2-butanol);
(1-phenyl-3-[2-[3-(2-cyanophenoxy)-2-hydroxypropyl]aminoethyl]hydantoin HCl);
(3,4-dihydro-8-(2-hydroxy-3-isopropylaminopropoxy)-3-nitroxy-2H-1-benzopyran) (nipradolol);

α- and β-Adrenergic Blocking Agents:

((±)-1-tert-butylamino)-3-[o-[2-(3-methyl-5-isoxazolyl)vinyl]phenoxy]-2-propanol) (isoxaprolol);
(1-isopropylamino-3-(4-(2-nitroxyethoxy)phenoxy)-2-propanol HCl);
(4-hydroxy-α-[[3-(4-methoxyphenyl)-1-methylpropyl]aminomethyl]-3-(methylsulfinyl)-benzmethanol HCl) (sulfinalol);
(5-[1-hydroxy-2-[[2-(o-methoxyphenoxy)ethyl]amino]ethyl]-2-methylbenzenesulfonamide HCl);
(5-[1-hydroxy-2-[(1-methyl-3-phenylpropyl)amino]ethyl]salicylamide HCl) (labetalol);
(1-((3-chloro-2-methyl-1H-indol-4-yl)oxy)-3-((2-phenoxyethyl)amino)-2-propanol-hydrogenmalonate) (ifendolol);
(4-(2-hydroxy-3-[(1-methyl-3-phenylpropyl)amino]propoxy)benzeneacetamide);
(1-[3-[[3-(1-naphthoxy)-2-hydroxypropyl]-amino]-3,3-dimethyl-propyl]-2-benzimidazolinone);
(3-(1-(2-hydroxy-2-(4-chlorophenylethyl)-4-piperidyl)-3,4-dihydroxy)quinoxolin-2(1H)-one);

CNS-Acting Agents:

clonidine; methyldopa;

Adrenergic Neuron Blocking Agents:

guanethidine; reserpine and other rauwolfia alkaloids such as rescinnamine;

Vasodilators:

diazoxide; hydralazine; minoxidil;

Angiotensin I Converting Enzyme Inhibitors:

1-(3-mercapto-2-methyl-1-oxopropyl)-L-proline (captopril);
(1-(4-ethoxycarbonyl-2,4(R,R)-dimethylbutanoyl)indoline-2(S)-carboxylic acid);
(2-[2-[[1-(ethoxycarbonyl)-3-phenyl-propyl]amino]-1-oxopropyl]-1,2,3,4-tetrahydro-3-isoquinoline carboxylic acid);
((S)-1-[2-[[1-(ethoxycarbonyl)-3-phenylpropyl]amino]-1-oxopropyl]octahydro-1H-indole-2-carboxylic acid HCl);
(N-cyclopentyl-N-(3-(2,2-dimethyl-1-oxopropyl)thiol-2-methyl-1-oxopropyl)glycine) (pivalopril);
((2R,4R)-2-(2-hydroxyphenyl)-3-(3-mercaptopropionyl)-4-thiazolidinecarboxylic acid);
(1-(N-[1(S)-ethoxycarbonyl-3-phenylpropyl]-(S)-alanyl)cis,syn-octahydroindol-2(S)-carboxylic acid HCl);
((-)-(S)-1-[(S)-3-mercapto-2-methyl-1-oxopropyl]indoline-2-carboxylic acid);
([1(S),4S]-1-[3-(benzoylthio)-2-methyl-1-oxopropyl]-4-phenylthio-L-proline;
(3-([1-ethoxycarbonyl-3-phenyl-(1S)-propyl]amino)-2,3,4,5-tetrahydro-2-oxo-1-(3S)-benzazepine-1-acetic acid HCl);
(N-(2-benzyl-3-mercaptopropanoyl)-S-ethyl-L-cysteine) and the S-methyl analogue;

(N-(1(S)-ethoxycarbonyl-3-phenylpropyl)-L-alanyl-L-proline maleate) (enalapril);
N-[1-(S) carboxy-3-phenylpropyl]-L-alanyl-1-proline;
$N^2$-[1-(S)-carboxy-3-phenylpropyl]-L-lysyl-L-proline (lisinopril);

Calcium Channel Blockers:

α-[3-[[2-(3,4-dimethoxyphenyl)ethyl]methylamino]propyl]-3,4-dimethoxy-α-(1-methylethyl)benzeneacetonitrile (verapamil);
1,4-dihydro-2,6-dimethyl-4-(2-nitrophenyl)-3,5-pyridinedicarboxylic acid dimethyl ester (nifedipine);
2-(2,2-dicyclohexylethyl)piperidine (perhexiline);
N-(1-methyl-2-phenylethyl)- -phenylbenzenepropanamine (prenylamine);
3-aminosulfonyl)-4-chloro-N-(2,3-dihydro-2-methyl-1H-indol-1-yl)benzamide (indapamide);
(2′-(2-diethylaminoethoxy)-3-phenylpropiophenone (etafenone);
(4-[4,4-bis-(4-fluorophenyl)butyl]-N-(2,6-dimethylphenyl)-1-piperazineacetamide) (lidoflazine);
(2-(N-benzyl-N-methylamino)ethylmethyl-2,6-dimethyl-4-(m-nitrophenyl)-1,4-dihydro-3,5-pyridinedicarboxylate HCl) (nicardipine);
(N-(3,4-dimethoxyphenethyl)-2-(3,4-dimethoxyphenyl)-N-methyl-m-dithiane-2-propylamine-1,1,3,3-tetraoxide) (tiapamil);
(5,6-dimethoxy-2-(3-[(α-(3,4-dimethoxy)phenylethyl)methylamino]propyl)phthalimidine) (falipamil);
(β-[(2-methylpropoxy)methyl]-N-phenyl-N-phenylmethyl-1-pyrrolidineethanamine HCl monohydrate) (bepridil);
((±) cis-3-(acetyloxy)-5-[2-(dimethylamino)ethyl]-2,3-dihydro-2-(4-methoxyphenyl)-1,5-benzothiazepin-4-(5H)-one) (diltiazem);
((E)-1-[bis-(p-fluorophenyl)methyl]-4-cinnamylpiperazine di HCl) (flunarizine);
(5 [(3,4-dimethoxyphenethyl)methylamino]-2-isopropyl-2-(3,4,5-trimethoxyphenyl)valeronitrile (gallopamil);
(ethylmethyl(2,3-dichlorophenyl)-1,4-dihydro-2,6-dimethyl-3,5-pyridinedicarboxylate (felodipine);
(isopropyl-2-methoxyethyl-1,4-dihydro-2,6-dimethyl-4-(3-nitrophenyl)-3,5-pyridinecarboxylate) (nimodipine);
(3-ethyl-5-methyl-1,4-dihydro-2,6-dimethyl-4-(3-nitrophenyl)-3,5-pyridine dicarboxylate) (nitrendipine); and

Other Antihypertensive Agents:

aminophylline; cryptenamine acetates and tannates; deserpidine; meremethoxylline procaine; pargyline; trimethaphan camsylate; and the like, as well as admixtures and combinations thereof.

8. The use of a peptide as claimed in Claim 1 for the preparation of a medicament useful for treating renin-associated hypertension or congestive heart failure in mammals.

9. The use as claimed in Claim 8, wherein mammals are human and the amount in the medicament is from 0.02 to 10 grams per day.

10. A method of diagnosing renin as a contributory factor in hypertension or conjestive heart failure comprising administering to a patient from 0.1 to 10 mg/kg of a body weight of the patient of peptide according to Claim 1 and monitoring the patients' blood pressure for transitory fall that would indicate supranormal plasma renin level.